(19) Europäisches Patentamt
European Patent Office
Office européen des brevets

(11) Veröffentlichungsnummer : **0 102 319**
**B1**

(12) # EUROPÄISCHE PATENTSCHRIFT

(45) Veröffentlichungstag der Patentschrift : **19.08.87**

(51) Int. Cl.[4] : **A 61 K 31/70**// C07H15/00, C07K7/04

(21) Anmeldenummer : **83810324.0**

(22) Anmeldetag : **18.07.83**

(54) **Verwendung von Muramylpeptiden oder deren Analogen zur Prophylaxe und Therapie von Virusinfektionen.**

(30) Priorität : **23.07.82 CH 4528/82**

(43) Veröffentlichungstag der Anmeldung : **07.03.84 Patentblatt 84/10**

(45) Bekanntmachung des Hinweises auf die Patenterteilung : **19.08.87 Patentblatt 87/34**

(84) Benannte Vertragsstaaten : **CH FR GB LI LU NL SE**

(56) Entgegenhaltungen :
**EP-A- 0 025 495**
**EP-A- 0 027 258**
**EP-A- 0 056 992**

(73) Patentinhaber : **CIBA-GEIGY AG**
**Klybeckstrasse 141**
**CH-4002 Basel (CH)**

(72) Erfinder : **Lukas, Bohumir, Dr.**
**Florastrasse 18/4**
**CH-4057 Basel (CH)**
Erfinder : **Schmidt-Ruppin, Karl Heinz, Dr.**
**Im Baumgarten 5**
**CH-4144 Arlesheim (CH)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Verwendung von Hexopyranosederivaten, die einen Phosphorylrest enthalten und in 3-Stellung des Zuckerteils eine Peptidseitenkette aufweisen, zur Herstellung von pharmazeutischen Präparaten, die für die Anwendung zur Prophylaxe und Therapie von Virusinfektionen bestimmt sind, sowie pharmazeutische Präparate, die weniger als 1 Gewichtsprozent eines solchen Hexopyranosederivats enthalten.

Die obengenannten Hexopyranoseverbindungen der Formel I und ihre Salze sowie ihre Herstellung sind beschrieben in den europäischen Patentanmeldungen mit den Publikationsnummern 0025 495, 0027 258 und 0056 992, die auf den Namen der Anmelderin der vorliegenden Erfindung lauten, wobei die letztgenannte Anmeldung erst nach dem Prioritätstag der vorliegenden Anmeldung veröffentlicht wurde. Die Verwendung der Phosphorylmuramylpeptide per se zur Herstellung von pharmazeutischen Präparaten für die Prophylaxe und Therapie von Virusinfektionen ist in den obengenannten Anmeldungen weder beschrieben noch nahegelegt.

Der vorletzte Absatz auf Seite 13 der obengenannten Anmeldung 0025 495 bezieht sich lediglich auf die Verwendung als Adjuvans in Mischung mit Impfstoffen.

Die obengenannten Anmeldungen 0025 495 und 0027 258 beschrieben auch pharmazeutische Präparate, die aber etwa 10 % bis etwa 95 % Wirkstoff enthalten. In der obengenannten Anmeldung 0056 992 sind pharmazeutische Präparate beschrieben, die etwa 1 % bis 100 %, insbesondere etwa 5 % bis etwa 50 %, Wirkstoff enthalten.

Die erfindungsgemässe Aufgabe bestand in der Bereitstellung von pharmazeutischen Präparaten, die zur Prophylaxe und Therapie von Virusinfektionen an Warmblütern hervorragend geeignet sind.

Die anmeldungsgemässe Lösung dieser Aufgabe beruht auf dem Befund, dass Hexopyranoseverbindungen der Formel I, worin

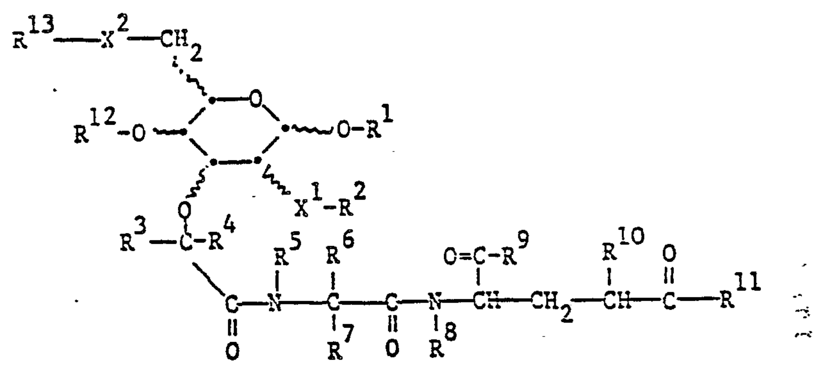

(I)

sich der Zuckerteil von (D)-Galactose, (D)-Mannose oder (D)-Glucose ableitet, die an einem asymmetrisch substituierten C(—$R^3$)-Atom die (D)-Konfiguration, an einem asymmetrisch substituierten C(—$R^6$)-Atom die (L)-Konfiguration und am C(—N—$R^8$)-Atom die (D)-Konfiguration aufweisen und worin $X^1$ und $X^2$ unabhängig voneinander eine Gruppe der Formel —O— oder —N($R^{14}$)—, wobei $R^{14}$ für Wasserstoff oder Niederalkyl steht, $R^1$, $R^2$, $R^{12}$ und $R^{13}$ unabhängig eineinander einen Rest der Formel Ia,

$$—(Z^1—Y^1—X^3)_n—A^1 \qquad \text{(Ia)}$$

worin n für 0 oder 1, $Z^1$ für Carbonyl oder Thiocarbonyl, $Y^1$ für unsubstituiertes oder substituiertes Alkylen, das durch Iminocarbonyl oder Oxycarbonyl unterbrochen sein kann, $X^3$ für eine Gruppe der Formel —O— oder —N($R^{14}$)—, worin $R^{14}$ die oben gegebene Bedeutung hat, und $A^1$ für einen Rest der Formel Ib,

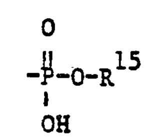

(Ib)

worin $R^{15}$ einen mindestens 7 Kohlenstoffatome aufweisenden aliphatischen oder cycloaliphatischen Rest darstellt, oder für eine Gruppe der Formel Ic,

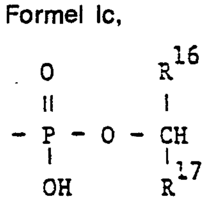

(Ic)

worin $R^{16}$ Wasserstoff und $R^{17}$ 2-Hydroxy- oder 1,2-Dihydroxyäthyl bedeuten, wobei mindestens eine Hydroxygruppe mit einem mindestens 7 Kohlenstoffatome aufweisenden Rest verestert oder veräthert ist, oder worin $R^{16}$ und $R^{17}$ unabhängig voneinander verestertes oder veräthertes Hydroxymethyl bedeuten, wobei die veresternden bzw. veräthernden Reste mindestens 7 Kohlenstoffatome aufweisen, stehen, oder $R^1$, $R^2$, $R^{12}$ und $R^{13}$ unabhängig voneinander Wasserstoff, vom Rest der Formel Ia, worin n für 1 steht, verschiedenes Acyl oder aliphatisch substituiertes Silyl, $R^3$, $R^4$, $R^5$, $R^7$ und $R^8$ unabhängig voneinander Wasserstoff oder Niederalkyl, $R^6$ Wasserstoff oder Niederalkyl, das unsubstituiert oder durch eine Gruppe der Formel Id,

$$—E—(Z^2—Y^2—X^4)_m—A^2 \quad \text{(Id)}$$

worin m für 0 oder 1, E für eine Gruppe der Formel —O—, —S— oder —N($R^{14}$)—, wobei $R^{14}$ die oben gegebene Bedeutung hat, $Z^2$ für Carbonyl oder Thiocarbonyl, $Y^2$ für unsubstituiertes oder substituiertes Alkylen, das durch Iminocarbonyl oder Oxycarbonyl unterbrochen sein kann, $X^4$ für eine Gruppe der Formel —O— oder —N($R^{14}$)—, wobei $R^{14}$ die oben gegebene Bedeutung hat, und $A^2$ für einen Rest der Formel Ib oder Ic stehen, durch freies oder veräthertes Hydroxy oder Mercapto, durch von einer Gruppe der Formel Id verschiedenes verestertes Hydroxy oder Mercapto, durch freies oder von einer Gruppe der Formel Id verschiedenes substituiertes Amino, durch freies, verestertes oder amidiertes Carboxy, oder durch Cycloalkyl, carbocyclisches Aryl oder stickstoffhaltiges Heteroaryl mit 5 bis 6 Ringgliedern im heterocyclischen Ring substituiert ist, oder $R^5$ und $R^6$ zusammen unsubstituiertes oder substituiertes 1,3- oder 1,4-Niederalkylen, $R^9$ und $R^{11}$ unabhängig voneinander einen Rest der Formel Ie,

$$—X^5—Y^3—X^6—A^3 \quad \text{(Ie)}$$

worin $X^5$ für eine Gruppe der Formel —O—, —S—, oder —N($R^{14}$)— und $X^6$ für eine Gruppe der Formel —O— oder —N($R^{14}$)—, wobei $R^{14}$ jeweils die oben gegebene Bedeutung hat, $Y^3$ für unsubstituiertes oder substituiertes Alkylen, das durch Iminocarbonyl oder Oxycarbonyl unterbrochen sein kann, und $A^3$ für einen Rest der Formel Ib oder Ic stehen, oder freies Hydroxy oder Mercapto, von einem Rest der Formel Ie verschiedenes veräthertes Hydroxy oder Mercapto, oder freies oder von einem Rest der Formel Ie verschiedenes substituiertes Amino und $R^{10}$ Wasserstoff oder freies, verestertes oder amidiertes Carboxy bedeuten, mit der Massgabe, dass das Präfix «Nieder» Reste bis und mit 7 C-Atomen bezeichnet und dass die Verbindungen der Formel I mindestens einen Rest $A^1$, $A^2$ oder $A^3$ aufweisen, und pharmazeutisch verwendbare Salze von solchen Verbindungen zur Herstellung von Arzneimitteln geeignet sind, die für die Anwendung an Warmblütern, insbesondere und einschliesslich des Menschen, zur Prophylaxe oder Therapie von durch Viren hervorgerufenen Krankheiten bestimmt sind.

Im Gegensatz zur Behandlung bakterieller Infektionen stehen zur Prophylaxe und Therapie von Virusinfektionen nur wenige und unzureichende Mittel zur Verfügung, so dass es zur Ueberwindung von Viruskrankheiten in fast allen Fällen nötig ist, dass der Organismus selbst genügend Abwehrkräfte besitzt.

Erfindungsgemäss wurde überraschenderweise gefunden, dass die obengenannten Hexopyranoseverbindungen der Formel I und ihre pharmazeutisch verwendbaren Salze sowohl zur Prophylaxe als auch zur Therapie von Virusinfektionen hervorragend geeignet sind, wie sich z. B. aus Tierversuchen, wie sie im Beispielteil exemplifiziert sind, ergibt. In diesen Tierversuchen werden Tiere, wie Mäuse oder Meerschweinchen, mit den verschiedensten Virusarten in einer Dosis, die für alle oder die grosse Mehrzahl der unbehandelten (Kontroll-) Tiere letal ist, z. B. $LD_{80-90}$, infiziert und der Infektionsverlauf bei den unbehandelten Kontrolltieren im Vergleich zu Tieren beobachtet, die vor, gleichzeitig mit oder nach der Infektion mit einer der obengenannten Hexopyranoseverbindungen oder einem Salz davon behandelt werden.

Dabei zeigt sich, dass die antivirale Wirkung der obengenannten Hexopyranoseverbindungen der Formel I und ihrer Salze von keiner bisher bekannten antiviralen Substanz beliebiger Struktur erreicht wird. Besonders bemerkenswert ist, dass ein prophylaktischer Effekt bei Verabreichung der Hexopyranoseverbindungen der Formel I schon mehrere Tage bis zu einigen, z. B. vier, Wochen vor der Infektion, und ein therapeutischer Effekt noch bei Verabreichung mehrere Tage, z. B. 1 Woche, nach der Infektion, eintritt.

Bemerkenswert und bisher einmalig ist auch das breite virale Spektrum, gegen das die obengenannten Verbindungen wirksam sind.

Die Hexopyranoseverbindungen der Formel I können insbesondere zur Propylaxe und Therapie von Krankheiten verwendet werden, die durch die nachstehend näher bezeichneten Viren hervorgerufen werden [zur Nomenklatur vgl. J.L. Melnick, Prog. med. Virol. 26, 214-232 (1980) und 28, 208-221 (1982)] :

DNA-Viren mit kubischer Symmetrie und nacktem Nuckleokapsid, DNA-Viren mit umhüllten Virion sowie RNA-Viren mit kubischer und solche mit helikaler Symmetrie des Kapsids.

Bevorzugterweise verwendet man die Verbindungen der Formel I im Falle von DNA-Viren mit umhüllten Virion und kubischer Symmetrie des Kapsids, im Falle von RNA-Viren mit kubischer Symmetrie des Kapsids und nacktem Virion und im Falle von RNA-Viren mit helikaler Symmetrie des Kapsids, in denen die Nukleokapsidhülle bei der Oberflächenmembran gelegen ist, aber auch im Falle von

Adenoviridae, Poxviridae und Coronaviridae, wie insbesondere menschlichen Coronaviren.

In erster Linie verwendet man die Verbindungen der Formel I im Falle von Herpesviridae, Picornaviridae und Myxoviren, aber auch im Falle von Mastadenoviren, wie insbesondere menschlichen Adenoviren, im Falle von Chordopoxvirinae, wie hauptsächlich Orthopoxviren, wie insbesondere z. B. Vacciniaviren, im Falle von Reoviridae, vornehmlich (insbesondere menschlichen) Rotaviren, sowie im Falle von Caliciviridae und Rhabdoviridae, wie in erster Linie Vesiculoviren des Menschen sowie von Pferden, Rindern und Schweinen.

Hauptsächlich verwendet man die Verbindungen der Formel I im Falle von Alphaherpesvirinae, wie Varicellaviren, z. B. menschlichen Varicella-zoster Viren, Rhinoviren, Cardioviren und Orthomyxoviridae, aber auch im Falle von Betaherpesvirinae, wie insbesondere menschlichen Cytomegaloviren, im Falle von Aphthoviren, in erster Linie Apthoviren von Paarhufern, wie hauptsächlich von Rindern, sowie im Falle von Paramyxoviridae, wie in erster Linie Pneumoviren, z. B. respiratorischen Syncitialviren des Menschen, und wie daneben Morbilliviren oder Paramyxoviren, wie Parainfluenzaviren, z. B. menschlichen Parainfluenzaviren, einschliesslich der Sendaiviren sowie im Falle von Arboviren oder Vesiculoviren, z. B. Vesicular stomatitis Viren.

In allererster Linie verwendet man die Verbindungen der Formel I im Falle von Simplexviren, z. B. menschlichen Herpes simplex Viren der Typen 1 und 2, im Falle von menschlichen Encephalomyocarditisviren, im Falle von Influenzaviren, wie hauptsächlich Influenza A und Influenza B Viren und ganz besonders im Falle der in den Beispielen genannten Viren.

Die Hexopyranoseverbindungen der Formel I können erfindungsgemäss verwendet werden, indem man sie enteral oder parenteral zusammen mit geeigneten Hilfs- oder Trägerstoffen appliziert. Bevorzugterweise appliziert man sie auf die Schleimhaut, z. B. intranasal, rektal, vaginal oder auf die Bindehaut des Auges, oder oral. Der antivirale Effekt tritt jedoch auch bei Applikation auf anderen Wegen, z. B. subkutan, intravenös, intramuskulär oder bei Applikation auf die normale Haut ein.

Die Dosierung des Wirkstoffs hängt u. a. von der Warmblüterspezies, der Abwehrlage des Organismus, der Applikationsweise und der Art des Virus ab. Die Dosis-Wirkungsbeziehung ist relativ schwach ausgeprägt.

Zur Vorbeugung appliziert man eine einmalige Dosis von etwa 0,01 mg bis etwa 25 mg, vorzugsweise 0,05 bis 7 mg, z. B. 0,5 mg, Wirkstoff an einen Warmblüter von etwa 70 kg Körpergewicht, z. B. den Menschen. Die prophylaktische Wirkung dieser Dosis erstreckt sich über mehrere Wochen. Bei Bedarf, z. B. in Zeiten erhöhter Ansteckungsgefahr, kann man die Verabreichung dieser Dosis wiederholen.

Die therapeutische Dosis für Warmblüter von etwa 70 kg Körpergewicht liegt zwischen 0,1 mg und 50 mg, vorzugsweise zwischen 1 und 10 mg, z. B. bei 5 mg, insbesondere bei oraler Applikation. Die Dosierung bei topischer, insbesondere intranasaler Applikation liegt bis zum Faktor 10 niedriger. Bei Bedarf kann man die Verabreichung der Hexopyranoseverbindungen der Formel I bis zum Eintritt einer Besserung der Erkrankung wiederholen. Normalerweise genügt jedoch eine einmalige Applikation.

Man verwendet die Hexopyranoseverbindungen der Formel I erfindungsgemäss in Form von pharmazeutischen Präparaten, die eine pharmakologisch wirksame Menge der Aktivsubstanz zusammen mit pharmazeutisch verwendbaren Trägerstoffen enthalten, die sich zur enteralen, z. B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z. B. Laktose, Dextrose, Sukrose, Mannitol, Sorbitol, Cellulose und/oder Glycerin, und/oder Schmiermitteln, z. B. Kieselerde, Talk, Stearinsäure oder Salze davon, wie Magnesium- oder Calciumstearat, und/oder Polyäthylenglykol, enthalten. Tabletten können ebenfalls Bindemittel, z. B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten, z. B. von Infusionslösungen verwenden. Solche Lösungen sind vorzugsweise isotonische wässrige Lösungen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz und/oder Emulgiermittel, Löslichkeitsvermittler, Salze zur Regulierung des osmotischen Druckes und/oder Puffer enthalten. Die vorliegenden pharmazeutischen Präparate werden in an sich bekannter Weise, z. B. mittels konventioneller Misch-, Granulier-, Dragier-, Lösungs- oder Lyophilisierungsverfahren, hergestellt und enthalten von etwa 0,001 % bis etwa 95 % des Wirkstoffs, wobei ein Wirkstoffgehalt von weniger als 1 % vornehmlich für topisch zu applizierende Präparate geeignet ist.

Pharmazeutische Präparate zur enteralen oder parenteralen Applikation, die eine wirksame Menge, jedoch weniger als 1 Gewichtsprozent einer Hexopyranoseverbindung der Formel I oder ein Salz davon zusammen mit einem pharmazeutischen Trägermaterial enthalten, sind neu und gehören ebenfalls zum Gegenstand der Erfindung.

Insbesondere betrifft die Erfindung solche pharmazeutischen Präparate, die eine Hexopyranoseverbindung der Formel I enthalten, die unten als für die erfindungsgemässe Verwendung bevorzugt

genannt ist.

Für die erfindungsgemässe Verwendung besonders geeignet sind die folgenden zur Verabreichung von Hexopyranoseverbindungen der Formel I nicht vorpublizierten Darreichungsformen : Crémes, Salben, Pasten oder ein Gel mit einem Wirkstoffgehalt von 0,001 Gewichtsprozent bis ausschliesslich 1 Gewichtsprozent, hauptsächlich von 0,001 % bis 0,9 %, insbesondere von 0,01 % bis 0,1 %, z. B. 0,05 %, z. B. Salben zur intranasalen Applikation, Vaginal- oder Rektalzäpfchen oder Lippenstifte, wässerige Lösungen mit einem Wirkstoffgehalt von 0,001 Gewichtsprozent bis ausschliesslich 1 Gewichtsprozent, hauptsächlich von 0,001 % bis 0,9 %, insbesondere von 0,05 % bis 0,5 %, z. B. 0,1 %, vorzugsweise isotonische, sterile und physiologisch verträgliche Lösungen, z. B. Augentropfen, vorzugsweise in Mikrobehältern zur einmaligen Verwendung, oder Sprays zur Anwendung im Mund- und Rachenraum, oder Tabletten oder Kapseln mit einem Wirkstoffgehalt von 0,1 bis ausschliesslich 1 Gewichtsprozent, z. B. bis 0,9 %.

Besonders geeignet sind die in den Beispielen beschriebenen pharmazeutischen Präparate.

Crémes sind Oel-in-Wasser-Emulsionen, die mehr als 50 % Wasser aufweisen. Als ölige Grundlage verwendet man in erster Linie Fettalkohole, z. B. Lauryl-, Cetyl- oder Stearylalkohol, Fettsäuren, z. B. Palmitin- oder Stearinsäure, flüssige bis feste Wachse, z. B. Isopropylmyristat, Wollwachs oder Bienenwachs, und/oder Kohlenwasserstoffe, z. B. Vaseline® (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z. B. Fettsäureester von Polyalkoholen oder Aethylenoxidaddukte davon, wie Polyglycerinfettsäureester oder Polyoxyäthylensorbitan-fettsäurester (Tweens®), ferner Polyoxyäthylen-fettalkoholäther oder -fettsäureester, oder entsprechende ionische Emulgatoren, wie Alkalimetallsalze von Fettalkoholsulfaten, z. B. Natriumlaurylsulfat, Natriumcetylsulfat oder Natriumstearylsulfat, die man üblicherweise in Gegenwart von Fettalkoholen, z. B. Cetylalkohol oder Stearylalkohol, verwendet. Zusätze zur Wasserphase sind u. a. Mittel, welche die Austrocknung der Crémes vermindern, z. B. Polyalkohol, wie Glycerin, Sorbit, Propylenglykol und/oder Polyäthylenglykole, ferner Konservierungsmittel und Riechstoffe.

Salben sind Wasser in-Oel-Emulsionen, die bis zu 70 %, vorzugsweise jedoch von etwa 20 % bis etwa 50 % Wasser oder wässrige Phase enthalten. Als Fettphase kommen in erster Linie Kohlenwasserstoffe, z. B. Vaseline®, Paraffinöl und/oder Hartparaffine in Frage, die zur Verbesserung des Wasserbindungsvermögens vorzugsweise geeignete Hydroxyverbindungen, wie Fettalkohole oder Ester davon, z. B. Cetylalkohol oder Wollwachsalkohole, bzw. Wollwachs, enthalten. Emulgatoren sind entsprechende lipophile Substanzen, wie Sorbitan-fettsäureester (Spans), z. B. Sorbitanoleat und/oder Sorbitanisostearat. Zusätze zur Wasserphase sind z. B. Feuchthaltungsmittel, wie Polyalkohole, z. B. Glycerin, Propylenglykol, Sorbit und/oder Polyäthylenglykol, wie Konservierungsmittel und Riechstoffe.

Fettsalben sind wasserfrei und enthalten als Grundlage insbesondere Kohlenwasserstoffe, z. B. Paraffin, Vaseline® und/oder flüssige Paraffine, ferner natürliche oder partialsynthetische Fette, z. B. Kokosfettsäuretriglycerid, oder vorzugsweise gehärtete Oele, z. B. hydriertes Erdnuss- oder Rizinusöl, ferner Fettsäurepartialester des Glycerins, z. B. Glycerinmono- und -distearat, sowie z. B. die im Zusammenhang mit den Salben erwähnten, die Wasseraufnahmefähigkeit steigernden Fettalkohole, Emulgatoren und/oder Zusätze.

Pasten sind Crèmes und Salben mit sekretabsorbierenden Puderbestandteilen, wie Metalloxiden, z. B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z. B. Dichlordifluormethan und Dichlortetrafluoräthan, als Treibmittel verwendet werden. Als Oelphase verwendet man u. a. Kohlenwasserstoffe, z. B. Paraffinöl, Fettalkohole, z. B. Cetylalkohol, Fettsäureester, z. B. Isopropylmyristat, und/oder andere Wachse. Als Emulgatoren verwendet man u. a. Gemische von solchen mit vorwiegend hydrophilen Eigenschaften, wie Polyoxyäthylen-sorbitan-fettsäureester (Tweens®), und solchen mit vorwiegend lipophilen Eigenschaften, wie Sorbitanfettsäureester (Spans®). Dazu kommen die üblichen Zusätze, wie Konservierungsmittel.

Tinkturen und Lösungen weisen meistens eine wässrig-äthanolische Grundlage auf, der u. a. Polyalkohole, z. B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglycolen, d. h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z. B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst ; bei Verarbeitung als Suspension wird er nach der Emulgierung mit einem Teil der Grundlage vermischt und dann dem Rest der Formulierung beigegeben.

Die oben zur Definition der Verbindungen der Formel I verwendeten Begriffe haben im Rahmen dieser Anmeldung vorzugsweise die folgenden Bedeutungen :

Acyl, z. B. als $R^1$, $R^2$, $R^{12}$ und $R^{13}$, bedeutet in erster Linie den Acylrest einer organischen Carbonsäure, insbesondere einer aliphatischen, ferner einer cycloaliphatischen, cycloaliphatisch-aliphatischen, aromatischen oder araliphatischen Carbonsäure, die z. B. bis zu 90 Kohlenstoffatome aufweisen kann.

Aliphatische Carbonsäuren sind u. a. unsubstituierte oder, z. B. durch Hydroxy oder veräthertes oder verestertes Hydroxy, wie Niederalkoxy oder Niederalkanoyloxy, ferner unsubstituiertes oder substituiertes Amino, wie Niederalkylamino, Diniederalkylamino oder Acylamino, z. B. Alkanoylamino, substituierte Alkancarbonsäuren, sowie entsprechende Alken- oder Alkincarbonsäuren, die eine oder mehrere Doppel- bzw. Dreifachbindungen aufweisen können. Diese Säuren können z. B. bis zu 90 Kohlenstoffatome enthalten, wobei $R^2$ als Rest einer aliphatischen Carbonsäure, falls $X^1$ für eine Gruppe der Formel $-N(R^{14})-$ steht, vorzugsweise den Acylrest einer unsubstituierten oder Hydroxy-substituierten Niederalkancarbonsäure bedeutet.

Cycloaliphatische Carbonsäuren können mono- oder polycyclisch sein und als cycloaliphatischen Rest unsubstituiertes oder, z. B. durch Hydroxy, substituiertes mono- oder polycyclisches Cycloalkyl, sowie entsprechendes Cycloalkenyl enthalten.

In cycloaliphatisch-aliphatischen Resten haben der cycloaliphatische und der aliphatische Teil die oben gegebenen Bedeutungen ; solche Reste sind insbesondere mono- oder polycyclisches Cycloalkylnieder-Alkyl Aromatische und araliphatische Carbonsäuren sind u. a. unsubstituierte oder, z. B. durch Niederalkyl, Hydroxy, Niederalkoxy oder Halogen, substituierte Benzoe- oder Phenylniederalkancarbonsäuren.

Aliphatisch substituiertes Silyl ist z. B. Triniederalkylsilyl.

Substituenten von Alkylen, das durch die Reste $Y^1$, $Y^2$ bzw. $Y^3$ repräsentiert wird, sind u. a. Hydroxy, verestertes oder veräthertes Hydroxy, wie Acyloxy, z. B. Niederalkanoyloxy, oder Niederalkoxy, Amino oder substituiertes Amino, wie Niederalkylamino, Diniederalkylamino oder Acylamino, z. B. Niederalkanoylamino. In dem von Iminocarbonyl oder Oxycarbonyl unterbrochenen Alkylen können eine oder mehrere, z. B. zwei, solche Gruppierungen vorkommen, wobei sie als solche der Formel $-N(R^{14})-C(=O)-$ bzw. $-O-C(=O)-$, sowie als solche der Formel $-C(=O)-N(R^{14})-$ bzw. $-C(=O)-O-$ vorliegen können, und $R^{14}$ die oben gegebene Bedeutung hat und vorzugsweise für Wasserstoff steht. Ein durch die Gruppen $R^5$ und $R^6$ gebildetes Alkylen mit 3 bis 4 Kohlenstoffatomen in der Kette kann z. B. durch Hydroxy, das, z. B. durch eine Gruppe der Formel Ia, acyliert sein kann, substituiert sein.

Ein die Gruppe $R^{15}$ darstellender oder ein, eine Hydroxygruppe in einem Rest $R^{16}$ und $R^{17}$ veräthernder aliphatischer Rest mit mindestens 7 Kohlenstoffatomen ist in erster Linie ein entsprechender unsubstituierter oder substituierter Alkylrest, kann aber auch für einen entsprechenden ungesättigten Rest, wie einen unsubstituierten oder substituierten, eine oder mehrere Doppelbindungen aufweisenden Alkenylrest stehen, wobei solche Reste z. B. von 7 bis und mit 90, vorzugsweise von 7 bis und 30 Kohlenstoffatome aufweisen. Substituenten von solchen aliphatischen Resten sind z. B. Hydroxy, veräthertes oder verestertes Hydroxy, wie Niederalkoxy oder Niederalkanoyloxy und/oder unsubstituiertes oder substituiertes Amino, wie Niederalkylamino, Diniederalkylamino oder Alkanoylamino.

Ein entsprechender cycloaliphatischer Rest, der die Gruppe $R^{15}$ oder den eine Hydroxygruppe in den Resten $R^{16}$ bzw. $R^{17}$ veräthernden Rest darstellt, ist insbesondere mono- oder polycyclisches Cycloalkyl, ferner entsprechendes Cycloalkenyl, das eine oder mehrere Doppelbindungen aufweisen kann. Dabei enthalten solche Reste mindestens 7, vorzugsweise 7 bis 30 Kohlenstoffatome, und können zudem, z. B. durch Hydroxy, veräthertes oder verestertes Hydroxy, wie Niederalkoxy oder Niederalkanoyloxy, oder unsubstituiertes oder substituiertes Amino, wie Niederalkylamino, Diniederalkylamino oder Alkanoylamino substituiert sein.

Veräthertes Hydroxy oder substituiertes Amino als Rest $R^9$ oder $R^{11}$ ist z. B. Niederalkoxy bzw. z. B. Niederalkylamino, worin Niederalkyl substituiert sein kann.

Die eine Hydroxygruppe in den Resten $R^{16}$ bzw. $R^{17}$ veresternden Reste sind in erster Linie Acylreste von organischen Carbonsäuren, insbesondere der obengenannten aliphatischen, sowie cycloaliphatischen, cycloaliphatisch-aliphatischen, aromatischen oder araliphatischen Carbonsäuren, vorzugsweise mit 7 bis 90 Kohlenstoffatomen.

Veräthertes bzw. vom Rest der Formel Id verschiedenes verestertes Hydroxy oder Mercapto als Substituent von Niederalkyl $R^6$ ist z. B. Niederalkoxy, Acyloxy, wie Alkanoyloxy, worin Alkanoyl bis zu 90, z. B. von 7 bis 30, Kohlenstoffatome enthalten und gegebenenfalls, z. B. durch Hydroxy, substituiert sein kann, oder Halogen, ferner Niederalkylthio oder Acylthio, wie Alkanoylthio, worin Alkanoyl bis zu 90, z. B. von 7 bis 30, Kohlenstoffatome enthält. Vom Rest der Formel Id verschiedenes substituiertes Amino als Substituent einer Niederalkylgruppe $R^6$ ist z. B. Niederalkylamino, Guanylamino oder Acylamino, wie Alkanoylamino, worin Alkanoyl bis zu 90, z. B. zu 30, Kohlenstoffatome enthalten kann. Verestertes Carboxy als Substituent eines Niederalkylrestes $R^6$ ist vorzugsweise ein mit einem aliphatischen Rest, wie Alkyl mit bis zu 30 Kohlenstoffatomen, verestertes Carboxy, d. h. z. B. entsprechendes Alkoxycarbonyl, während entsprechendes amidiertes Carboxy z. B. Aminocarbonyl oder Niederalkylaminocarbonyl ist, worin Niederalkyl z. B. durch Carboxy, Alkoxycarbonyl oder Aminocarbonyl substituiert sein kann. Verestertes bzw. amidiertes Carboxy im Rest $R^6$ kann auch einen Rest der Formel

$$-\overset{\displaystyle O}{\overset{\|}{C}}-E-(Y^2-X^4)_m-A^2 \qquad \text{(Ida)}$$

darstellen, worin m, E, $Y^2$, $X^4$ und $A^2$ die oben gegebenen Bedeutungen haben.

Aryl als Substituent einer Niederalkylgruppe $R^6$ ist in erster Linie unsubstituiertes oder, z. B. durch Niederalkyl, Hydroxy oder veräthertes oder verestertes Hydroxy, wie Niederalkoxy oder Halogen, substituiertes Phenyl, während stickstoffhaltiges Heteroaryl mit 5 bis 6 Ringgliedern im heterocyclischen Ring als entsprechender Substituent von $R^6$ mono- oder bicyclisches, ein oder zwei Stickstoffatome als Ringglieder enthaltendes Heteroaryl darstellt.

Veräthertes Mercapto als Rest $R^9$ oder $R^{11}$ ist insbesondere Niederalkylthio, während in einem Niederalkylaminorest $R^9$ oder $R^{11}$ die Niederalkylgruppe z. B. durch Carboxy, Niederalkoxycarbonyl oder Aminocarbonyl substituiert sein kann.

Verestertes Carboxy $R^{10}$ ist insbesondere Niederalkoxycarbonyl, während amidiertes Carboxyl $R^{10}$ Carbamoyl oder N-Niederalkyl-carbamoyl sein kann, worin Niederalkyl z. B durch Carboxy, Niederalkoxycarbonyl oder Aminocarbonyl substituiert sein kann.

Die oben verwendeten Allgemeinbegriffe haben im Zusammenhang mit der vorliegenden Beschreibung die folgenden Bedeutungen, wobei mit « nieder » bezeichnete Reste und Verbindungen bis und mit 7, vorzugsweise bis und mit 4 Kohlenstoffatome enthalten :

Alkyl ist z. B. Niederalkyl, wie Methyl, Aethyl, n-Propyl, Isopropyl, n-Butyl, Tributyl, sek.-Butyl oder tert.-Butyl, sowie n-Pentyl, Neopentyl, n-Hexyl oder n-Heptyl, oder Höheralkyl, wie geradkettiges oder verzweigtes Octyl, Nonyl, Decyl, Undecyl, Dodecyl, Tridecyl, Tetradecyl, Pentadecyl, Hexadecyl, Heptadecyl, Nonadecyl oder Heneicosyl, sowie Höheralkyl der Triacontyl-, Tetracontyl-, Pentacontyl-, Hexacontyl-, Heptacontyl-, Octacontyl- oder Nonacontylserien.

Niederalkoxy ist z. B. Methoxy, Aethoxy, n-Propyloxy, Isopropyloxy, n-Butyloxy, Isobutyloxy oder tert.-Butyloxy.

Alkanoyloxy ist Nieder- oder Höheralkanoyloxy, wobei Niederalkanoyloxy z. B. Formyloxy, Acetoxy, Propionyloxy oder Butyryloxy ist, während Höheralkanoyloxy z. B. Lauryloxy, Myristinoyloxy, Palmitoyloxy, Stearoyloxy oder Behenoyloxy ist. Durch Hydroxy substituiertes Alkanoyloxy, z. B. Höheralkanoyloxy, ist u. a. Mycoloyloxy.

Niederalkylamino ist z. B. Methylamino, Aethylamino, n-Propylamino oder Isopropylamino. Diniederalkyl-amino ist z. B. Dimethylamino, Diäthylamino oder Di-isopropylamino. Alkanoylamino ist Niederalkanoylamino, z. B. Formylamino, Acetylamino oder Propionylamino, oder Höheralkanoylamino, z. B. Laurylamino, Palmitoylamino, Stearoylamino oder Behenoylamino.

Eine Alkancarbonsäure ist z. B. eine Niederalkancarbonsäure, wie Essigsäure, Propionsäure, Buttersäure oder Capronsäure, oder eine Höheralkancarbonsäure, wie Laurinsäure, Myristinsäure, Palmitinsäure, Stearinsäure oder Behensäure, während z. B. eine durch Hydroxy substituierte Alkansäure u. a. Mycolsäure sein kann.

Alken- und Alkincarbonsäuren sind u. a. Niederalken- bzw. Niederalkincarbonsäuren, wie Acrylsäure, Crotonsäure oder Tetrolsäure, oder Höheralken- bzw. Höheralkincarbonsäuren, wie Undecylensäure, Oelsäure oder Elaidinsäure. Der Acylrest einer Niederalkancarbonsäure, welcher die Gruppe $R^2$ darstellt, falls $X^1$ für den Rest der Formel $—N(R^{14})—$ steht, ist in erster Linie Acetyl oder Hydroxyacetyl, sowie Propionyl.

Cycloalkyl ist z. B. Cyclopentyl, Cyclohexyl oder Adamantyl, während Cycloalkenyl z. B. 1-Cyclohexenyl, und Cycloalkylniederalkyl z. B. 3-Cholanylmethyl oder der Acylrest der Cholansäure sein kann.

Phenylniederalkancarbonsäuren sind z. B. Phenylessigsäure oder Phenylpropionsäure, die, z. B. wie angegeben, substituiert sein können.

Halogen ist vorzugsweise Halogen mit einer Ordnungszahl bis zu 35 und steht besonders für Chlor, ferner auch für Fluor oder Brom.

Triniederalkylsilyl ist in erster Linie Trimethylsilyl.

Alkylen ist geradkettig oder verzweigt und ist insbesondere Niederalkylen, z. B. Methylen, Aethylen, 1,2-Propylen, 1,3-Propylen oder 1,6-Hexylen, ferner auch Höheralkylen, wie 1,11-Undecylen.

Alkenyl ist Niederalkenyl, z. B. Allyl oder Methallyl, oder Höheralkenyl, z. B. Decenyl.

Niederalkylthio ist z. B. Methylthio oder Aethylthio.

In einem Alkanoylthiorest bedeutet der Alkanoylrest Niederalkanoyl, z. B. Acetyl, Propionyl, Butyryl oder Hexanoyl, kann aber auch Höheralkanoyl, z. B. Lauryl, Myristinoyl, Palmitoyl, Stearoyl oder Behenoyl, sein.

Alkoxycarbonyl ist Niederalkoxycarbonyl, z. B. Methoxycarbonyl, Aethoxycarbonyl oder tert.-Butyloxycarbonyl, oder Höheralkoxycarbonyl, z. B. Dodecyloxycarbonyl, Tetradecyloxycarbonyl, Hexadecyloxycarbonyl oder Heneicosyloxycarbonyl.

Niederalkylaminocarbonyl ist z. B. Methylaminocarbonyl oder Aethylaminocarbonyl, ferner Carboxy-, Niederalkoxycarbonyl- oder Carbamoylniederalkylaminocarbonyl, wie Carboxymethylaminocarbonyl, 1-Carboxyäthylaminocarbonyl, Methoxycarbonylmethylaminocarbonyl oder Carbamoylmethylaminocarbonyl.

Stickstoffhaltiges Heteroaryl mit 5 bis 6 Ringgliedern im heterocyclischen Ring ist z. B. Imidazolyl, wie 4-Imidazolyl, oder Indolyl, wie 3-Indolyl.

Die Hexopyranose-Verbindungen der Formel I können in Form von Isomerengemischen oder reinen Isomeren vorliegen.

Die gegebenenfalls substituierte 1-Hydroxygruppe der Formel $—O—R^1$ kann die α- oder die β-

Konfiguration haben ; die neuen Verbindungen der Formel I können jedoch auch als Gemisch der 1-α- und 1-β-Isomeren vorliegen.

In den Verbindungen der Formel I kann das über ein Sauerstoffatom an den Phosphor gebundene Proton leicht durch ein Kation ersetzt werden, d. h. die Verbindungen bilden Salze. Dabei können die Verbindungen der Formel I in Salzform oder in Form eines Gemisches der freien Verbindungen und ihrer Salze vorliegen. Es sind dies in erster Linie Metall- oder Ammoniumsalze, wie Alkalimetall- und Erdalkalimetall-, z. B. Natrium-, Kalium-, Magnesium- oder Calciumsalze, sowie Ammoniumsalze oder Salze mit geeigneten organischen Aminen, wie Niederalkylaminen, z. B. Triäthylamin. Verbindungen der Formel I mit basischen Gruppen z. B. Aminogruppen, liegen als innere Salze vor, können aber, wenn mehr basische als saure Gruppen in einem Molekül der Formel I vorhanden sind, zusätzlich auch Säureadditionssalze mit externen Säuren bilden, wie Salze mit anorganischen Säuren, wie Mineralsäuren, z. B. Salz-, Schwefel- oder Phosphorsäure, oder organischen Carbon- oder Sulfonsäuren, z. B. Essig-, Malein-, Fumar-, Wein-, Zitronen-, Methansulfon- oder 4-Toluolsulfonsäure. Zur therapeutischen Anwendung gelangen nur die pharmazeutisch verwendbaren, nicht-toxischen Salze.

Die vorliegende Erfindung betrifft insbesondere die Verwendung von (a) Verbindungen der Formel I, worin $X^1$ und $X^2$ die oben gegebenen Bedeutungen haben, $R^1$ für den Rest der Formel Ia steht, worin n, $Z^1$, $Y^1$, $X^3$ und $A^1$ die oben gegebenen Bedeutungen haben, $R^2$, $R^{12}$ und $R^{13}$ unabhängig voneinander Wasserstoff, ein vom Rest der Formel Ia, worin n für 1 steht, verschiedenes Acyl oder aliphatisch substituiertes Silyl darstellen, $R^3$, $R^4$, $R^5$, $R^7$, $R^8$ und $R^{10}$ die oben gegebenen Bedeutungen haben, $R^6$ für Wasserstoff, unsubstituiertes oder durch freies oder veräthertes Hydroxy oder Mercapto, von einer entsprechenden Gruppe der Formel Id verschiedenes, verestertes Hydroxy oder Mercapto, freies oder von einer entsprechenden Gruppe der Formel Id verschiedenes, substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie verschiedenes, veräthertes Hydroxy oder Mercapto oder freies oder von einem Rest der Formel Ie verschiedenes, substituiertes Amino bedeuten, wobei freie funktionelle Gruppen in geschützter Form vorliegen können, und von pharmazeutisch verwendbaren Salzen von solchen Verbindungen, (b) Verbindungen der Formel I, worin $X^1$ und $X^2$ die oben gegebenen Bedeutungen haben, $R^2$ für den Rest der Formel Ia steht, worin n, $Z^1$, $Y^1$, $Y^3$ und $A^1$ die oben gegebenen Bedeutungen haben, $R^1$, $R^{12}$ und $R^{13}$ unabhängig voneinander Wasserstoff, ein vom Rest der Formel Ia, worin n für 1 steht, verschiedenes Acyl oder aliphatisch substituiertes Silyl darstellen, $R^3$, $R^4$, $R^5$, $R^7$, $R^8$ und $R^{10}$ die oben gegebenen Bedeutungen haben, $R^6$ für Wasserstoff, unsubstituiertes oder durch freies oder veräthertes Hydroxy oder Mercapto, von einer entsprechenden Gruppe der Formel Id verschiedenes, verestertes Hydroxy oder Mercapto, freies oder von einer entsprechenden Gruppe der Formel Id verschiedenes substituiertes Amino, freies, verestertes oder amidiertes Carboxy, Cycloalkyl, carbocyclisches Aryl oder stickstoffhaltiges Heteroaryl mit 5 bis 6 Ringgliedern im heterocyclischen Ring substituiertes Niederalkyl steht, oder $R^5$ und $R^6$ zusammen unsubstituiertes oder substituiertes 1,3- oder 1,4-Niederalkylen darstellen, und worin $R^9$ und $R^{11}$ unabhängig voneinander freies Hydroxy oder Mercapto, von einem Rest der Formel Ie verschiedenes, veräthertes Hydroxy oder Mercapto oder freies oder von einem Rest der Formel Ie verschiedenes substituiertes Amino bedeuten, wobei freie funktionelle Gruppen in geschützter Form vorliegen können, und von pharmazeutisch verwendbaren Salzen von solchen Verbindungen, (c) Verbindungen der Formel I, worin $X^1$ und $X^2$ die oben gegebenen Bedeutungen haben, $R^{13}$ für den Rest der Formel Ia steht, worin n, $Z^1$, $Y^1$, $X^3$ und $A^1$ die oben gegebenen Bedeutungen haben, $R^1$, $R^2$ und $R^{12}$ unabhängig voneinander Wasserstoff, ein vom Rest der Formel Ia, worin n für 1 steht, verschiedenes Acyl oder aliphatisch substituiertes Silyl darstellen, $R^3$, $R^4$, $R^5$, $R^7$, $R^8$ und $R^{10}$ die oben gegebenen Bedeutungen haben, $R^6$ für Wasserstoff, unsubstituiertes oder durch freies oder veräthertes Hydroxy oder Mercapto, von einer entsprechenden Gruppe der Formel Id verschiedenes, verestertes Hydroxy oder Mercapto, freies oder von einer entsprechenden Gruppe der Formel Id verschiedenes substituiertes Amino, freies, verestertes oder amidiertes Carboxy, Cycloalkyl, carbocyclisches Aryl oder stickstoffhaltiges Heteroalkyl mit 5 bis 6 Ringgliedern im heterocyclischen Ring substituiertes Niederalkyl steht, oder $R^5$ und $R^6$ zusammen unsubstituiertes oder substituiertes Niederalkylen mit 3 bis 4 Kohlenstoffatomen in der Kette darstellen, und worin $R^9$ und $R^{11}$ unabhängig voneinander freies Hydroxy oder Mercapto, von einem Rest der Formel Ie verschiedenes veräthertes Hydroxy oder Mercapto oder freies oder von einem Rest der Formel Ie verschiedenes substituiertes Amino bedeuten, wobei freie funktionelle Gruppen in geschützter Form vorliegen können, und von pharmazeutisch verwendbaren Salzen von solchen Verbindungen, (d) Verbindungen der Formel I, worin $X^1$ und $X^2$ die oben gegebenen Bedeutungen haben, $R^1$, $R^2$, $R^{12}$ und $R^{13}$ unabhängig voneinander Wasserstoff, vom Rest der Formel Ia, worin n für 1 steht, verschiedenes Acyl oder aliphatisch substituiertes Silyl darstellen, $R^3$, $R^4$, $R^5$, $R^7$, $R^8$ und $R^{10}$ die oben gegebenen Bedeutungen haben, $R^6$ für durch den Rest der Formel Id substituiertes Niederalkyl steht, worin m, E, $Z^2$, $Y^2$, $X^4$ und $A^2$ die oben gegebenen Bedeutungen haben oder durch die Gruppe der Formel Ida substituiertes Niederalkyl bedeutet, worin m, E, $Y^2$, $X^4$ und $A^2$ die oben gegebenen Bedeutungen haben, und worin $R^9$ und $R^{11}$ unabhängig voneinander freies Hydroxy oder Mercapto, von einem Rest der Formel Ie verschiedenes veräthertes Hydroxy oder Mercapto, freies oder von einem Rest der Formel Ie

verschiedenes substituiertes Amino bedeuten, wobei freie funktionelle Gruppen in geschützter Form vorliegen können, und von pharmazeutisch verwendbaren Salzen von solchen Verbindungen, sowie neue pharmazeutische Präparate, die diese Verbindungen enthalten.

Die Erfindung umfasst in erster Linie die Verwendung von D-Galacto-, D-Manno- oder insbesondere D-Glucopyranose Verbindungen der Formel I, worin $X^1$ und $X^2$ die oben gegebenen Bedeutungen haben, $R^1$, $R^2$, $R^{12}$ und $R^{13}$ unabhängig voneinander für Wasserstoff, den Acylrest einer aliphatischen, cycloaliphatische, aromatischen oder araliphatischen Carbonsäure, insbesondere einer unsubstituierten oder z. B. durch Hydroxy, Amino und/oder Alkanoylamino, wie Höheralkanoylamino, substituierten Alkancarbonsäure, mit bis zu 90 Kohlenstoffatomen, eine Tri-niederalkyl-silylgruppe oder einen Rest der Formel Ia stehen, worin n und $X^3$ die oben gegebenen Bedeutungen haben, $Z^1$ für Thiocarbonyl oder vorzugsweise Carbonyl steht, $Y^1$ Niederalkylen bedeutet, das durch Iminocarbonyl oder Oxycarbonyl unterbrochen sein kann, und $A^1$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder mit einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, $R^3$, $R^4$, $R^5$, $R^7$ und $R^8$ die oben gegebenen Bedeutungen haben, $R^6$ Wasserstoff oder Niederalkyl bedeutet, das unsubstituiert oder durch einen Rest der Formel Id, worin m, E und $X^4$ die oben gegebenen Bedeutungen haben, $Z^2$ für Thiocarbonyl oder insbesondere Carbonyl steht, $Y^2$ Niederalkylen bedeutet, das durch Iminocarbonyl oder Oxycarbonyl unterbrochen sein kann, und $A^2$ für einen Rest der Formel Ib oder Ic, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, durch Hydroxy oder Mercapto, durch mit einem bis zu 90 Kohlenstoffatome enthaltenden aliphatischen Rest veräthertes Hydroxy oder Mercapto, durch mit einem bis zu 90 Kohlenstoffatome aufweisenden aliphatischen Acylrest verestertes Hydroxy oder Mercapto, der von der Gruppe der Formel Id verschieden ist, durch Amino, durch mit einem bis zu 90 Kohlenstoffatome enthaltenden Acylrest substituiertes Amino, der von einem Rest der Formel Id verschieden ist, durch freies Carboxy, Niederalkoxycarbonyl, Carbamoyl, Niederalkylaminocarbonyl, Carboxyniederalkylaminocarbonyl oder amidiertes Carboxyl der Formel Ida, unsubstituiertes oder durch Hydroxy, Niederalkoxy oder Halogen substituiertes Phenyl oder durch Imidazolyl oder Indolyl substituiert ist, oder $R^5$ und $R^6$ zusammen 1,3- oder 1,4-Niederalkylen bedeuten, $R^9$ und $R^{11}$ unabhängig voneinander Hydroxy, Niederalkoxy, Amino, Niederalkylamino, Carboxyniederalkylamino, oder einen Rest der Formel Ie bedeuten, worin $X^5$ und $X^6$ die oben gegebenen Bedeutungen haben, $Y^3$ Niederalkylen bedeutet, das durch Iminocarbonyl oder Oxycarbonyl unterbrochen sein kann, und $A^3$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, und $R^{10}$ für Wasserstoff, Carboxy, Niederalkoxycarbonyl, Carbamoyl, Niederalkylaminocarbonyl oder Carboxyniederalkylaminocarbonyl steht, wobei freie funktionelle Gruppen in geschützter Form vorliegen können, mit der Massgabe, dass die Verbindungen der Formel I mindestens einen Rest $A^1$, $A^2$ oder $A^3$ aufweisen, und von pharmazeutisch verwendbaren Salzen von solchen Verbindungen sowie neue pharmazeutische Präparate, die diese Verbindungen enthalten.

Die Erfindung betrifft in erster Linie e) die Verwendung der unter (a), (b), (c) und (d) genannten D-Galacto-, D-Manno- oder insbesondere D-Glucopyranose - Verbindungen der Formel I, worin einer, mehrere oder alle der Reste $X^1$, $X^2$, $R^1$, $R^2$, $R^3$, $R^4$, $R^5$, $R^6$, $R^7$, $R^8$, $R^9$, $R^{10}$, $R^{11}$, $R^{12}$ und $R^{13}$ die im vorangegangenen Paragraphen gegebenen Bedeutungen haben, und von pharmazeutisch verwendbaren Salzen von solchen Verbindungen.

Die Erfindung betrifft in erster Linie f) die Verwendung von D-Gluco-pyranose-Verbindungen der Formel If,

(Siehe Formel Seite 10 f.)

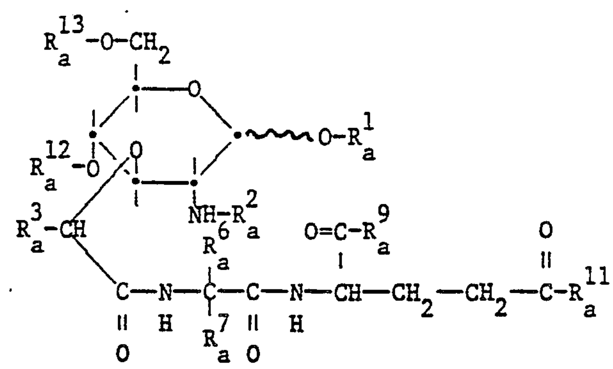

(If)

worin $R_a^1$ Wasserstoff, Niederalkanoyl oder die Gruppe der Formel Ia bedeutet, worin n für 1 steht, $Z^1$ Carbonyl bedeutet, $Y^1$ Niederalkylen darstellt, das durch Iminocarbonyl unterbrochen sein kann, $X^3$ für eine Gruppe der Formel —O— oder —NH— steht, und $A^1$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden Alkylrest, der unsubstituiert oder durch Hydroxy, Amino und/oder Alkanoylamino mit bis zu 30 Kohlenstoffatomen substituiert ist, veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, $R_a^2$ Niederalkanoyl, Hydroxyniederalkanoyl, Benzoyl oder die Gruppe der Formel Ia bedeutet, worin n, $Z^1$, $Y^1$, $X^3$ und $A^1$ die oben gegebenen Bedeutungen haben, $R_a^{12}$ Wasserstoff oder Niederalkanoyl darstellt, $R_a^{13}$ Wasserstoff, Alkanoyl oder Hydroxyalkanoyl mit bis zu 90 Kohlenstoffatomen, Alkanoylaminoalkanoyl mit bis zu 30 Kohlenstoffatomen oder die Gruppe der Formel Ia bedeutet, worin n, $Z^1$, $Y^1$, $X^3$ und $A^1$ die oben gegebenen Bedeutungen haben, $R_a^3$ und $R_a^7$ für Wasserstoff oder Methyl stehen, $R_a^6$ Wasserstoff, unsubstituiertes oder durch freies Hydroxy oder Mercapto, Niederalkoxy, Niederalkylthio, Alkanoyloxy oder Hydroxyalkanoyloxy mit bis zu 90 Kohlenstoffatomen, Phenyl, Imidazolyl, Indolyl oder durch eine Gruppe der Formel Id substituiertes Niederalkyl bedeutet, worin m für 1 steht, E für eine Gruppe der Formel —O— oder —S— steht, $Z^2$ Carbonyl bedeutet, $Y^2$ Niederalkylen darstellt, das durch Iminocarbonyl unterbrochen sein kann, $X^4$ für eine Gruppe der Formel —O— steht, und $A^2$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem, 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, und jeder der Reste $R_a^9$ und $R_a^{11}$ unabhängig voneinander für Hydroxy, Niederalkoxy, Amino, Niederalkylamino, Carboxyniederalkylamino oder einen Rest der Formel Ie stehen, worin $X^5$ für eine Gruppe der Formel —O— oder —NH— steht, $Y^3$ Niederalkylen darstellt, das durch Iminocarbonyl unterbrochen sein kann, $X^6$ für eine Gruppe der Formel —O— steht und $A^3$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten

Alkanoylrest verestert ist, $R_a^9$ vorzugsweise für eine der Aminogruppen und $R_a^{11}$ vorzugsweise für Hydroxy stehen, mit der Massgabe, dass die Verbindungen mindestens einen, und vorzugsweise nur einen, Rest $A^1$, $A^2$ bzw. $A^3$ aufweisen, und von pharmazeutisch verwendbaren Salzen davon sowie neue pharmazeutische Präparate, die diese Verbindungen enthalten.

Die Erfindung betrifft in erster Linie g) die Verwendung der unter (a), (b), (c) und (d) genannten D-Glucopyranose-Verbindungen der Formel I, worin $X^1$ für die Gruppe der Formel —NH—, $X^2$ für die Gruppe der Formel —O—, $R^4$, $R^5$, $R^8$ und $R^{10}$ für Wasserstoff stehen, und $R^1$, $R^2$, $R^3$, $R^6$, $R^7$, $R^9$, $R^{11}$, $R^{12}$ und $R^{13}$ die im vorangegangenen Abschnitt für die Reste $R_a^1$, $R_a^2$, $R_a^3$, $R_a^6$, $R_a^7$, $R_a^9$, $R_a^{11}$, $R_a^{12}$ bzw. $R_a^{13}$ gegebenen Bedeutungen haben, und von pharmazeutisch verwendbaren Salzen davon.

Die Erfindung betrifft hauptsächlich h) die Verwendung von Verbindungen der Formel I, worin $X^1$ eine Gruppe der Formel —N($R^{14}$)—, wobei $R^{14}$ für Wasserstoff oder $C_{1-4}$-Alkyl steht, $X_2$ Sauerstoff, $R^1$ Wasserstoff, Niederalkanoyl oder die Gruppe der Formel Ia, worin n für 0 oder 1, $Z^1$ für Carbonyl, $Y^1$ für Niederalkylen, das durch Iminocarbonyl unterbrochen sein kann, $X^3$ für Sauerstoff und $A^1$ für einen Rest der Formel Ib oder Ic stehen, worin $R^{15}$ für einen 7-30 Kohlenstoffatome aufweisenden unsubstituierten oder durch Hydroxy, Amino und/oder Alkanoylamino mit bis zu 22 Kohlenstoffatomen substituierten Alkyl- oder Alkenylrest, $R^{16}$ für Wasserstoff und $R^{17}$ für 2-Hydroxy- oder 1,2-Dihydroxyäthyl, wobei in einem Rest $R^{17}$ mindestens eine Hydroxygruppe mit einem 7-30 Kohlenstoffatome aufweisenden Alkyl- oder Alkenylrest veräthert oder mit einem 7-30 Kohlenstoffatome aufweisenden Alkanoyl- oder Alkenoylrest verestert ist, oder $R^{16}$ und $R^{17}$ unabhängig voneinander für Hydroxymethyl, wobei die Hydroxygruppe mit einem 7-30 Kohlenstoffatome aufweisenden Alkyl- oder Alkenylrest veräthert oder mit einem 7-30 Kohlenstoffatome aufweisenden Alkanoyl- oder Alkenoylrest verestert ist, stehen, $R^2$ Niederalkanoyl, Hydroxyniederalkanoyl, Benzoyl oder unabhängig von $R^1$, $R^{12}$ und $R^{13}$ eine wie vorstehend definierte Gruppe der Formel Ia, $R^3$, $R^4$, $R^5$, $R^7$ und $R^8$ unabhängig voneinander Wasserstoff, Methyl oder Aethyl, $R^6$ Wasserstoff oder unsubstituiertes oder durch freies Hydroxy, freies Mercapto, Niederalkoxy, Niederalkylthio, Alkanoyloxy mit 2-30 Kohlenstoffatomen, Alkenoyloxy mit 6-30 Kohlenstoffatomen, Phenyl, 4-Hydroxy-phenyl oder durch eine Gruppe der Formel Id substituiertes Niederalkyl, worin m für 0 oder 1, E für Sauerstoff oder Schwefel, $Z^2$ für Carbonyl, $Y^2$ für unsubstituiertes oder durch Phenyl substituiertes Niederalkylen, das durch Iminocarbonyl unterbrochen sein kann, $X^4$ für Sauerstoff und $A^2$ unabhängig von $A^1$ und $A^3$ für einen Rest der wie vorstehend definierten Formeln Ib und Ic stehen, $R^9$ und $R^{11}$ unabhängig voneinander Hydroxy, Niederalkoxy, Amino, Niederalkylamino, Carboxyniederalkylamino, Niederalkoxycarbonylniederalkylamino, Carbamoylniederakylamino oder einen Rest der Formel Ie, worin $X^5$ für Sauerstoff oder NH, $Y^3$ für Niederalkylen, das durch Iminocarbonyl unterbrochen sein kann, $X^6$ für Sauerstoff und $A^3$ unabhängig von $A^1$ und $A^2$ für einen Rest der wie vorstehend definierten Formeln Ib und Ic stehen, $R^{10}$ Wasserstoff, $R^{12}$ Wasserstoff, Niederalkanoyl oder den gleichen Rest wie $R^{13}$, und $R^{13}$ Wasserstoff, Alkanoyl oder Alkenoyl mit jeweils bis zu 30 Kohlenstoffatomen oder unabhängig von $R^1$ und $R^2$ einen wie vorstehend definierten Rest der Formel Ia bedeuten, mit der Massgabe, dass die Verbindungen mindestens einen und höchstens zwei Reste aus der Gruppe $A^1$, $A^2$ und $A^3$ aufweisen und von pharmazeutisch verwendbaren Salzen dieser Verbindungen.

Die Erfindung betrifft besonders i) die Verwendung von Verbindungen der Formel I, worin $X^1$ die Gruppe NH, $X^2$ Sauerstoff, $R^1$ Wasserstoff oder Niederalkanoyl, $R^2$ Niederalkanoyl, Benzoyl oder eine Gruppe der Formel Ia, worin n für 0 oder 1, $Z^1$ für Carbonyl, $Y^1$ für Niederalkylen, das durch ein oder zwei Iminocarbonylgruppen unterbrochen sein kann, $X^3$ für Sauerstoff und $A^1$ für einen Rest der Formel Ib oder Ic stehen, worin $R^{15}$ für einen Alkylrest mit 7 bis 22 C-Atomen, $R^{16}$ für Wasserstoff und $R^{17}$ für 1,2-Dihydroxyäthyl stehen, wobei beide Hydroxygruppen unabhängig voneinander jeweils mit einem 10-22 Kohlenstoffatome aufweisenden Alkanoyl- oder Alkenoylrest verestert sind, $R^3$ Wasserstoff oder Methyl, $R^4$, $R^5$, $R^7$ und $R^8$ Wasserstoff, $R^6$ Wasserstoff oder unsubstituiertes oder durch freies Hydroxy, Alkanoyloxy mit 2 bis 22 Kohlenstoffatomen, Alkenoyloxy mit 6-22 Kohlenstoffatomen, Phenyl oder durch eine Gruppe der Formel Id substituiertes Niederalkyl, worin m für 0 oder 1, E für Sauerstoff, $Z^2$ für Carbonyl, $Y^2$ für unsubstituiertes oder durch Phenyl substituiertes Niederalkylen, das durch Iminocarbonyl unterbrochen sein kann, $X^4$ für Sauerstoff und $A^2$ unabhängig von $A^1$ und $A^3$ für einen Rest der vorstehend definierten Formeln Ib und Ic stehen, $R^9$ und $R^{11}$ unabhängig voneinander Hydroxy, Niederalkoxy, Amino, Niederalkylamino, α-Carboxy-niederalkylamino, α-Niederalkoxycarbonyl-niederalkylamino, α-Carbamoyl-niederalkylamino oder einen Rest der Formel Ie, worin $X^5$ für die Gruppe NH, $Y^3$ für Niederalkylen, das durch ein oder zwei Iminocarbonylgruppen unterbrochen sein kann, $X^6$ für Sauerstoff und $A^3$ unabhängig von $A^1$ und $A^2$ für einen Rest der vorstehend definierten Formeln Ib oder Ic stehen, $R^{10}$ Wasserstoff, $R^{12}$ Wasserstoff, Niederalkanoyl oder den gleichen Rest wie $R^{13}$ und $R^{13}$ Wasserstoff, Alkanoyl mit 2 bis 22 Kohlenstoffatomen, Alkenoyl mit 6 bis 22 Kohlenstoffatomen oder unabhängig von $R^2$ einen wie vorstehend definierten Rest der Formel Ia bedeuten, und von pharmazeutisch verwendbaren Salzen dieser Verbindungen.

Die Erfindung betrifft ganz besonders j) die Verwendung von Verbindungen der Formel I, worin $X^1$ die Gruppe NH, $X^2$ Sauerstoff, $R^1$ Wasserstoff oder $C_{2-4}$-Alkanoyl, $R^2$ $C_{2-4}$-Alkanoyl oder eine Gruppe der Formel Ia, worin n für 0 oder 1, $Z^1$ für Carbonyl, $Y^1$ für Niederalkylen, das durch ein oder zwei Iminocarbonylgruppen unterbrochen sein kann, $X^3$ für Sauerstoff und $A^1$ für einen Rest der Formel Ib oder Ic stehen, worin $R^{15}$ für einen unverzweigten Alkylrest mit 12 bis 18 C-Atomen, $R^{16}$ für Wasserstoff und $R^{17}$ für 1,2-Dipalmitoyloxy-äthyl oder 2-Oleoyloxy-1-palmitoyl-oxyäthyl stehen, $R^3$ Wasserstoff oder

Methyl, $R^4$, $R^5$, $R^7$, $R^8$ und $R^{10}$ Wasserstoff, $R^6$ unsubstituiertes oder durch einen Rest der Formel Id substituiertes Methyl, Aethyl oder Isopropyl, worin m für 0 oder 1, E für Sauerstoff, $Z^2$ für Carbonyl, $Y^2$ für unsubstituiertes oder durch Phenyl substituiertes Niederalkylen, das durch ein oder zwei Iminocarbonylgruppen unterbrochen sein kann, $X^4$ für Sauerstoff und $A^2$ unabhängig von $A^1$ und $A^3$ für einen Rest der vorstehend definierten Formeln Ib oder Ic stehen, $R^9$ Amino, $R^{11}$ Hydroxy oder einen Rest der Formel Ie, worin $X^5$ für die Gruppe NH, $Y^3$ für Niederalkylen, das durch ein oder zwei Iminocarbonylgruppen unterbrochen sein kann, $X^6$ für Sauerstoff und $A^3$ unabhängig von $A^1$ und $A^2$ für einen Rest der vorstehend definierten Formeln Ib oder Ic stehen, $R^{12}$ Wasserstoff, Acetyl oder den gleichen Rest wie $R^{13}$ und $R^{13}$ Wasserstoff, Acetyl oder, unabhängig von $R^2$, einen wie vorstehend definierten Rest der Formel Ia bedeuten, und von pharmazeutisch verwendbaren Salzen dieser Verbindungen.

Die Erfindung betrifft hauptsächlich k) die Verwendung der obengenannten Verbindungen der Formel I, worin die Reste $A^1$, $A^2$ und $A^3$, falls vorhanden, für einen Rest der Formel Ic stehen, und ihrer pharmazeutisch verwendbaren Salze.

In erster Linie betrifft die Erfindung 1) die Verwendung der obengenannten Verbindungen der Formel I, die nur einen Phosphorylsubstituenten tragen, und zwar im Rest $R^{11}$ oder $R^9$, und ihrer pharmazeutisch verwendbaren Salze.

Ganz besonders betrifft die Erfindung m) die Verwendung der vorstehend unter i) oder k) genannten Verbindungen der Formel I, die als Zukkerteil ein Derivat der D-Glucose enthalten, die im Falle asymmetrischer Substitution am C—$R^3$ die (D)-, am C—$R^6$ die (L)- und am C—N—$R^8$ die (D)-Konfiguration aufweisen und die (nur) einen Phosphorylsubstituenten tragen, und zwar im Rest $R^9$ oder $R^{11}$, wobei in diesem Phosphorylsubstituenten der Formel Ie der Rest $A^3$ für einen Rest der Formel Ic steht, der gemäss h) oder j) definiert ist, und ihrer pharmazeutisch verwendbaren Salze.

Bevorzugterweise betrifft die Erfindung die Verwendung von Verbindungen der Formel I, die an einem asymmetrisch substituierten C(—$R^3$)-Atom die (D)-Konfiguration, an einem asymmetrisch substituierten C(—$R^6$)-Atom die (L)-Konfiguration und am C(—N—$R^8$)-Atom die (D)-Konfiguration aufweisen, und worin sich der Zuckerteil von (D)-Glucose ableitet, $X^1$ die Gruppe NH, $X^2$ Sauerstoff, $R^1$, $R^4$, $R^5$, $R^7$, $R^8$, $R^{10}$, $R^{12}$ und $R^{13}$ Wasserstoff, $R^2$ $C_{1-3}$-Alkyl oder Phenyl, $R^3$ Wasserstoff oder $C_{1-3}$-Alkyl, $R^6$ Wasserstoff, gegebenenfalls durch Hydroxy, Methoxy, Mercapto, Methylmercapto oder Halogen substituiertes $C_{1-3}$-Alkyl, gegebenenfalls durch Hydroxy, Methoxy oder Halogen substituiertes Phenyl oder Phenylmethyl, ein oder zwei Azaatome enthaltendes Heteroaryl oder Heteroarylmethyl mit 5 Ringgliedern, oder $R^5$ und $R^6$ zusammen Trimethylen, einer der Reste $R^9$ und $R^{11}$ einen Rest der Formel Ie, worin $X^5$ für die Gruppe NH oder Sauerstoff, $Y^3$ für $C_{2-3}$ Alkylen oder für einen Rest der Formeln

$$-U^2-COO-U^3- \quad \text{oder} \quad -U^2-\overset{O}{\overset{\|}{C}}-NH-U^3- ,$$

worin $U^2$ und $U^3$ unabhängig voneinander je $C_{1-3}$-Alkylen, das unsubstituiert oder durch gegebenenfalls durch Hydroxy, Niederalkoxy, Mercapto oder Niederalkylmercapto substituiertes $C_{1-3}$-Alkyl oder durch gegebenenfalls durch Hydroxy, Methoxy oder Halogen substituiertes Phenyl oder Phenylniederalkyl oder durch ein oder zwei Azaatome enthaltendes Heterocyclyl oder Heterocyclyl-($C_{1-3}$-alkyl) mit jeweils 5-6 Ringgliedern substituiert ist, bedeuten, $X^6$ für Sauerstoff und $A^3$ für einen Rest der Formel Ic stehen, worin $R^{16}$ Wasserstoff und $R^{17}$ eine 1,2-Dihydroxyäthyl- oder 2-Hydroxyäthylgruppe bedeuten, in der mindestens eine der Hydroxygruppen mit einer gegebenenfalls ein oder zweifach ungesättigten aliphatischen $C_{16-20}$-Carbonsäure verestert oder mit einem gegebenenfalls ein- oder zweifach ungesättigten aliphatischen $C_{12-18}$-Alkohol veräthert ist, und der andere der Reste $R^9$ und $R^{11}$ Hydroxy, Niederalkoxy, Amino, Niederalkylamino oder Aminocarbonylniederalkylamino bedeuten, und von pharmazeutisch verwendbaren Salzen von solchen Verbindungen.

Ganz bevorzugterweise betrifft die Erfindung die Verwendung von Verbindungen der Formel I, die an einem asymmetrisch substituierten C(—$R^3$)-Atom die (D)-Konfiguration, an einem asymmetrisch substituierten C(—$R^6$)-Atom die (L)-Konfiguration und am C(—N—$R^8$)-Atom die (D)-Konfiguration aufweisen, und worin sich der Zuckerteil von (D)-Glucose ableitet, $X^1$ die Gruppe NH, $X^2$ Sauerstoff, $R^1$, $R^4$, $R^7$, $R^8$, $R^{10}$, $R^{12}$ und $R^{13}$ Wasserstoff, $R^2$ Niederalkyl oder Phenyl, $R^3$ und $R^5$ unabhängig voneinander Wasserstoff oder Methyl, $R^6$ $C_{1-4}$-Alkyl, $R^9$ Amino und $R^{11}$ einen Rest der Formel

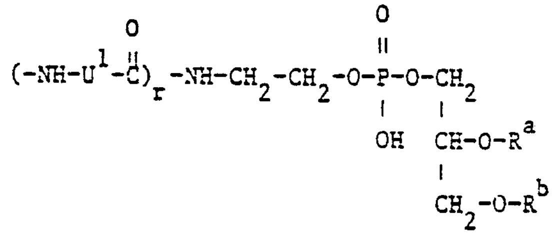

worin r für 0 oder 1, $U^1$ für einen Rest einer der Formeln

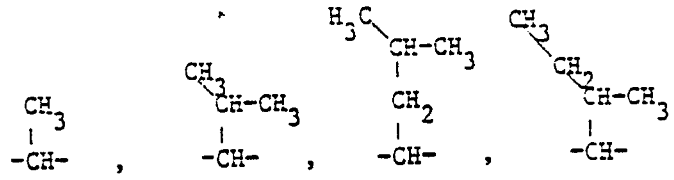

, , ,

(L) (L) (L) (L)

und $R^a$ und $R^b$ unabhängig voneinander für das Acylradikal einer gesättigten oder ein oder zwei Doppelbindungen aufweisenden unsubstituierten aliphatischen Carbonsäure mit 12 bis 22 C-Atomen stehen, bedeuten, und von pharmazeutisch verwendbaren Salzen von solchen Verbindungen.

Vor allem betrifft die Erfindung die erfindungsgemässe Verwendung der folgenden Verbindungen : N-Acetyl-muramyl-L-alanyl-D-isoglutamin-2-(1,2-dipalmitoyl-sn-glycero-3-hydroxyphosphoryloxy)-äthylamid-natriumsalz, N-Acetyl-desmethylmuramyl-L-alanyl-D-isoglutamin-2-(1,2-dipalmitoyl-sn-glycero-3-hydroxyphosphoryloxy)-äthylamid-natriumsalz, N-Acetyl-desmethylmuramyl-L-alanyl-D-isoglutamin-2-(1-palmitoyl-2-oleoyl-sn-glycero-3-hydroxyphosphoryloxy)-äthylamid-natriumsalz, N-Acetyl-desmethylmuramyl-L-alanyl-D-isoglutaminyl-L-alanin-2-(1,2-dipalmitoyl-sn-glycero-3-hydroxyphosphoryloxy)-äthylamid-natriumsalz, N-Acetyl-muramyl-L-alanyl-D-isoglutaminyl-L-alanin-2-(3-palmitoyl-rac-glycero-1-hydroxyphosphoryloxy)-äthylamid-natriumsalz, N-Acetyl-muramyl-L-α-aminobutyryl-D-isoglutaminyl-L-alanin-2-(1,2-dipalmitoyl-sn-glycero-3-hydroxyphosphoryloxy)-äthylamid-natriumsalz, N-Benzoyl-muramyl-L-alanyl-D-isoglutaminyl-L-alanin-2-(1,2-dipalmitoyl-sn-glycero-3-hydroxyphosphoryloxy)-äthylamid-natriumsalz, N-Acetyl-muramyl-L-alanyl-D-isoglutaminyl-L-alanin-2-(tetradecyloxy-hydroxyphosphoryloxy)-äthylamid-natriumsalz, N-Acetyl-muramyl-L-alanyl-D-isoglutaminyl-L-alanin-2-(hexadecyloxy-hydroxyphosphoryloxy)-äthylamid-natriumsalz, N-Acetyl-muramyl-L-α-aminobutyryl-D-isoglutaminyl-L-alanin-2-[(3R)-hydroxy-(2S)-palmitoylamino-4t-octadecenyloxy-hydroxyphosphoryloxy]-äthylamid-natriumsalz, N-Acetyl-muramyl-L-α-aminobutyryl-D-isoglutaminyl-L-alanin-2-[(3R)-hydroxy-(2S)-palmitoylamino-octadecyloxy-hydroxyphosphoryloxy]-äthylamid-natriumsalz, N-Acetyl-muramyl-L-alanyl-D-isoglutaminyl-L-alanin-2-(cholest-5-en-3β-oxy-hydroxyphosphoryloxy]-äthylamid-natriumsalz, N-Acetyl-muramyl-L-O- {(N-[2-(1-palmitoyl-2-oleoyl-sn-glycero-3-hydroxyphosphoryloxy)-äthyl]-succinamoyl)-glycyl}-seryl-D-isoglutamin-natriumsalz, N-Acetyl-normuramyl-L-O-(1,2-dipalmitoyl-rac-glycero-3-hydroxyphosphoryl)-seryl-D-isoglutamin-natriumsalz, N-Acetyl-6-0-([1,2-dipalmitoyl-rac-glycero-3-hydroxyphosphoryloxy]-acetyl)-normuramyl-L-alanyl-D-isoglutamin-natriumsalz, N-Acetyl-6-0-(1,2-dipalmitoyl-rac-glycero-3-hydroxyphosphoryloxy)-normuramyl-L-alanyl-D-isoglutamin-natriumsalz, N-{N-[2-(1,2-dipalmitoyl-sn-glycero)-3-hydroxyphosphoryloxy]-äthyl}-succinamoyl-muramyl-L-alanyl-D-isoglutamin-natriumsalz, N-Acetyl-6-0-{N-[2-(1,2-dipalmitoyl-sn-glycero-3-hydroxyphosphoryloxy)-äthyl]-succinamoyl}-muramyl-L-α-aminobutyryl-D-isoglutamin-natriumsalz, N-Acetyl-normuramyl-L-O-{N-[2-tetradecyloxy-hydroxyphosphoryloxy)-äthyl]-succinamoyl}-seryl-D-isoglutamin-natriumsalz, N-Acetyl-muramyl-L-O-{[N-1-palmitoyl-2-oleoyl-sn-glycero-3-hydroxyphosphoryloxy)-äthyl]-succinamoyl-L-phenylalanyl}-seryl-D-isoglutamin-natriumsalz, N-Acetyl-muramyl-L-O-(hexadecyloxy-hydroxyphosphoryl)-seryl-D-isoglutamin-natriumsalz und N-Acetyl-muramyl-L-O-(1,2-dipalmitoyl-3-sn-glycero-hydroxyphosphoryl)-seryl-D-isoglutamin-natriumsalz.

Insbesondere betrifft die Erfindung die Verwendung der in den Beispielen beschriebenen Verbindungen der Formel I und ihrer pharmazeutisch verwendbaren Salze, in allererster Linie die Verwendung von N-Acetyl-muramyl-L-alanyl-D-isoglutaminyl-L-alanin-2-(1,2-dipalmitoyl-sn-glycero-3-hydroxyphosphoryloxy)-äthylamid, insbesondere von dem Natriumsalz dieser Verbindung, nachstehend als Verbindung I bezeichnet.

Die nachstehenden Beispiele illustrieren die Erfindung, ohne sie in irgendeiner Form einzuschränken.

## Beispiel 1

Gruppen von 30, 40 oder 50 weiblichen MF-2f SPF-Mäusen mit einem Körpergewicht von 14-16 g bzw. 12-14 g bei den Versuchen mit Herpes simplex 1 Viren werden unter leichter Narkose mit einem Gemisch aus gleichen Teilen Aether, Äthanol und Chloroform mit letalen Dosen (ungefähr eine $LD_{80-90}$ ; Anzahl der plaque forming units [PFU] siehe Tabelle 1) der untengenannten Viren-Stämme in Form von je 0,05 ml einer Suspension der Viren intranasal infiziert.

10 oder 20 von diesen Mäusen werden zum unten angegebenen Zeitpunkt [Tage] bezogen auf den Tag der Infektion einmal (Einzeldosis) die in Tabelle 1 genannten Mengen der Wirksubstanz N-Acetyl-muramyl-L-alanyl-D-isoglutaminyl-L-alanin-2-(1,2-dipalmitoyl-sn-glycero-3-hydroxyphosphoryloxy)-äthylamid-natriumsalz (Verbindung I) in 0,05 bzw. 0,1 ml Phosphat-gepufferter-Salzlösung (PBS) bzw. in 0,2 ml einer 0,05 Gew.-%igen Lösung von Natriumcarboxymethylcellulose in doppelt destilliertem Wasser im Falle von intranasaler, intravenöser bzw. oraler Applikation auf die in Tabelle 1 genannte Art appliziert.

Die restlichen der obengenannten Mäuse, d. h. 20 oder 30, dienen zur Kontrolle, d. h. sie bleiben entweder unbehandelt oder erhalten ein Placebo.

Die intranasale Applikation von Verbindung I wird unter leichter Narkose mit einem Gemisch aus gleichen Teilen Diäthyläther, Aethanol und Chloroform durchgeführt.

Tabelle 1

| Virus [PFU] | Applikationsart | Applikationszeit [Tage] - : vor der Infektion + : nach der Infektion | Prozentsatz der 20 Tage nach der Infektion noch lebenden Mäuse in Abhängigkeit von der Wirksubstanzmenge [mg/kg] statistische Signifikanz $^{x}P \leq 0{,}05$, $^{xx}P \leq 0{,}01$ (Vierfelder Test) | | | | | | |
|---|---|---|---|---|---|---|---|---|---|
| | | | 500 | 100 | 20 | 10 | 1 | 0,1 | 0 ≙ Kontrolle |
| Influenza A/ Victoria/3/75 (H3N2) (Maus-adaptiert) [$2 \times 10^3$-$1 \times 10^4$ PFU] | intravenös | -7 | | $80^{xx}$ | | $90^{xx}$ | 30 | | 20 |
| | | -5 | | $80^{xx}$ | | $80^{xx}$ | 30 | | |
| | | -1 | | $80^{xx}$ | | $80^{xx}$ | $70^{x}$ | | |
| | | 0 | | $90^{xx}$ | | $100^{xx}$ | $70^{x}$ | | |
| | | +1 | | $90^{xx}$ | | $70^{x}$ | 60 | | |
| | intranasal | -14 | | | | 60 | $70^{(x)}$ | $80^{x}$ | 40 |
| | | -7 | | | | $100^{xx}$ | $100^{xx}$ | $80^{x}$ | |
| | oral | +3 | $70^{xx}$ | $90^{xx}$ | $80^{xx}$ | | | | 15 |
| | | +7 | $100^{xx}$ | $100^{xx}$ | $70^{xx}$ | | | | |

Tabelle 1 (Fortsetzung)

| Virus [PFU] | Applikationsart | Applikationszeit [Tage] - : vor der Infektion + : nach der Infektion | Prozentsatz der 20 Tage nach der Infektion noch lebenden Mäuse in Abhängigkeit von der Wirksubstanzmenge [mg/kg] statistische Signifikanz $^{x}P \leq 0{,}05$, $^{xx}P \leq 0{,}01$ (Vierfelder-Test) | | | | | | |
|---|---|---|---|---|---|---|---|---|---|
| | | | 500 | 100 | 20 | 10 | 1 | 0,1 | 0 ≙ Kontrolle |
| Influenza A/ USSR/92/77 (H1N1) (Maus-adaptiert) [$3x10^2$-$1x10^3$ PFU] | intravenös | -7 | | $66^{x}$ | | 40 | 40 | | 10 |
| | | -2 | | $60^{x}$ | | $70^{xx}$ | $50^{x}$ | | |
| | | -1 | | $70^{x}$ | | $70^{xx}$ | $50^{x}$ | | |
| | | 0 | | $100^{xx}$ | | $80^{xx}$ | $60^{x}$ | | |
| | | +1 | | $90^{xx}$ | | $90^{xx}$ | $60^{x}$ | | |
| | | +2 | | $90^{xx}$ | | $50^{xx}$ | 40 | | |
| | | +6 | | $60^{x}$ | | $60^{x}$ | 40 | | |
| Influenza A/ Texas/1/77 ($H_3N_2$) (Maus-adaptiert) [$2x10^0$-$1x10^1$ PFU] | intranasal | -7 | | | | $50^{xx}$ | $40^{x}$ | $30^{(x)}$ | 0 |
| | | -7 | | | | $100^{xx}$ | $90^{x}$ | $70^{(x)}$ | 35 |
| | oral | +7 | | | | $65^{xx}$ | | | 0 |

Tabelle 1' (Fortsetzung)

| Virus [PFU] | Applikations-art | Applikations-zeit | Prozentsatz Ueberlebende 500 | 100 | 20 | 10 | 1 | 0,1 | 0 |
|---|---|---|---|---|---|---|---|---|---|
| Influenza B / Hong Kong/5/72 [$3x10^5$-$1x10^6$ PFU] | intravenös | -7 | | $90^x$ | | 70 | 70 | | 42 |
| | | -5 | | $90^x$ | | $100^{xx}$ | $80^{(x)}$ | | |
| | | -2 | | $100^{xx}$ | | $90^x$ | $100^{xx}$ | | |
| | | -1 | | $100^{xx}$ | | $90^x$ | $80^{(x)}$ | | |
| | | 0 | | $100^{xx}$ | | $100^{xx}$ | $90^x$ | | |
| | | +1 | | $80^{(x)}$ | | $80^{(x)}$ | 60 | | |
| | | +2 | | $100^{xx}$ | | 70 | $90^x$ | | |
| | | +6 | | $100^{xx}$ | | $80^{(x)}$ | 70 | | |
| | intranasal | -7 | | | | $100^{xx}$ | $80^x$ | $80^x$ | 30 |
| | oral | +3 | | $50^x$ | | | $50^x$ | | 15 |
| | | +7 | | $80^{xx}$ | | $90^{xx}$ | $50^x$ | | |
| Influenza B / Ann Arbor (Ms.Lu.25.Russ) (Maus-adaptiert) [$1x10^1$ PFU] | intranasal | -14 | | | | $50^x$ | 20 | 20 | 0 |
| | | -7 | | | | $80^{xx}$ | $50^x$ | 20 | |
| | oral | +3 | | $100^{xx}$ | | $90^{xx}$ | $80^{xx}$ | | 15 |
| | | +7 | | $100^{xx}$ | | $80^{xx}$ | $80^{xx}$ | | |

Tabelle 1 (Fortsetzung)

| Virus [PFU] | Applikations-ort | Applikations-zeit | Prozentsatz Ueberlebende 500 | 100 | 20 | 10 | 1 | 0,1 | 0 |
|---|---|---|---|---|---|---|---|---|---|
| Encephalomyo-carditis [$1x10^1$-$1x10^2$ PFU] | oral | 0 | | $80^x$ | | $100^{xx}$ | 60 | | 30 |
| | | +2 | | 60 | | $80^x$ | $90^{xx}$ | | |
| | | +4 | | $100^{xx}$ | | $70^{(x)}$ | $90^{xx}$ | | |
| Herpes Simplex 1/TUP [$2x10^4$ PFU] | intranasal | -7 | | | | $70^x$ | $60^x$ | $60^x$ | 20 |
| Herpes Simplex 1/Virtue [$2x10^3$ PFU] | intranasal | -7 | | | | $90^x$ | $100^x$ | 70 | 40 |

Der zeitliche Verlauf einiger der obenbeschriebenen Infektionen wird durch die Figuren 1 und 2 anschaulich wiedergegeben.

Die grosse Ueberlegenheit der Phosphorylverbindungen der Formel i gegenüber Muramylpeptiden, wie N-Acetyl-muramyl-L-alanyl-D-glutamin-n-butylester (Verbindung II) zeigt sich z. B. darin,dass in den obengenannten vergleichend durchgeführten Versuchen bei oraler Applikation von 10 mg dieser Verbindung am Tage + 7 sämtliche Mäuse an der Infektion mit Influenza A/Texas/1/77 sterben, während bei Verabreichung der gleichen Menge des Phosphorylmuramylpeptids Verbindung I zum gleichen Zeitpunkt, wie aus der Tabelle ersichtlich ist, 65 % der Mäuse die Infektion überleben.

Auch bei intranasaler Applikation von 1 mg/kg Verbindung II am Tag — 7 wird im Falle der Infektion mit Influenza A/Texas/1/77 keinerlei Schutzwirkung festgestellt, während bei Verabreichung der gleichen Menge von Verbindung I zur gleichen Zeit 90 % der Mäuse die Infektion überleben.

Keinerlei Schutzwirkung findet man auch bei intranasaler Applikation von 10 mg/kg Verbindung II am Tag — 7 im Falle der intranasalen Infektion mit Herpesvirus hominis 1/TUP, wie aus Figur 3 hervorgeht.

## Beispiel 2

Die in der nachfolgenden Tabelle angegebene Anzahl weiblicher Albino Meerschweinchen des Pirbright Stammes (150-180 g Körpergewicht) werden intravaginal mit ~ $10^4$ PFU (plaque forming units) Herpesvirus hominis 2/Angelotti, kultiviert in HEL (human embryonal lungs)-Zellen, infiziert, wie es in B. Lukáš et al., Arch. ges. Virusforsch. 44, 153-155 (1974) beschrieben ist.

Zu dem in der nachfolgenden Tabelle angegebenen Zeitpunkt [Zeitdifferenz in Tagen bezogen auf den Tag der Infektion, wobei — (minus) einen Zeitpunkt vor der Infektion angibt] wird die in der Tabelle 2 genannte Anzahl N der Meerschweinchen mit einer Einzeldosis von je 0,1 ml eines Gels intravaginal behandelt, das die in Tabelle 2 angegebene Konzentration von Verbindung I enthält.

Das Gel ohne Wirkstoff hat die folgende Zusammensetzung :

2,25 % Natrium-Carboxymethylcellulose (Hercules, USA) 10 % Glycerin ad 100 % bidestilliertes Wasser.

Die Wirkung der Behandlung geht aus Tabelle 2 hervor. Die bei unbehandelten Tieren auftretenden Symptome sind in B. Lukáš et al., Arch. Virol. 49, 1-11 (1975) beschrieben.

(Siehe Tabelle 2 Seite 19 f.)

## Beispiel 3

Augentropfen :

Zusammensetzung :

| | |
|---|---|
| Verbindung I | 0,10 mg |
| Borsäure | 30,00 mg |
| Natriumtetraborat · $10H_2O$ | 0,10 mg |
| Benzalkoniumchlorid | 0,20 mg |
| Wasser zur Injektion | ad 1,0 ml |

Herstellung :

In einem Teil der obengenannten Menge des Wassers für Injektionszwecke werden unter aseptischen Bedingungen und unter Rühren Borsäure, Natriumtetraborat und Benzalkoniumchlorid bei Raumtemperatur gelöst. Anschliessend löst man in der so erhaltenen Lösung CGP 19835 auf und ergänzt mit Wasser für Injektionszwecke auf das Endvolumen von 1,0 ml.

Die Lösung oder ein Teil oder Vielfaches davon wird durch einen Membranfilter filtriert und in gereinigte Behälter abgefüllt. Geeignete Behälter sind z. B.

Behälter aus flexiblem Kunststoff (à 5 ml oder 10 ml) mit Tropfeinsatz,

Behälter aus Glas (à 5 ml oder 10 ml) mit einer Tropfpipette aus Glas oder Kunststoff und einem elastomeren Pipettensauger oder

Plastikeinmaldosispipetten (Inhalt 1-2 Tropfen).

## Beispiel 4

Nicht-wässerige Einzeldosis zur Nasenapplikation

Zusammensetzung :

| | |
|---|---|
| Verbindung I | 0,03 mg |
| Miglyol 812 | ad 30,00 mg |

Tabelle 2

Effekt auf lokale Symptome bei der topischen Chemotherapie von Herpes genitalis am Meerschweinchen (statistische Signifikanz $^{x}P < 0{,}05$, $^{xx}P < 0{,}01$ im Fisher Test)

| Wirkstoff konzentration [%] | Behandlungszeitpunkt [Tage] | N | Tiere [%] mit einer Regression der Symptome von ≥ 66% am Tag | | | | symptomfreie Tiere [%] am Tag | | | | |
|---|---|---|---|---|---|---|---|---|---|---|---|
| | | | 7 | 10 | 12 | 14 | 10 | 12 | 14 | 21 | 34 |
| 1,0 | -7 | 14 | 29 | $79^{xx}$ | $71^{xx}$ | $93^{xx}$ | 21 | $57^{x}$ | $86^{xx}$ | $86^{x}$ | $100^{x}$ |
| 0,1 | -7 | 14 | 21 | $57^{x}$ | $86^{xx}$ | $100^{xx}$ | $43^{x}$ | $64^{xx}$ | $86^{xx}$ | $86^{xx}$ | $100^{xx}$ |
| 1,0 | +4 | 14 | $79^{xx}$ | $79^{xx}$ | $86^{xx}$ | $93^{xx}$ | 36 | $64^{xx}$ | $79^{xx}$ | $86^{xx}$ | $100^{xx}$ |
| 0,1 | +4 | 13 | $62^{xx}$ | $85^{xx}$ | $100^{xx}$ | $93^{xx}$ | $70^{xx}$ | $77^{xx}$ | $85^{xx}$ | $100^{xx}$ | $100^{xx}$ |
| 0 (Placebo) | +4 | 14 | 0 | 0 | 0 | 0 | 0 | 0 | 0 | 0 | 0 |
| Kontrolle (unbehandelt) | | 20 | 0 | 0 | 0 | 0 | 0 | 0 | 0 | 0 | 20 |

Herstellung :

0,03 mg Verbindung I werden unter aseptischen Bedingungen in 29,97 mg Miglyol 812 gelöst.

Diese Lösung wird in einen handelsüblichen Einmalnasenapplikator, z. B. einen gemäss US-Patent Nr. 3 739 951, abgefüllt, der vor den Anwendung auf eine Treibstoffdose aufgesetzt wird.

Beispiel 5

Nasentropfen

Zusammensetzung :

| | I | II |
|---|---|---|
| Verbindung I | 0,15 mg | 0,10 mg |
| Thiomersal | 0,02 mg | -- |
| Natriummonohydrogenphosphat•$2H_2O$ | 0,30 mg | 0,30 mg |
| Natriumdihydrogenphosphat•$12H_2O$ | 10,10 mg | 10,10 mg |
| Benzalkoniumchlorid | -- | 0,10 mg |
| Aethylendiamintetraessigsäure-dinatriumsalz (EDTA) | 0,50 mg | 0,50 mg |
| Natriumchlorid | 3,70 mg | 4,50 mg |
| Entmineralisiertes Wasser | 988,30 mg | 987,60 mg |
| pH-Wert: | 5,0 $\pm$ 0,3 | 5,0 $\pm$ 0,3 |
| Gefrierpunktserniedrigung Δt | - 0,51 °C | - 0,56 °C |

Herstellung :

In einem Teil der obengenannten Menge von entmineralisiertem Wasser werden unter Rühren Natriumdihydrogenphosphat, Dinatriumhydrogenphosphat, Natriumchlorid, Thiomersal und EDTA-Dinatriumsalz bei Raumtemperatur gelöst.

In dieser Lösung löst man anschliessend Verbindung I auf und ergänzt mit dem restlichen entmineralisierten Wasser.

Die Lösung oder ein Teil oder Vielfaches davon wird durch einen Membranfilter filtriert und in gereinigte Behälter abgefüllt. Geeignete Behälter sind z. B.

a) Glas oder Kunststoffbehälter (à 5 ml oder 10 ml) welche eine Pipette aus Glas oder Kunststoff mit einem elastomeren Pipettensauger besitzen,

b) Knautschflaschen aus Kunststoff mit einem Steigrohr und einem Sprühkopf aus Kunststoff,

c) Einzeldosisbehälter aus Kunststoff (Inhalt 2-3 Tropfen) oder

d) Glas oder Kunststoffflaschen, die mit einem normierten Pumpdosierspray aus Kunststoff versehen sind (ohne Treibgas).

Beispiel 6

Gel

Zusammensetzung :

| | |
|---|---|
| Verbindung I | 0,01 g |
| Glycerin 85 % | 10,00 g |
| Methylparaben | 0,12 g |
| Propylparaben | 0,03 g |
| Natriumcarboxymethylcellulose (hohe Viskosität) | 2,50 g |
| entmineralisiertes Wasser | 87,34 g |

Herstellung :

Methyl- und Propylparaben werden in einem Teil des heissen entmineralisierten Wassers gelöst. Hierauf wird die Natriumcarboxymethylcellulose unter kräftigem Rühren in die erhaltene Lösung eingearbeitet. Man lässt den Schleim unter Rühren ausquellen. Nach dem Erkalten werden das Glycerin und eine Lösung des Wirkstoffs Verbindung I in dem restlichen Wasser hinzugefügt.

Beispiel 7

Crème

Zusammensetzung :

| | |
|---|---|
| Verbindung I | 0,10 g |
| Sorbitanmonostearat | 0,60 g |
| Cetylalkohol | 3,00 g |
| Isopropylpalmitat | 2,00 g |
| Methylparaben | 0,12 g |
| Paraffinöl dickflüssig | 10,00 g |
| PEG (20)-Sorbitanmonostearat | 4,40 g |
| Propylparaben | 0,03 g |
| 70 %ige Lösung von kristallinem Sorbit in entmineralisiertem Wasser | 6,00 g |
| Stearinsäure | 9,00 g |
| entmineralisiertes Wasser | 64,67 g |

Herstellung :

Die Fettphase, bestehend aus Sorbitanmonostearat, Cetylalkohol, Stearinsäure, PEG (20)-Sorbitanmonostearat, Isopropylpalmitat und Paraffinöl, wird zusammengeschmolzen. Hierauf werden Methyl- und Propylparaben in einem Teil des heissen entmineralisierten Wassers gelöst. Der Wasserphase wird die Sorbitlösung hinzugefügt. Bei ca. 75 °C wird sodann unter Rühren die Wasserphase zur Fettphase gegeben. Die Crèmegrundlage wird hierauf unter Rühren abkühlen gelassen. Bei ca. 40 °C wird eine Lösung des Wirkstoffs Verbindung I in dem restlichen Wasser der Crèmegrundlage zugesetzt.

Beispiel 8

Nasensalbe

Zusammensetzung :

| | |
|---|---|
| Verbindung I | 0,03 g |
| Paraffinöl dickflüssig | 20,00 g |
| weisses Vaselin | 30,00 g |
| Wollfett, wasserfrei | 40,00 g |
| entmineralisiertes Wasser | 19,97 g |

Herstellung :

Die Fettphase, bestehend aus Paraffinöl, Vaselin und Wollfett, wird zusammengeschmolzen. Die wässrige Lösung des Wirkstoffs wird bei ca. 50 °C in die Fettphase eingearbeitet.

Beispiel 9

Hautsalbe

Zusammensetzung :

| | |
|---|---|
| Verbindung I | 0,25 g |
| Sorbitansesquioleat | 10,00 g |
| weisses Bienenwachs | 5,00 g |
| Cetylalkohol | 2,50 g |
| Methylparaben | 0,15 g |
| Paraffinöl dickflüssig | 20,00 g |
| Propylparaben | 0,02 g |
| Stearylalkohol | 2,50 g |
| weisses Vaselin | 40,00 g |
| entmineralisiertes Wasser | 19,58 g |

Herstellung :

Die Fettphase, bestehend aus Sorbitansesquioleat, weissem Bienenwachs, Cetylalkohol, Paraffinöl, Stearylalkohol und weissem Vaselin, wird zusammengeschmolzen. Hierauf werden Methyl- und Propylparaben in der Hauptmenge des Wassers heiss gelöst. Bei ca. 80 °C wird die Wasserphase in die Fettphase eingearbeitet. Bei ca. 40 °C wird eine Lösung des Wirkstoffs Verbindung I in dem restlichen Wasser der so erhaltenen Salbengrundlage zugefügt.

Beispiel 10

Lippenstift

Zusammensetzung :

| | |
|---|---|
| Verbindung I | 1,00 g |
| Polyethylenglykol mit dem mittleren Molekulargewicht 400 | 15,00 g |
| Polyethylenglykol mit dem mittleren Molekulargewicht 1000 | 83,00 g |
| Polyethylenglykol mit dem mittleren Molekulargewicht 4000 | 1,00 g |

Herstellung:

Der Wirkstoff wird in der Schmelze der Polyethylenglykole fein verteilt. Die zähflüssige Schmelze wird in geeignete Stifthülsen gegossen und erstarren lassen.

## Beispiel 11

Herstellung von Verbindung I

Zu einer Lösung von 1 mMol 2-(1,2-Dipalmitoyl-sn-glycero-3-hydroxy-phosphoryloxy)-äthylamin und 3,5 mMol N-Aethylmorpholin in 25 ml Chloroform : Methanol : Wasser = 65 : 25 : 4 wird eine Lösung von 1,5 mMol N-Acetylmuramyl-L-alanyl-D-isoglutaminyl-L-alanin-N-hydroxysuccinimidester in 5 ml Dimethylacetamid zugetropft. Nach 8 Stunden Rühren bei Raumtemperatur ist die Reaktion beendet. Das Reaktionsgemisch wird mit 130 ml Wasser versetzt. Chloroform und Teile des Methanols werden unter reduziertem Druck abdestilliert. Die wässrige Lösung wird durch einen Millipore-Filter aus Teflon (Porengrösse 5 µm) filtriert. Das Filtrat wird unter Rühren in einer Amicon Ultrafiltrationszelle durch eine Amicon YM 10 Membran (aus Polysaccharid, nominale Ausschlussgrenze 10 000 Dalton) im Diafiltrationsverfahren zunächst gegen Wasser (400 ml), danach gegen 0,1 M Natriumphosphatpuffer- 0,1 M NaCl, pH 7 (200 ml) und anschliessend gegen Wasser (850 ml) dialysiert. Das Innendialysat wird durch einen Millipore-Filter, Porengrösse 0,45 µm filtriert und gefriergetrocknet, worauf man N-Acetyl-muramyl-L-alanyl-D-isoglutaminyl-L-alanin-2-(1,2-dipalmitoyl-sn-glycero-3-hydroxyphosphoryloxy)-äthylamid-natriumsalz-hydrat erhält.

## Beispiel 12

Gruppen von 20 weiblichen Tif : MF-2f (SPF) Mäusen mit einem Körpergewicht von 14-16 g werden unter leichter Narkose mit einem Gemisch aus gleichen Teilen Diäthyläther, Aethanol und Chloroform mit $2 \times 10^3$ PFU Vaccinia Virus IHD in Form von je 0,1 ml einer Suspension des Virus in Rinderherz-Infusionsnährlösung (« beef heart infusion broth ») intravenös infiziert.

10 dieser Mäuse aus je einer der obengenannten Gruppen werden am vierten oder sechsten Tag nach der Infektion, d. h. am Tag + 4 bzw. + 6 (Applikationstag), mit einer einmaligen oralen Gabe von 1 mg/kg Verbindung I in 0,2 ml einer 0,05 Gew.-%igen wässrigen Lösung von Natriumcarboxymethylcellulose behandelt.

Die restlichen 10 Mäuse aus jeder einzelnen der obengenannten Gruppen dienen zur Kontrolle und erhalten ein Placebo.

Am siebten, neunten, zehnten und zwölften Tag nach der Infektion wird die Anzahl der Schwanzverletzungen bei den Mäusen ermittelt, und zwar im wesentlichen wie beschrieben von J.J. Boyle, R.F. Haff und R.C. Stewart in Antimicrobial Agents and Chemotherapy, 536-539 (1966), wobei zu bemerken ist, dass die ersten Schwanzverletzungen erst am sechsten bis siebten Tag erkennbar sind. Die Resultate sind aus Tabelle 3 ersichtlich :

Tabelle 3

| Applikationstag | Anzahl der Schwanzverletzungen (Mittelwert ± Standardabweichung) am Tag | | | |
|---|---|---|---|---|
| | +7 | +9 | +10 | +12 |
| +4 | 0,9±0,7* | 0,9±0,9* | 0,9±1,4* | 0,2±0,4* |
| +6 | 1,1±1,4* | 0,9±1,6* | 0,3±0,7* | 0,5±1,0* |
| Kontrolle | 4,3±1,9 | 4,3±1,9 | 5,9±1,9 | 4,6±1,8 |

* statistische Signifikanz P ≦ 0,01 (Student's T-Test)

Beispiel 13

Gruppen von 50-54 weiblicher Albino Meerschweinchen des Pirbright Stammes (150-180 g Körpergewicht) werden intravaginal mit $1 \times 10^3$ PFU des neurotropen Virusstammes Herpes simplex 2/MS analog Beispiel 2 infiziert. Im Falle des Alabama Stammes wird an 15-18 dieser Meerschweinchen am dritten Tag nach der Infektion eine einmalige Dosis, wie sie in Figur 4 angegeben ist, Verbindung I in 0,2 ml einer 0,005 Gew.-%igen wässerigen Lösung von Natriumcarboxymethylcellulose oral verabreicht. Im Falle des MS-Stammes wird an 17-19 der obengenannten Meerschweinchen unter leichter Narkose mit einem Gemisch aus gleichen Teilen Diäthyläther, Aethanol und Chloroform am siebten Tag vor der Infektion eine einmalige Dosis, wie sie in Figur 5 angegeben, ist, Verbindung I in 0,2 ml einer 0,005 %igen wässrigen Lösung von Natriumcarboxymethylcellulose intranasal verabreicht.

Die Kontrollgruppen umfassen jeweils 35 Tiere und bleiben unbehandelt (Fig. 4) oder erhalten ein Placebo (Fig. 5). Die Resultate sind in den Figuren 4 bzw. 5 dargestellt.

Beispiel 14

Gruppen von 30 bzw. 34 weiblichen Tif : MF 2f (SPF) Mäusen mit einem Körpergewicht von 14-16 g werden unter leichter Narkose mit 10 bis 50 PFU Parainfluenza Virus 1 (Sendai)/52 (Maus-adaptiert, aufbewahrt bei — 70 °C als Mäuselungen-Suspension in Ampullen) intranasal infiziert. Für die obengenannte Narkose verwendet man ein Gemisch aus gleichen Teilen von Diäthyläther, Alkohol und Chloroform.

10 (im Fall von Fig. 6) bzw. 14 (im Fall von Fig. 7) dieser Mäuse werden zu dem in den Figuren 6 und 7 angegebenen Zeitpunkt, im Falle intranasaler Wirkstoffapplikation vor der Infektion unter Narkose mit einem Gemisch aus gleichen Teilen von Diäthyläther, Alkohol und Chloroform, im Falle intranasaler Wirkstoffapplikation nach der Infektion unter Narkose mit Nembutal (0,5 mg/Maus, intraperitoneal), nach 20-25 Minuten gefolgt von intranasaler Einträufelung von 0,02 ml 1 %iger wässeriger Cinchocainhydrochlorid Lösung, die in den Figuren angegebenen Mengen Verbindung I in 0,2 ml 0,005 Gewichtsprozent Natrium-Carboxymethylcellulose in doppelt-destilliertem Wasser auf die dort angegebene Art einmal appliziert.

Die restlichen 20 Mäuse von jeder der obengenannten Gruppen dienen zur Kontrolle. Die Kontrolltiere sind entweder unbehandelt oder sie erhalten ein Placebo.

Die Wirkung von Verbindung I ist aus den Figuren 6 und 7 ersichtlich.

Beispiel 15

Gruppen von 30 weiblichen Tif : MF-2f (SPF)-Mäusen mit einem Körpergewicht von 14-16 g werden unter leichter Narkose mit einem Gemisch aus gleichen Volumenanteilen Diäthyläther, Aethanol und Chloroform mit letalen Dosen (ungefähr eine $LD_{80-90}$) der in Tabelle 4 genannten Virusstämme intranasal infiziert. Je 10 von diesen Mäusen werden an dem in Tabelle 4 genannten Zeitpunkt [Tage bezogen auf den Tag der Infektion] einmal (Einzeldosis) die jeweilige in Tab. 4 genannte Menge der jeweiligen in Tabelle 4 genannten Wirksubstanz auf die jeweilige in Tabelle 4 genannte Art verabreicht.

Die Wirksubstanzen sind :

N-Acetyl-6-0-{[N-2-(1,2-dipalmitoyl-sn-glycerol-3-hydroxyphosphoryloxy)-äthyl]-succinamoyl}-normuramyl-L-alanyl-D-isoglutamin-natriumsalz (III), N-Acetyl-1,4,6,0-triacetyl-muramyl-L-alanyl-D-isoglutaminyl-L-alanin-2-(1,2-dipalmitoyl-sn-glycero-3-hydroxyphosphoryloxy)-äthylamid-natriumsalz (IV), N-Acetyl-muramyl-L-alanyl-D-isoglutamin-2-(1,2-dipalmitoyl-sn-glycero-3-hydroxyphosphoryloxy)-äthylamid-natriumsalz (V) und N-Acetyl-muramyl-L-alanyl-D-isoglutaminyl-γ-oxymethylcarbanoyl-2-(1,2-dipalmitoyl-sn-glycero-3-hydroxyphosphoryloxy)-äthylamid-natriumsalz (VI).

Die restlichen 20 Mäuse pro Gruppe dienen zur Kontrolle, d. h. sie erhalten anstelle des Wirkstoffes ein Placebo (0,005 Gewichtsprozent Natriumcarboxymethylcellulose in doppelt-destilliertem Wasser).

Die Versuchsergebnisse sind in Tabelle 4 zusammengestellt.

(Siehe Tabelle 4 Seite 24 f.)

Beispiel 16

Herstellung von 1 000 Tabletten, enthaltend 0,5 % Wirkstoff

Zusammensetzung pro 1 000 Tabletten :

| | |
|---|---|
| Verbindung I | 0,5 g |
| Lactose gemahlen | 43,0 g |

Tabelle 4

| Wirkstoff | Virus | Applikationsart | Applikationszeit | Prozentsatz der 23 Tage nach der Infektion noch lebenden Mäuse in Abhängigkeit von der Wirksubstanzmenge [mg/kg] | | | | |
|---|---|---|---|---|---|---|---|---|
| | | | | 100 | 10 | 1 | 0,1 | 0 (Placebo) |
| III | Influenza A/Texas/1/77 ($H_3N_2$) | oral | +7 | | 60 | 60 | 60 | 30 |
| III | Influenza A/Texas/1/77 ($H_3N_2$) | intranasal | -7 | | | 50 | 40 | 5 |
| IV | Influenza A/Texas/1/77 ($H_3N_2$) | oral | +7 | | 50 | 40 | | 15 |
| V | Influenza A/Texas/1/77 ($H_3N_2$) | oral | +7 | 60 | 60 | 80 | | 15 |
| VI | Influenza A/Texas/1/77 ($H_3N_2$) | oral | +7 | 60 | 40 | | | 15 |

| | |
|---|---|
| Maisstärke | 52,0 g |
| Pharmacoat 603® (Hydroxypropylmethylcellulose, enthaltend 28-30 % Methoxylgruppen, geliefert von Shinetsu Chemical Company, Tokio, Japan) | 3,0 g |
| Aerosil® (Kolloidales Siliziumdioxid, geliefert von Degussa, Frankfurt, Bundesrepublik Deutschland) | 1,0 g |
| Magnesiumstearat | 0,5 g |

Herstellung :

Verbindung I und 15 g Lactose werden vorgemischt. Die so erhaltene Vormischung wird mit 28 g Lactose und 47 g Maisstärke zusammengemischt. Mit der so erhaltenen Mischung und einer wässerigen Lösung des Pharmacoat wird eine granulierfertige Masse hergestellt, die granuliert, getrocknet und gemahlen wird. Hierzu mischt man 5 g Maisstärke, Aerosil und Magnesiumstearat und komprimiert zu 1 000 Tabletten mit einem Gewicht von je 100 mg.

Die Presslinge können auf an sich bekannte Weise magensaftresisten lackiert werden.

Beispiel 17

Wirkstoff in Form einer lyophilisierten Trockensubstanz

0,5 mg Verbindung I und 500 mg Mannit (pyrogenfrei) werden in Wasser für Injektionszwecke gelöst und durch einen Membranfilter sterilfiltriert. Unter aseptischen Bedingungen wird die sterilfiltrierte Lösung in eine sterilisierte Glasampulle oder in ein Glasvial abgefüllt und gefriergetrocknet. Nach der Lyophilisation wird die Ampulle verschlossen bzw. das Vial wird mit einem gummielastomeren Verschluss und Alukappe verschlossen.

Beispiel 18

Eindosispipetten mit Nasentropfen

Man stellt eine 0,05 %ige Lösung von Verbindung I in 1,2-Propylenglykol, Benzylalkohol oder Aethanol oder in einem Gemisch aus 1,2-Propylenglykol un Polyäthylenglykol mit dem mittleren Molekulargewicht 300 her.

Die Lösung wird filtriert und in Eindosispipetten aus plastischem Kunststoff abgefüllt. Die Eindosispipetten enthalten die für eine Anwendung benötigte Menge Nasentropfen, d. h. je 0,1 ml der obigen Lösung.

**Patentansprüche**

1. Pharmazeutisches Präparat zur enteralen oder parenteralen Applikation, das als alleinigen Wirkstoff eine wirksame Menge, jedoch weniger als 1 Gewichtsprozent eines Wirkstoffs der Formel I,

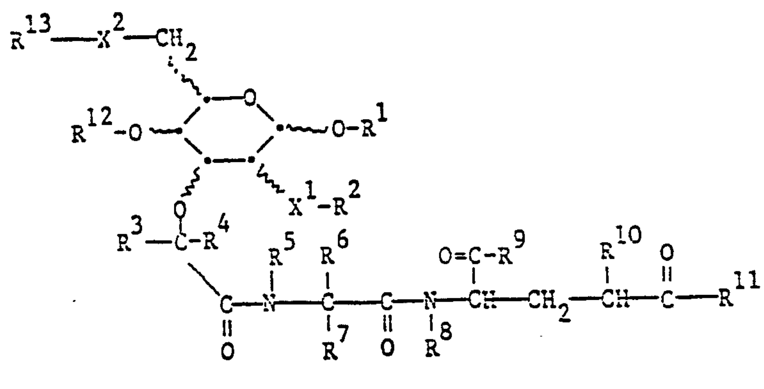

, (I)

worin sich der Zuckerteil von (D)-Galactose, (D)-Mannose oder (D)-Glucose ableitet, der an einem asymmetrisch substituierten C(—$R^3$)-Atom die (D)-Konfiguration, an einem asymmetrisch substituierten C(—$R^6$)-Atom die (L)-Konfiguration und am C(—N—$R^8$)-Atom die (D)-Konfiguration aufweist und worin $X^1$ und $X^2$ unabhängig voneinander eine Gruppe der Formel —O— oder —N($R^{14}$)—, wobei $R^{14}$ für Wasserstoff oder Niederalkyl steht, $R^1$, $R^2$, $R^{12}$ und $R^{13}$ unabhängig voneinander einen Rest der Formel Ia.

$$—(Z^1—Y^1—X^3)_n—A^1 \quad (Ia)$$

worin n für 0 oder 1, $Z^1$ für Carbonyl oder Thiocarbonyl, $Y^1$ für unsubstituiertes oder substituiertes Alkylen, das durch Iminocarbonyl oder Oxycarbonyl unterbrochen sein kann, $X^3$ für eine Gruppe der

Formel —O— oder —N($R^{14}$)—, worin $R^{14}$ die oben gegebene Bedeutung hat, und $A^1$ für einen Rest der Formel Ib,

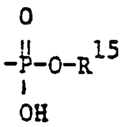

(Ib)

worin $R^{15}$ einen mindestens 7 Kohlenstoffatome aufweisenden aliphatischen oder cycloaliphatischen Rest darstellt, oder für eine Gruppe der Formel Ic,

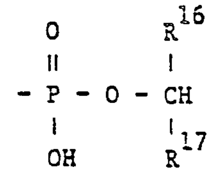

(Ic)

worin $R^{16}$ Wasserstoff und $R^{17}$ 2-Hydroxy- oder 1,2-Dihydroxyäthyl bedeuten, wobei mindestens eine Hydroxygruppe mit einem mindestens 7 Kohlenstoffatome aufweisenden Rest verestert oder veräthert ist, oder worin $R^{16}$ und $R^{17}$ unabhängig voneinander verestertes oder veräthertes Hydroxymethyl bedeuten, wobei die veresternden bzw. veräthernden Reste mindestens 7 Kohlenstoffatome aufweisen, stehen, oder $R^1$, $R^2$, $R^{12}$ und $R^{13}$ unabhängig voneinander Wasserstoff, vom Rest der Formel Ia, worin n für 1 steht, verschiedenes Acyl oder aliphatisch substituiertes Silyl, $R^3$, $R^4$, $R^5$, $R^7$ und $R^8$ unabhängig voneinander Wasserstoff oder Niederalkyl, $R^6$ Wasserstoff oder Niederalkyl, das unsubstituiert oder durch eine Gruppe der Formel Id,

$$—E—(Z^2—Y^2—X^4)_m—A^2 \qquad (Id)$$

worin m für 0 oder 1, E für eine Gruppe der Formel —O—, —S— oder —N($R^{14}$)—, wobei $R^{14}$ die oben gegebene Bedeutung hat, $Z^2$ für Carbonyl oder Thiocarbonyl, $Y^2$ für unsubstituiertes oder substituiertes Alkylen, das durch Iminocarbonyl oder Oxycarbonyl unterbrochen sein kann, $X^4$ für eine Gruppe der Formel —O— oder —N($R^{14}$)—, wobei $R^{14}$ die oben gegebene Bedeutung hat, und $A^2$ für einen Rest der Formel Ib oder Ic stehen, durch freies oder veräthertes Hydroxy oder Mercapto, durch von einer Gruppe der Formel Id verschiedenes verestertes Hydroxy oder Mercapto, durch freies oder von einer Gruppe der Formel Id verschiedenes substituiertes Amino, durch freies, verestertes oder amidiertes Carboxy, oder durch Cycloalkyl, carbocyclisches Aryl oder stickstoffhaltiges Heteroaryl mit 5 bis 6 Ringgliedern im heterocyclischen Ring substituiert ist, oder $R^5$ und $R^6$ zusammen unsubstituiertes oder substituiertes 1,3- oder 1,4-Niederalkylen, $R^9$ und $R^{11}$ unabhängig voneinander einen Rest der Formel Ie,

$$—X^5—Y^3—X^6—A^3 \qquad (Ie)$$

worin $X^5$ für eine Gruppe der Formel —O—, —S— oder —N($R^{14}$)— und $X^6$ für eine Gruppe der Formel —O— oder —N($R^{14}$)—, wobei $R^{14}$ jeweils die oben gegebene Bedeutung hat, $Y^3$ für unsubstituiertes oder substituiertes Alkylen, das durch Iminocarbonyl oder Oxycarbonyl unterbrochen sein kann, und $A^3$ für einen Rest der Formel Ib oder Ic stehen, oder freies Hydroxy oder Mercapto, von einem Rest der Formel Ie verschiedenes veräthertes Hydroxy oder Mercapto, oder freies oder von einem Rest der Formel Ie verschiedenes substituiertes Amino und $R^{10}$ Wasserstoff oder freies, verestertes oder amidiertes Carboxy bedeuten, mit der Massgabe, dass das Präfix « Nieder » Reste bis und mit 7 C-Atomen bezeichnet und dass die Verbindungen der Formel I mindestens einen Rest $A^1$, $A^2$ oder $A^3$ aufweisen, und/oder ein pharmazeutisch verwendbares Salz davon zusammen mit einem pharmazeutischen Trägermaterial enthält.

2. Präparat nach Anspruch 1, das eine Verbindung der Formel I, worin $R^1$, $R^2$, $R^{12}$ und $R^{13}$ unabhängig voneinander für Wasserstoff, den Acylrest einer aliphatischen, cycloaliphatischen, aromatischen oder araliphatischen Carbonsäure mit bis zu 90 Kohlenstoffatomen, Tri-niederalkylsilyl oder einen Rest der Formel Ia stehen, worin n und $X^3$ die in Anspruch 1 gegebenen Bedeutungen haben, $Z^1$ für Carbonyl steht, $Y^1$ Niederalkylen bedeutet, das durch Iminocarbonyl oder Oxycarbonyl unterbrochen sein kann, und $A^1$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder mit einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, $R^6$ Wasserstoff oder Niederalkyl bedeutet, das unsubstituiert oder durch einen Rest der Formel Id substituiert ist, worin m, E und $X^4$ die in Anspruch 1 gegebenen

Bedeutungen haben, $Z^2$ für Carbonyl steht, $Y^2$ Niederalkylen bedeutet, das durch Iminocarbonyl oder Oxycarbonyl unterbrochen sein kann, und $A^2$ für einen Rest der Formel Ib oder Ic, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Acylrest verestertes Hydroxymethyl bedeuten, oder $R^6$ Niederalkyl bedeutet, das durch Hydroxy oder Mercapto, durch mit einem bis zu 90 Kohlenstoffatome enthaltenden aliphatischen Rest veräthertes Hydroxy oder Mercapto, durch mit einem bis zu 90 Kohlenstoffatome aufweisenden aliphatischen Acylrest verestertes Hydroxy oder Mercapto, der von der Gruppe der Formel Id verschieden ist, durch Amino, durch mit einem bis zu 90 Kohlenstoffatome enthaltenden Acylrest substituiertes Amino, der von einem Rest der Formel Id verschieden ist, durch freies Carboxy, Niederalkoxycarbonyl, Carbamoyl, Niederalkylaminocarbonyl, Carboxyniederalkylaminocarbonyl oder amidiertes Carboxyl der Formel Ida

$$-\overset{\displaystyle O}{\overset{\|}{C}} - E - (Y^2 - X^4)_m - A^2 \qquad \text{(Ida)}$$

worin m, E, $Y^2$, $X^4$ und $A^2$ die in Anspruch 1 gegebenen Bedeutungen haben, unsubstituiertes oder durch Hydroxy, Niederalkoxy oder Halogen substituiertes Phenyl oder durch Imidazolyl oder Indolyl substituiert ist, oder $R^5$ und $R^6$ zusammen 1,3- oder 1,4-Niederalkylen bedeuten, $R^9$ und $R^{11}$ unabhängig voneinander Hydroxy, Niederalkoxy, Amino, Niederalkylamino, Carboxyniederalkylamino, oder einen Rest der Formel Ie bedeuten, worin $X^5$ und $X^6$ die im Anspruch 1 gegebenen Bedeutungen haben, $Y^3$ Niederalkylen bedeutet, das durch Iminocarbonyl oder Oxycarbonyl unterbrochen sein kann, und $A^3$ für einen Rest der Formel Ib oder Ic steht, worin $R^{15}$ einen mindestens 7 Kohlenstoffatome aufweisenden aliphatischen Rest bedeutet, $R^{16}$ Wasserstoff und $R^{17}$ 2-Hydroxy- oder 1,2-Dihydroxyäthyl bedeuten, worin mindestens eine Hydroxygruppe mit einem mindestens 7 und bis zu 90 Kohlenstoffatome aufweisenden aliphatischen Rest veräthert oder einem entsprechenden aliphatischen Acylrest verestert ist, oder $R^{16}$ und $R^{17}$ unabhängig voneinander durch einen mindestens 7 und bis zu 90 Kohlenstoffatome aufweisenden aliphatischen Rest veräthertes oder einen entsprechenden aliphatischen Acylrest verestertes Hydroxymethyl bedeuten, und $R^{10}$ für Wasserstoff, Carboxy, Niederalkoxycarbonyl, Carbamoyl, Niederalkylaminocarbonyl oder Carboxyniederalkylaminocarbonyl steht, mit der Massgabe, dass die Verbindungen der Formel I mindestens einen Rest $A^1$, $A^2$ oder $A^3$ aufweisen, und/oder ein pharmazeutisch verwendbares Salz einer solchen Verbindung enthält.

3. Präparat nach Anspruch 1, das eine Verbindung der Formel I, worin $X^1$ für die Gruppe NH und $X^2$ für Sauerstoff stehen, $R^1$ Wasserstoff, Niederalkanoyl oder die Gruppe der Formel Ia bedeutet, worin n für 1 steht, $Z^1$ Carbonyl bedeutet, $Y^1$ Niederalkylen darstellt, das durch Iminocarbonyl unterbrochen sein kann, $X^3$ für eine Gruppe der Formel —O— oder —NH— steht, und $A^1$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen unabhängig voneinander mit einem 7 bis 30 Kohlenstoffatome aufweisenden Alkylrest, der unsubstituiert oder durch Hydroxy, Amino und/oder Alkanoylamino mit bis zu 30 Kohlenstoffatomen substituiert ist, veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, $R^2$ Niederalkanoyl, Hydroxyniederalkanoyl, Benzoyl oder die Gruppe der Formel Ia bedeutet, worin n, $Z^1$, $Y^1$, $X^3$ und $A^1$ die oben in diesem Anspruch gegebenen Bedeutungen haben, $R^{12}$ Wasserstoff oder Niederalkanoyl darstellt, $R^{13}$ Wasserstoff, Alkanoyl oder Hydroxyalkanoyl mit bis zu 90 Kohlenstoffatomen, Alkanoylaminoalkanoyl mit bis zu 30 Kohlenstoffatomen oder die Gruppe der Formel Ia bedeutet, worin n, $Z^1$, $Y^1$, $X^3$ und $A^1$ die oben in diesem Anspruch gegebenen Bedeutungen haben, $R^3$ und $R^7$ für Wasserstoff oder Methyl stehen, $R^4$, $R^5$, $R^8$ und $R^{10}$ für Wasserstoff stehen, $R^6$ Wasserstoff, unsubstituiertes oder durch freies Hydroxy oder Mercapto, Niederalkoxy, Niederalkylthio, Alkanoyloxy oder Hydroxyalkanoyloxy mit bis zu 90 Kohlenstoffatomen, Phenyl, Imidazolyl, Indolyl oder durch eine Gruppe der Formel Id substituiertes Niederalkyl bedeutet, worin m für 1 steht, E, für eine Gruppe der Formel —O— oder —S— steht, $Z^2$ Carbonyl bedeutet, $Y^2$ Niederalkylen darstellt, das durch Iminocarbonyl unterbrochen sein kann, $X^4$ für eine Gruppe der Formel —O— steht, und $A^2$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasser-

stoff und $R^{17}$ 2-Hydroxy- oder 1,2-Dihydroxyäthyl bedeuten, wobei in einem Rest $R^{17}$ die Hydroxygruppen unabhängig voneinander mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, und jeder der Reste $R^9$ und $R^{11}$ unabhängig voneinander für Hydroxy, Niederalkoxy, Amino, Niederalkylamino, Carboxyniederalkylamino oder einen Rest der Formel Ie stehen, worin $X^5$ für eine Gruppe der Formel —O— oder —NH— steht, $Y^3$ Niederalkylen darstellt, das durch Iminocarbonyl unterbrochen sein kann, $X^6$ für eine Gruppe der Formel —O— steht und $A^3$ den Rest der Formel Ib oder Ic bedeutet, worin $R^{15}$ einen 7 bis 30 Kohlenstoffatome aufweisenden unsubstituierten oder durch Hydroxy, Amino und/oder Alkanoylamino mit bis zu 30 Kohlenstoffatomen substituierten Alkylrest darstellt, $R^{16}$ Wasserstoff und $R^{17}$ 2-Hydroxy- oder 1,2-Dihydroxyäthyl bedeuten, wobei in einem Rest $R^{17}$ die Hydroxygruppen unabhängig voneinander mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert sind, oder $R^{16}$ und $R^{17}$ unabhängig voneinander Hydroxymethyl darstellen, worin die Hydroxygruppe mit einem 7 bis 30 Kohlenstoffatome aufweisenden, unsubstituierten oder durch Hydroxy, Amino und/oder Alkanoylamino mit bis zu 30 Kohlenstoffatomen substituierten Alkylrest veräthert oder mit einem 7 bis 90 Kohlenstoffatome aufweisenden, unsubstituierten oder durch Hydroxy, Amino und/oder Alkanoylamino mit bis zu 30 Kohlenstoffatomen substituierten Alkanoylrest verestert ist, mit der Massgabe, dass die Verbindungen mindestens einen Rest aus der Gruppe $A^1$, $A^2$ und $A^3$ aufweisen, und/oder ein pharmazeutisch verwendbares Salz dieser Verbindung enthält.

4. Präparat nach Anspruch 1, das eine Verbindung der Formel I, worin $X^1$ eine Gruppe der Formel —$N(R^{14})$—, wobei $R^{14}$ für Wasserstoff oder $C_{1-4}$-Alkyl steht, $X_2$ Sauerstoff, $R^1$ Wasserstoff, Niederalkanoyl oder die Gruppe der Formel Ia, worin n für 0 oder 1, $Z^1$ für Carbonyl, $Y^1$ für Niederalkylen, das durch Iminocarbonyl unterbrochen sein kann, $X^3$ für Sauerstoff und $A^1$ für einen Rest der Formel Ib oder Ic stehen, worin $R^{15}$ für einen 7-30 Kohlenstoffatome aufweisenden unsubstituierten oder durch Hydroxy, Amino und/oder Alkanoylamino mit bis zu 22 Kohlenstoffatomen substituierten Alkyl- oder Alkenylrest, $R^{16}$ für Wasserstoff und $R^{17}$ für 2-Hydroxy- oder 1,2-Dihydroxyäthyl, wobei in einem Rest $R^{17}$ mindestens eine Hydroxygruppe mit einem 7-30 Kohlenstoffatome aufweisenden Alkyl- oder Alkenylrest veräthert oder mit einem 7-30 Kohlenstoffatome aufweisenden Alkanoyl- oder Alkenoylrest verestert ist, oder $R^{16}$ und $R^{17}$ unabhängig voneinander für Hydroxymethyl, wobei die Hydroxygruppe mit einem 7-30 Kohlenstoffatome aufweisenden Alkyl- oder Alkenylrest veräthert oder mit einem 7-30 Kohlenstoffatome aufweisenden Alkanoyl- oder Alkenoylrest verestert ist, stehen, $R^2$ Niederalkanoyl, Hydroxyniederalkanoyl Benzoyl oder unabhängig von $R^1$, $R^{12}$ und $R^{13}$ eine wie vorstehend in diesem Anspruch definierte Gruppe der Formel Ia, $R^3$, $R^4$, $R^5$, $R^7$ und $R^8$ unabhängig voneinander Wasserstoff, Methyl oder Aethyl, $R^6$ Wasserstoff oder unsubstituiertes oder durch freies Hydroxy, freies Mercapto, Niederalkoxy, Niederalkylthio, Alkanoyloxy mit 2-30 Kohlenstoffatomen, Alkenoyloxy mit 6-30 Kohlenstoffatomen, Phenyl, 4-Hydroxy-phenyl oder durch eine Gruppe der Formel Id substituiertes Niederalkyl, worin m für 0 oder 1, E für Sauerstoff oder Schwefel, $Z^2$ für Carbonyl, $Y^2$ für unsubstituiertes oder durch Phenyl substituiertes Niederalkylen, das durch Iminocarbonyl unterbrochen sein kann, $X^4$ für Sauerstoff und $A^2$ unabhängig von $A^1$ und $A^3$ für einen Rest der wie vorstehend in diesem Anspruch definierten Formeln Ib oder Ic stehen, $R^9$ und $R^{11}$ unabhängig voneinander Hydroxy, Niederalkoxy, Amino, Niederalkylamino, Carboxyniederalkylamino, Niederalkoxycarbonylniederalkylamino, Carbamoylniederalkylamino oder einen Rest der Formel Ie, worin $X^5$ für Sauerstoff oder, NH, $Y^3$ für Niederalkylen, das durch Iminocarbonyl unterbrochen sein kann, $X^6$ für Sauerstoff und $A^3$ unabhängig von $A^1$ und $A^2$ für einen Rest der wie vorstehend in diesem Anspruch definierten Formeln Ib oder Ic stehen, $R^{10}$ Wasserstoff, $R^{12}$ Wasserstoff, Niederalkanoyl, oder den gleichen Rest wie $R^{13}$, und $R^{13}$ Wasserstoff, Alkanoyl oder Alkenoyl mit jeweils bis zu 30 Kohlenstoffatomen oder unabhängig von $R^1$ und $R^2$ einen wie vorstehend in diesem Anspruch definierten Rest der Formel Ia bedeuten, mit der Massgabe, dass die Verbindungen mindestens einen und höchstens zwei Reste aus der Gruppe $A^1$, $A^2$ und $A^3$ aufweisen, und/oder ein pharmazeutisch verwendbares Salz dieser Verbindung enthält.

5. Präparat nach Anspruch 4, das eine Verbindung der Formel I, worin $X^1$ die Gruppe NH, $X^2$ Sauerstoff, $R^1$ Wasserstoff oder Niederalkanoyl, $R^2$ Niederalkanoyl, Benzoyl oder eine Gruppe der Formel Ia, worin n für 0 oder 1, $Z^1$ für Carbonyl, $Y^1$ für Niederalkylen, das durch ein oder zwei Iminocarbonylgruppen unterbrochen sein kann, $X^3$ für Sauerstoff und $A^1$ für einen Rest der Formel Ib oder Ic stehen, worin $R^{15}$ für einen Alkylrest mit 7 bis 22 C-Atomen, $R^{16}$ für Wasserstoff und $R^{17}$ für 1,2-Dihydroxyäthyl stehen, wobei beide Hydroxygruppen unabhängig voneinander jeweils mit einem 10-22 Kohlenstoffatome aufweisenden Alkanoyl- oder Alkenoylrest verestert sind, $R^3$ Wasserstoff oder Methyl,

$R^4$, $R^5$, $R^7$ und $R^8$ Wasserstoff, $R^6$ Wasserstoff oder unsubstituiertes oder durch freies Hydroxy, Alkanoyloxy mit 2 bis 22 Kohlenstoffatomen ; Alkenoyloxy mit 6-22 Kohlenstoffatomen, Phenyl oder durch eine Gruppe der Formel Id substituiertes Niederalkyl, worin m für 0 oder 1, E für Sauerstoff, $Z^2$ für Carbonyl, $Y^2$ für unsubstituiertes oder durch Phenyl substituiertes Niederalkylen, das durch Iminocarbonyl unterbrochen sein kann, $X^4$ für Sauerstoff und $A^2$ unabhängig von $A^1$ und $A^3$ für einen Rest der vorstehend definierten Formeln Ib und Ic stehen, $R^9$ und $R^{11}$ unabhängig voneinander Hydroxy, Niederalkoxy, Amino, Niederalkylamino, α-Carboxy-niederalkylamino, α-Niederalkoxycarbonyl-niederalkylamino, α-Carbamoyl-niederalkylamino oder einen Rest der Formel Ie, worin $X^5$ für die Gruppe NH, $Y^3$ für Niederalkylen, das durch ein oder zwei Iminocarbonylgruppen unterbrochen sein, kann, $X^6$ für Sauerstoff und $A^3$ unabhängig von $A^1$ und $A^2$ für einen Rest der vorstehend definierten Formeln Ib oder Ic stehen, $R^{10}$ Wasserstoff, $R^{12}$ Wasserstoff, Niederalkanoyl oder den gleichen Rest wie $R^{13}$ und $R^{13}$ Wasserstoff, Alkanoyl mit 2 bis 22 Kohlenstoffatomen, Alkenoyl mit 6 bis 22 Kohlenstoffatomen oder unabhängig von $R^2$ einen wie vorstehend definierten Rest der Formel Ia bedeuten, und/oder ein pharmazeutisch verwendbares Salz dieser Verbindungen enthält.

6. Präparat nach Anspruch 5, das eine Verbindung der Formel I, worin $X^1$ die Gruppe NH, $X^2$ Sauerstoff, $R^1$ Wasserstoff oder $C_{2-4}$-Alkanoyl, $R^2$ $C_{2-4}$-Alkanoyl oder eine Gruppe der Formel Ia, worin n für 0 oder 1, $Z^1$ für Carbonyl, $Y^1$ für Niederalkylen, das durch ein oder zwei Iminocarbonylgruppen unterbrochen sein kann, $X^3$ für Sauerstoff und $A^1$ für einen Rest der Formel Ib oder Ic stehen, worin $R^{15}$ für einen unverzweigten Alkylrest mit 12 bis 18 C-Atomen, $R^{16}$ für Wasserstoff und $R^{17}$ für 1,2-Dipalmitoyloxy-äthyl oder 2-Oleoyloxy-1-palmitoyloxyäthyl stehen, $R^3$ Wasserstoff oder Methyl, $R^4$, $R^5$, $R^7$, $R^8$ und $R^{10}$ Wasserstoff, $R^6$ unsubstituiertes oder durch einen Rest der Formel Id substituiertes Methyl, Aethyl oder Isopropyl, worin m für 0 oder 1, E für Sauerstoff, $Z^2$ für Carbonyl, $Y^2$ für unsubstituiertes oder durch Phenyl substituiertes Niederalkylen, das durch ein oder zwei Iminocarbonylgruppen unterbrochen sein kann, $X^4$ für Sauerstoff und $A^2$ unabhängig von $A^1$ und $A^3$ für einen Rest der vorstehend definierten Formeln Ib oder Ic stehen, $R^9$ Amino, $R^{11}$ Hydroxy oder einen Rest der Formel Ie, worin $X^5$ für die Gruppe NH, $Y^3$ Niederalkylen, das durch ein oder zwei Iminocarbonylgruppen unterbrochen sein kann, $X^6$ für Sauerstoff und $A^3$ unabhängig von $A^1$ und $A^2$ für einen Rest der vorstehend definierten Formeln Ib oder Ic stehen, $R^{12}$ Wasserstoff, Acetyl oder den gleichen Rest wie $R^{13}$ und $R^{13}$ Wasserstoff, Acetyl oder, unabhängig von $R^2$, einen wie vorstehend definierten Rest der Formel Ia bedeuten, und/oder ein pharmazeutisch verwendbares Salz dieser Verbindung enthält.

7. Präparat nach einem der Ansprüche 1 bis 6, das eine Verbindung der Formel I, worin die Reste $A^1$, $A^2$ und $A^3$, falls vorhanden, für einen Rest der Formel Ic stehen, und/oder ein pharmazeutisch verwendbares Salz dieser Verbindung enthält.

8. Präparat nach einem der Ansprüche 1 bis 7, das eine Verbindung der Formel I, worin sich der Zuckerteil von (D)-Galactose oder (D)-Mannose ableitet, und/oder ein pharmazeutisch verwendbares Salz dieser Verbindung enthält.

9. Präparat nach einem der Ansprüche 1 bis 7, das eine Verbindung der Formel I, worin sich der Zuckerteil von (D)-Glucose ableitet, und/oder ein pharmazeutisch verwendbares Salz dieser Verbindung enthält.

10. Präparat nach einem der Ansprüche 1 bis 9, das eine Verbindung der Formel I, die nur einen Phosphorylsubstituenten trägt, und zwar im Rest $R^{13}$, und/oder ein pharmazeutisch verwendbares Salz dieser Verbindung enthält.

11. Präparat nach einem der Ansprüche 1 bis 9, das eine Verbindung der Formel I, die nur einen Phosphorylsubstituenten trägt, und zwar im Rest $R^6$, und/oder ein pharmazeutisch verwendbares Salz dieser Verbindung enhält.

12. Präparat nach einem der Ansprüche 1 bis 9, das eine Verbindung der Formel I, die nur einen Phosphorylsubstituenten trägt, und zwar im Rest $R^{11}$, und/oder ein pharmazeutisch verwendbares Salz dieser Verbindung enthält.

13. Präparat nach Anspruch 1, das N-Acetyl-muramyl-L-alanyl-D-isoglutaminyl-L-alanin-2-(1,2-dipalmitoyl-sn-glycero-3-hydroxyphosphoryloxy)-äthylamid und/oder ein pharmazeutisch verwendbares Salz davon enthält.

14. Präparat nach einem der Anspruche 1 bis 13, das ein pharmazeutisch verwendbares Salz einer Verbindung der Formel I enthält.

15. Präparat nach Anspruch 14, das ein Natriumsalz einer Verbindung der Formel I enthält.

16. Präparat nach Anspruch 1, das eine Verbindung der Formel I, die an einem asymmetrisch substituierten C(—$R^3$)-Atom die (D)-Konfiguration, an einem asymmetrisch substituierten C(—$R^6$)-Atom die (L)-Konfiguration und am C(—N—$R^8$)-Atom die (D)-Konfiguration aufweisen, und worin sich der Zuckerteil von (D)-Glucose ableitet, $X^1$ die Gruppe NH, $X^2$ Sauerstoff, $R^1$, $R^4$, $R^5$, $R^7$, $R^8$, $R^{10}$, $R^{12}$ und $R^{13}$ Wasserstoff, $R^2$ $C_{1-3}$-Alkyl oder Phenyl, $R^3$ Wasserstoff oder $C_{1-3}$-Alkyl, $R^6$ Wasserstoff, gegebenenfalls durch Hydroxy, Methoxy, Mercapto, Methylmercapto oder Halogen substituiertes $C_{1-3}$-Alkyl, gegebenenfalls durch Hydroxy, Methoxy oder Halogen substituiertes Phenyl oder Phenylmethyl, ein oder zwei Azaatome enthaltendes Heteroaryl oder Heteroarylmethyl mit 5 Ringgliedern, oder $R^5$ und $R^6$ zusammen Trimethylen, einer der Reste $R^9$ und $R^{11}$ einen Rest der Formel Ie, worin $X^5$ für die Gruppe NH oder Sauerstoff, $Y^3$ für $C_{2-3}$ Alkylen oder für einen Rest der Formeln

$-U^2-COO-U^3-$ oder 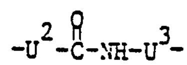 ,

worin $U^2$ und $U^3$ unabhängig voneinander je $C_{1-3}$-Alkylen, das unsubstituiert oder durch gegebenenfalls durch Hydroxy, Niederalkoxy, Mercapto oder Niederalkylmercapto substituiertes $C_{1-3}$-Alkyl oder durch gegebenenfalls durch Hydroxy, Methoxy oder Halogen substituiertes Phenyl oder Phenylniederalkyl oder durch ein oder zwei Azaatome enthaltendes Heterocyclyl oder Heterocyclyl-($C_{1-3}$-alkyl) mit jeweils 5-6 Ringgliedern substituiert ist, bedeuten, $X^6$ für Sauerstoff und $A^3$ für einen Rest der Formel Ic stehen, worin $R^{16}$ Wasserstoff und $R^{17}$ eine 1,2-Dihydroxyäthyl- oder 2-Hydroxyäthylgruppe bedeuten, in der mindestens eine der Hydroxygruppen mit einer gegebenenfalls ein oder zweifach ungesättigten aliphatischen $C_{16-20}$-Carbonsäure verestert oder mit einem gegebenenfalls ein- oder zweifach ungesättigten aliphatischen $C_{12-18}$-Alkohol veräthert ist, und der andere der Reste $R^9$ und $R^{11}$ Hydroxy, Niederalkoxy, Amino, Niederalkylamino oder Aminocarbonylniederalkylamino bedeuten, und/oder ein pharmazeutisch verwendbares Salz einer solchen Verbindung enthält.

17. Präparat nach Anspruch 1, das eine Verbindung der Formel I, die an einem asymmetrisch substituierten C(—$R^3$)-Atom die (D)-Konfiguration, an einem asymmetrisch substituierten C(—$R^6$)-Atom die (L)-Konfiguration und am C(—N—$R^8$)-Atom die (D)-Konfiguration aufweisen, und worin sich der Zuckerteil von (D)-Glucose ableitet, $X^1$ die Gruppe NH, $X^2$ Sauerstoff, $R^1$, $R^4$, $R^7$, $R^8$, $R^{10}$, $R^{12}$ und $R^{13}$ Wasserstoff, $R^2$ Niederalkyl oder Phenyl, $R^3$ und $R^5$ unabhängig voneinander Wasserstoff oder Methyl, $R^6$ $C_{1-4}$-Alkyl, $R^9$ Amino und $R^{11}$ einen Rest der Formel

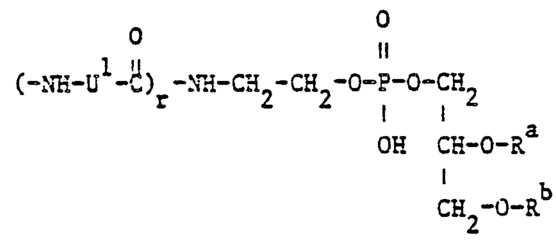

worin r für 0 oder 1, $U^1$ für einen Rest einer der Formeln

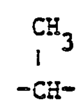 , 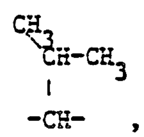 , 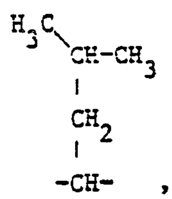 , 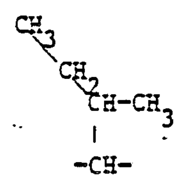

(L) (L) (L) (L)

und $R^a$ und $R^b$ unabhängig voneinander für das Acylradikal einer gesättigten oder zwei Doppelbindungen aufweisenden unsubstituierten aliphatischen Carbonsäure mit 12 bis 22 C-Atomen stehen, bedeuten, und/oder ein pharmazeutisch verwendbares Salz einer solchen Verbindung enthält.

18. Topisch applizierbares pharmazeutisches Präparat nach einem der Ansprüche 1-19, das 0,001 % bis ausschliesslich 1 % Wirkstoff enthält.

19. Augentropfen nach Anspruch 18.

20. Wasserhaltige Nasentropfen nach Anspruch 18.

21. Wasserfreie Nasentropfen nach Anspruch 18.

22. Gel nach Anspruch 18.

23. Creme nach Anspruch 18.

24. Nasensalbe nach Anspruch 18.

25. Hautsalbe nach Anspruch 18.

26. Lippenstift nach Anspruch 18.

27. Pharmazeutisches Präparat nach einem der Ansprüche 1-17 zur oralen Applikation in Form von Tabletten oder Kapseln mit einem Wirkstoffgehalt von 0,1 bis 0,9 Gew.%.

28. Pharmazeutisches Präparat nach einem der Ansprüche 1-17 in Dosiseinheitsform mit einem Wirkstoffgehalt von 0,1-1 mg pro Dosiseinheit.

29. Pharmazeutisches Präparat nach einem der Ansprüche 1-28, welches für die Verwendung an Warmblütern zur Prophylaxe oder Therapie von durch Viren hervorgerufenen Krankheiten bestimmt ist.

30. Verwendung einer in einem der Ansprüche 1-17 definierten Verbindung der Formel I oder eines pharmazeutisch verwendbaren Salzes einer solchen Verbindung zur Herstellung von Arzneimitteln, die für die Anwendung an Warmblütern zur Prophylaxe oder Therapie von durch Viren hervorgerufenen

Krankheiten bestimmt sind.

31. Verwendung nach Anspruch 30 zur Prophylaxe oder Therapie von Krankheiten, die durch Influenza oder Herpes simplex Viren hervorgerufen werden.

32. Verwendung nach Anspruch 30 zur Prophylaxe oder Therapie von Krankheiten, die durch Encephalomyocarditis, Vaccinia oder Parainfluenza Viren hervorgerufen werden.

## Claims

1. A pharmaceutical composition for enteral or parenteral application, which composition contains, as sole active ingredient, an effective amount, but less than 1 % by weight, of a compound of the formula I

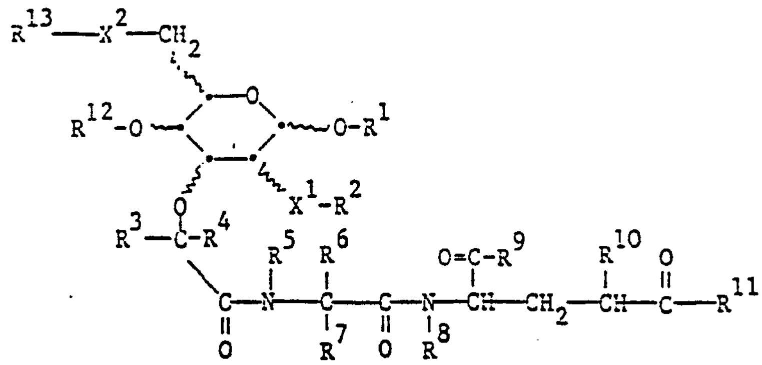

(I)

in which the sugar moiety is derived from (D)-galactose, (D)-mannose or (D)-glucose, which compound has at an asymmetrically substituted C(—$R^3$) atom the (D)-configuration, at an asymmetrically substituted C(—$R^6$) atom the (L)-configuration and at an asymmetrically substituted C(—$R^8$) atom the (D)-configuration, and in which compound each of $X^1$ and $X^2$ independently of the other represents a group of the formula —O— or —N($R^{14}$)-, $R^{14}$ representing hydrogen or lower alkyl, each of $R^1$, $R^2$, $R^{12}$ and $R^{13}$ independently represents a radical of the formula Ia

$$—(Z^1—Y^1—X^3)_n—A^1 \qquad \text{(Ia)}$$

in which n represents 0 or 1, $Z^1$ represents carbonyl or thiocarbonyl, $Y^1$ represents unsubstituted or substituted alkylene which may be interrupted by iminocarbonyl or oxycarbonyl, $X^3$ represents a group of the formula —O— or —N($R^{14}$)-, wherein $R^{14}$ has the meaning given above, and $A^1$ represents a radical of the formula Ib

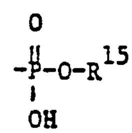

(Ib)

in which $R^{15}$ represents an aliphatic or cycloaliphatic radical having at least 7 carbon atoms, or $A^1$ represents a group of the formula Ic

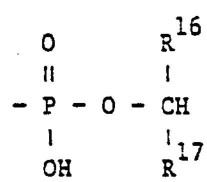

(Ic)

in which $R^{16}$ represents hydrogen and $R^{17}$ represents 2-hydroxyethyl or 1,2-dihydroxyethyl, wherein at least one hydroxy group is esterified or etherified by a radical having at least 7 carbon atoms, or in which each or $R^{16}$ and $R^{17}$ independently of the other represents esterified or etherified hydroxymethyl, the esterifying or etherifying radicals having at least 7 carbon atoms, or each of $R^1$, $R^2$, $R^{12}$ and $R^{13}$ independently represents hydrogen, acyl other than a radical of the formula Ia in which n represents 1, or aliphatically substituted silyl, each of $R^3$, $R^4$, $R^5$, $R^7$ and $R^8$ independently represents hydrogen or lower alkyl, $R^6$ represents hydrogen or lower alkyl that is unsubstituted or substituted by a group of the formula Id

$$-E-(Z^2-Y^2-X^4)_m-A^2 \qquad (Id)$$

in which m represents 0 or 1, E represents a group of the formula —O—, —S— or —N($R^{14}$)—, $R^{14}$ having the meaning given above, $Z^2$ represents carbonyl or thiocarbonyl, $Y^2$ represents unsubstituted or substituted alkylene which may be interrupted by iminocarbonyl or oxycarbonyl, $X^4$ represents a group of the formula —O— or —N($R^{14}$)—, $R^{14}$ having the meaning given above, and $A^2$ represents a radical of the formula Ib or Ic ; or by free or etherified hydroxy or mercapto, by esterified hydroxy or mercapto other than a group of the formula Id, by free amino or substituted amino other than a group of the formula Id, by free, esterified or amidated carboxy, by cycloalkyl, by carbocyclic aryl or by nitrogen-containing heteroaryl having 5 or 6 ring members in the heterocyclic ring, or $R^5$ and $R^6$ together represent unsubstituted or substituted 1,3- or 1,4-lower alkylene, each of $R^9$ and $R^{11}$ independently of the other represents a radical of the formula Ie

$$-X^5-Y^3-X^6-A^3 \qquad (Ie)$$

in which $X^5$ represents a group of the formula —O—, —S— or —N($R^{14}$)—, and $X^6$ represents a group of the formula —O— or —N($R^{14}$)—, in each case $R^{14}$ having the meaning given above, $Y^3$ represents unsubstituted or substituted alkylene which may be interrupted by iminocarbonyl or oxycarbonyl, and $A^3$ represents a radical of the formula Ib or Ic, or free hydroxy or mercapto, etherified hydroxy or mercapto other than a radical of the formula Ie, or free amino or substituted amino other than a radical of the formula Ie, and represents hydrogen or free, esterified or amidated carboxy, with the proviso that radicals qualified by the term « lower » contain up to and including 7 carbon atoms and that the compounds of the formula I have at least one radical $A^1$, $A^2$ or $A^3$ and/or a pharmaceutically acceptable salt thereof, together with a pharmaceutical carrier.

2. A composition according to claim 1, which contains a compound of the formula 1, in which each or $R^1$, $R^2$, $R^{12}$ and $R^{13}$ independently represents hydrogen, the acyl radical of an aliphatic, cycloaliphatic, aromatic or araliphatic carboxylic acid having up to 90 carbon atoms, tri-lower alkylsilyl, or a radical of the formula Ia in which n and $X^3$ have the meanings given in claim 1, $Z^1$ represents carbonyl, $Y^1$ represents lower alkylene which may be interrupted by iminocarbonyl or oxycarbonyl, and $A^1$ represents a radical of the formula Ib or Ic in which $R^{15}$ represents an aliphatic radical having at least 7 carbon atoms, $R^{16}$ represents hydrogen and $R^{17}$ represents 2-hydroxyethyl or 1,2-dihydroxyethyl, in which at least one hydroxy group is etherified by an aliphatic radical having at least 7 and up to 90 carbon atoms or is esterified by a corresponding aliphatic acyl radical, or each of $R^{16}$ and $R^{17}$, independently of the other, represents hydroxymethyl etherified by an aliphatic radical having at least 7 and up to 90 carbon atoms or esterified by a corresponding aliphatic acyl radical, $R^6$ represents hydrogen or lower alkyl that is unsubsituted or substituted by a radical of the formula Id in which m, E and $X^4$ have the meanings given in claim 1, $Z^2$ represents carbonyl, $Y^2$ represents lower alkylene which may be interrupted by iminocarbonyl or oxycarbonyl, and $A^2$ represents a radical of the formula Ib or Ic in which $R^{15}$ represents an aliphatic radical having at least 7 carbon atoms, $R^{16}$ represents hydrogen and $R^{17}$ represents 2-hydroxyethyl or 1,2-dihydroxyethyl, in which at least one hydroxy group is esterified by an aliphatic radical having at least 7 and up to 90 carbon atoms or by a corresponding aliphatic acyl radical, or each of $R^{16}$ and $R^{17}$, independently of the other, represents hydroxymethyl esterified by an aliphatic acyl radical having at least 7 and up to 90 carbon atoms, or $R^6$ represents lower alkyl substituted by hydroxy or mercapto, by hydroxy or mercapto etherified by an aliphatic radical containing up to 90 carbon atoms, by hydroxy or mercapto that is esterified by an aliphatic acyl radical containing up to 90 carbon atoms and is other than the group of the formula Id, by amino, by amino that is substituted by an acyl radical containing up to 90 carbon atoms and is other than a radical of the formula Id, by free carboxy, by lower alkoxycarbonyl, by carbamoyl, by lower alkylaminocarbonyl, by carboxy-lower alkylaminocarbonyl or by amidated carboxyl of the formula

$$-\overset{\displaystyle O}{\overset{\|}{C}}-E-(Y^2-X^4)_m-A^2$$

in which m, E, $Y^2$, $X^4$ and $A^2$ have the meanings given in claim 1, by phenyl that is unsubstituted or substituted by hydroxy, lower alkoxy or halogen, or by imidazolyl or indolyl, or $R^5$ and $R^6$ together represent 1,3- or 1,4- lower alkylene, each of $R^9$ and $R^{11}$, independently of the other, represents hydroxy, lower alkoxy, amino, lower alkylamino, carboxy-lower alkylamino, or a radical of the formula Ie, in which $X^5$ and $X^6$ have the meanings given in claim 1, $Y^3$ represents lower alkylene which may be interrupted by iminocarbonyl or oxycarbonyl, and $A^3$ represents a radical of the formula Ib or Ic, in which $R^{15}$ represents an aliphatic radical having at least 7 carbon atoms, $R^{16}$ represents hydrogen and $R^{17}$ represents 2-hydroxyethyl or 1,2-dihydroxyethyl, in which at least one hydroxy group is etherified by an aliphatic radical having at least 7 and up to 90 carbon atoms or is esterified by a corresponding aliphatic acyl radical, or each of $R^{16}$ and $R^{17}$, independently of the other, represents hydroxymethyl etherified by an

aliphatic radical having at least 7 and up to 90 carbon atoms or esterified by a corresponding aliphatic acyl radical, and $R^{10}$ represents hydrogen, carboxy, lower alkoxycarbonyl, carbamoyl, lower alkylaminocarbonyl or carboxy-lower alkylaminocarbonyl, with the proviso that the compounds of the formula I have at least one radical $A^1$, $A^2$ or $A^3$, and/or a pharmaceutically acceptable salt thereof.

3. A composition according to claim 1 which contains a compound of the formula I, wherein $X^1$ represents the group NH and $X^2$ represents oxygen, $R^1$ represents hydrogen, lower alkanoyl, or a group of the formula Ia in which n represents 1, $Z^1$ represents carbonyl, $Y^1$ represents lower alkylene which may be interrupted by iminocarbonyl, $X^3$ represents a group of the formula —O— or —NH—, and $A^1$ represents a radical of the formula Ib or Ic in which $R^{15}$ represents an alkyl radical having from 7 to 30 carbon atoms that is unsubstituted or substituted by hydroxy, amino and/or alkanoylamino having up to 30 carbon atoms, $R^{16}$ represents hydrogen and $R^{17}$ represents 2-hydroxyethyl or 1,2-dihydroxyethyl, wherein each of the hydroxy groups in a radical $R^{17}$, independently of the other, is etherified by an alkyl radical having from 7 to 30 carbon atoms that is unsubstituted or substituted by hydroxy, amino and/or alkanoylamino having up to 30 carbon atoms, or is esterified by an alkanoyl radical having from 7 to 90 carbon atoms that is unsubstituted or substituted by hydroxy, amino and/or alkanoylamino having up to 30 carbon atoms, or each of $R^{16}$ and $R^{17}$, independently of the other, represents hydroxymethyl in which the hydroxy group is etherified by an alkyl radical having from 7 to 30 carbon atoms that is unsubstituted or substituted by hydroxy, amino and/or alkanoylamino having up to 30 carbon atoms, or is esterified by an alkanoyl radical having from 7 to 90 carbon atoms that is unsubstituted or substituted by hydroxy, amino and/or alkanoylamino having up to 30 carbon atoms, $R^2$ represents lower alkanoyl, hydroxy-lower alkanoyl, benzoyl, or a group of the formula Ia in which n, $Z^1$, $Y^1$, $X^3$ and $A^1$ have the meanings given hereinbefore in this claim, $R^{12}$ represents hydrogen or lower alkanoyl, $R^{13}$ represents hydrogen, alkanoyl or hydroxyalkanoyl having up to 90 carbon atoms, alkanoylaminoalkanoyl having up to 30 carbon atoms, or a group of the formula Ia in which n, $Z^1$, $Y^1$, $X^3$ and $A^1$ have the meanings given hereinbefore in this claim, $R^3$ and $R^7$ each represents hydrogen or methyl, $R^4$, $R^5$, $R^8$ and $R^{10}$ each represents hydrogen, $R^6$ represents hydrogen, lower alkyl that is unsubstituted or substituted by free hydroxy or mercapto, lower alkoxy, lower alkylthio, alkanoyloxy or hydroxyalkanoyloxy having up to 90 carbon atoms, or by phenyl, imidazolyl or indolyl, or by a group of the formula Id in which m represents 1, E represents a group of the formula —O— or —S—, $Z^2$ represents carbonyl, $Y^2$ represents lower alkylene which may be interrupted by iminocarbonyl, $X^4$ represents a group of the formula —O—, and $A^2$ represents a radical of the formula Ib or Ic in which $R^{15}$ represents an alkyl radical having from 7 to 30 carbon atoms that is unsubstituted or substituted by hydroxy, amino and/or alkanoylamino having up to 30 carbon atoms, $R^{16}$ represents hydrogen, and $R^{17}$ represents 2-hydroxyethyl or 1,2-dihydroxyethyl, wherein each of the hydroxy groups in a radical $R^{17}$, independently of the other, is etherified by an alkyl radical having from 7 to 30 carbon atoms that is unsubstituted or substituted by hydroxy, amino and/or alkanoylamino having up to 30 carbon atoms, or is esterified by an alkanoyl radical having from 7 to 90 carbon atoms that is unsubstituted or substituted by hydroxy, amino and/or alkanoylamino having up to 30 carbon atoms, or each of $R^{16}$ and $R^{17}$, independently of the other, represents hydroxymethyl in which the hydroxy group is etherified by an alkyl radical having from 7 to 30 carbon atoms that is unsubstituted or substituted by hydroxy, amino and/or alkanoylamino having up to 30 carbon atoms, or is esterified by an alkanoyl radical having from 7 to 90 carbon atoms that is unsubstituted or substituted by hydroxy, amino and/or alkanoylamino having up to 30 carbon atoms, and each of the radicals $R^9$ and $R^{11}$, independently of the other, represents hydroxy, lower alkoxy, amino, lower alkylamino, carboxy-lower alkylamino, or a radical of the formula Ie in which $X^5$ represents a group of the formula —O— or —NH—, $Y^3$ represents lower alkylene which may be interrupted by iminocarbonyl, $X^6$ represents a group of the formula —O— and $A^3$ represents a radical of the formula Ib or Ic in which $R^{15}$ represents an alkyl radical having from 7 to 30 carbon atoms that is unsubstituted or substituted by hydroxy, amino and/or alkanoylamino having up to 30 carbon atoms, $R^{16}$ represents hydrogen and $R^{17}$ represents 2-hydroxyethyl or 1,2-dihydroxyethyl, wherein each of the hydroxy groups in a radical $R^{17}$, independently of the other, is etherified by an alkyl radical having from 7 to 30 carbon atoms that is unsubstituted or substituted by hydroxy, amino and/or alkanoylamino having up to 30 carbon atoms, or is esterified by an alkanoyl radical having from 7 to 90 carbon atoms that is unsubstituted or substituted by hydroxy, amino and/or alkanoylamino having up to 30 carbon atoms, or each $R^{16}$ and $R^{17}$, independently of the other, represents hydroxymethyl in which the hydroxy group is etherified by an alkyl radical having from 7 to 30 carbon atoms that is unsubstituted or substituted by hydroxy, amino and/or alkanoylamino having up to 30 carbon atoms, or is esterified by an alkanoyl radical having from 7 to 90 carbon atoms that is unsubstituted or substituted by hydroxy, amino and/or alkanoylamino having up to 30 carbon atoms, with the proviso that the compounds contain at least one radical selected from the group $A^1$, $A^2$ and $A^3$, and/or a pharmaceutically acceptable salt thereof.

4. A composition according to claim 1 which contains a compound of the formula I, wherein $X^1$ represents a group of the formula $—N(R^{14})—$, $R^{14}$ representing hydrogen or $C_{1-4}$-alkyl, $X^2$ represents oxygen, $R^1$ represents hydrogen, lower alkanoyl, or a group of the formula Ia in which n represents 0 or 1, $Z^1$ represents carbonyl, $Y^1$ represents lower alkylene which may be interrupted by iminocarbonyl, $X^3$ represents oxygen, and $A^1$ represents a radical of the formula Ib or Ic in which $R^{15}$ represents an alkyl or alkenyl radical having from 7 to 30 carbon atoms that is unsubstituted or substituted by hydroxy, amino

and/or alkanoylamino having up to 22 carbon atoms, $R^{16}$ represents hydrogen and $R^{17}$ represents 2-hydroxyethyl or 1,2-dihydroxyethyl, at least one hydroxy group in a radical $R^{17}$ being etherified by an alkyl or alkenyl radical having from 7 to 30 carbon atoms, or being esterified by an alkanoyl or alkenoyl radical having from 7 to 30 carbon atoms, or each of $R^{16}$ and $R^{17}$, independently of the other, represents hydroxymethyl in which the hydroxy group is etherified by an alkyl or alkenyl radical having from 7 to 30 carbon atoms, or is esterified by an alkanoyl or alkenoyl radical having from 7 to 30 carbon atoms, $R^2$ represents lower alkanoyl, hydroxy-lower alkanoyl, benzoyl or, independently of $R^1$, $R^{12}$ and $R^{13}$, a group of the formula Ia as defined hereinbefore in this claim, each of $R^3$, $R^4$, $R^5$, $R^7$ and $R^8$ independently represents hydrogen, methyl or ethyl, $R^6$ represents hydrogen, or lower alkyl that is unsubstituted or substituted by free hydroxy, free mercapto, lower alkoxy, lower alkylthio, alkanoyloxy having from 2 to 30 carbon atoms, alkenoyloxy having from 6 to 30 carbon atoms, phenyl, 4-hydroxyphenyl, or by a group of the formula Id in which m represents 0 or 1, E represents oxygen or sulphur, $Z^2$ represents carbonyl, $Y^2$ represents lower alkylene that is unsubstituted or substituted by phenyl and may be interrupted by iminocarbonyl, $X^4$ represents oxygen and $A^2$, independently of $A^1$ and $A^3$, represents a radical of the formula Ib or Ic as defined hereinbefore in this claim, each of $R^9$ and $R^{11}$, independently of the other, represents hydroxy, lower alkoxy, amino, lower alkylamino, carboxy-lower alkylamino, lower alkoxycarbonyl-lower alkylamino, carbamoyl-lower alkylamino, or a radical of the formula Ie in which $X^5$ represents oxygen or NH, $Y^3$ represents lower alkylene which may be interrupted by iminocarbonyl, $X^6$ represents oxygen and $A^3$, independently of $A^1$ and $A^2$, represents a radical of the formula Ib or Ic as defined hereinbefore in this claim, $R^{10}$ represents hydrogen, $R^{12}$ represents hydrogen, lower alkanoyl or the same radical as $R^{13}$, and $R^{13}$ represents hydrogen, or alkanoyl or alkenoyl each having up to 30 carbon atoms or, independently of $R^1$ and $R^2$, a radical of the formula Ia as defined hereinbefore in this claim, with the proviso that the compounds contain at least one, and at most two, radical selected from the group $A^1$, $A^2$ and $A^3$, and/or a pharmaceutically acceptable salt thereof.

5. A composition according to claim 4, which contains a compound of the formula I, wherein $X^1$ represents the group NH, $X^2$ represents oxygen, $R^1$ represents hydrogen or lower alkanoyl, $R^2$ represents lower alkanoyl, benzoyl or a group of the formula Ia in which n represents 0 or 1, $Z^1$ represents carbonyl, $Y^1$ represents lower alkylene which may be interrupted by one or two iminocarbonyl groups, $X^3$ represents oxygen, and $A^1$ represents a radical of the formula Ib or Ic in which $R^{15}$ represents an alkyl radical having from 7 to 22 carbon atoms, $R^{16}$ represents hydrogen, and $R^{17}$ represents 1,2-dihydroxyethyl in which each of the hydroxy groups, independently of the other, is esterified by an alkanoyl or alkenoyl radical having from 10 to 22 carbon atoms, $R^3$ represents hydrogen or methyl, $R^4$, $R^5$, $R^7$ and $R^8$ each represents hydrogen, $R^6$ represents hydrogen, or lower alkyl that is unsubstituted or substituted by free hydroxy, alkanoyloxy having from 2 to 22 carbon atoms, alkenoyloxy having from 6 to 22 carbon atoms, phenyl or by a group of the formula Id in which m represents 0 or 1, E represents oxygen, $Z^2$ represents carbonyl, $Y^2$ represents lower alkylene that is unsubstituted or substituted by phenyl and may be interrupted by imino-carbonyl, $X^4$ represents oxygen and $A^2$, independently of $A^1$ and $A^3$, represents a radical of the above-defined formula Ib or Ic, each of $R^9$ and $R^{11}$, independently of the other, represents hydroxy, lower alkoxy, amino lower alkylamino, $\alpha$-carboxy-lower alkylamino, $\alpha$-lower alkoxycarbonyl-lower alkylamino, $\alpha$-carbamoyl-lower alkylamino, or a radical of the formula Ie in which $X^5$ represents the group NH, $Y^3$ represents lower alkylene which may be interrupted by one or two iminocarbonyl groups, $X^6$ represents oxygen and $A^3$, independently of $A^1$ and $A^2$, represents a radical of the formula Ib or Ic defined hereinbefore, $R^{10}$ represents hydrogen, $R^{12}$ represents hydrogen, lower alkanoyl or the same radical as $R^{13}$, and $R^{13}$ represents hydrogen, alkanoyl having from 2 to 22 carbon atoms, alkenoyl having from 6 to 22 carbon atoms or, independently of $R^2$, a radical of the formula Ia as defined hereinbefore, and/or a pharmaceutically acceptable salt thereof.

6. A compound according to claim 5, which contains a compound of the formula I, wherein $X^1$ represents the group NH, $X^2$ represents oxygen, $R^1$ represents hydrogen or $C_{2-4}$-alkanoyl, $R^2$ represents $C_{2-4}$-alkanoyl or a group of the formula Ia in which n represents 0 or 1, $Z^1$ represents carbonyl, $Y^1$ represents lower alkylene which may be interrupted by one or two iminocarbonyl groups, $X^3$ represents oxygen, and $A^1$ represents a radical of the formula Ib or Ic in which $R^{15}$ represents an unbranched alkyl radical having from 12 to 18 carbon atoms, $R^{16}$ represents hydrogen and $R^{17}$ represents 1,2-dipalmitoyloxyethyl or 2-oleoyloxy-1-palmitoyloxyethyl, $R^3$ represents hydrogen or methyl, $R^4$, $R^5$, $R^7$, $R^8$ and $R^{10}$ each represents hydrogen, $R^6$ represents methyl, ethyl or isopropyl, each of which is unsubstituted or substituted by a radical of the formula Id in which m represents 0 or 1, E represents oxygen, $Z^2$ represents carbonyl, $Y^2$ represents lower alkylene that is unsubstituted or substituted by phenyl and may be interrupted by one or two iminocarbonyl groups, $X^4$ represents oxygen and $A^2$, independently of $A^1$ and $A^3$, represents a radical of the formula Ib or Ic defined hereinbefore, $R^9$ represents amino, $R^{11}$ represents hydroxy or a radical of the formula Ie in which $X^5$ represents the group NH, $Y^3$ represents lower alkylene which may be interrupted by one or two iminocarbonyl groups, $X^6$ represents oxygen and $A^3$, independently of $A^1$ and $A^2$, represents a radical of the formula Ib or Ic defined hereinbefore, $R^{12}$ represents hydrogen, acetyl or the same radical as $R^{13}$, and $R^{13}$ represents hydrogen, acetyl or, independently of $R^2$, a radical of the formula Ia as defined hereinbefore, and/or a pharmaceutically acceptable salt thereof.

7. A composition according to any one of claims 1 to 6 which contains a compound of formula I, in

which the radicals $A^1$, $A^2$ and $A^3$, if present, represent a radical of the formula Ic, and/or a pharmaceutically acceptable salt thereof.

8. A composition according to any one of claims 1 to 7 which contains a compound of formula I, in which the sugar moiety is derived from (D)-galactose or (D)-mannose, and/or a pharmaceutically acceptable salt thereof.

9. A composition according to any one of claims 1 to 7 which contains a compound of formula I, in which the sugar moiety is derived from (D)-glucose, and/or a pharmaceutically acceptable salt thereof.

10. A composition according to any one of claims 1 to 9 which contains a compound of formula I that carries only one phosphoryl substituent, namely in the radical $R^{13}$, and/or a pharmaceutically acceptable salt thereof.

11. A composition according to any one of claims 1 to 9 which contains a compound of formula I that carries only one phosphoryl substituent, namely in the radical $R^6$ and/or a pharmaceutically acceptable salt thereof.

12. A composition according to any one of claims 1 to 9 which contains a compound of formula I that carries only one phosphoryl substituent, namely in the radical $R^{11}$, and/or a pharmaceutically acceptable salt thereof.

13. A composition according to claim 1 which contains N-acetylmuramyl-L-alanyl-D-isoglutaminyl-L-alanine-2-(1,2-dipalmitoyl-sn-glycero-3-hydroxyphosphoryloxy)-ethylamide and/or a pharmaceutically acceptable salt thereof.

14. A composition according to any of claims 1 to 13 which contains a pharmaceutically acceptable salt of a compound of the formula I.

15. A composition according to claim 14 which contains a sodium salt of a compound of the formula I.

16. A composition according to claim 1 which contains a compound of formula I that has at an asymmetrically substituted C(-$R^3$)-atom the (D)-configuration, at an asymmetrically substituted C(-$R^6$)-atom the (L)-configuration, and at the C(-N-$R^8$)-atom the (D)-configuration, and in which the sugar moiety is derived from (D)-glucose, $X^1$ represents the group NH, $X^2$ represents oxygen, $R^1$, $R^4$, $R^5$, $R^7$, $R^8$, $R^{10}$, $R^{12}$ and $R^{13}$ each represents hydrogen, $R^2$ represents $C_{1-3}$-alkyl or phenyl, $R^3$ represents hydrogen of $C_{1-3}$-alkyl, $R^6$ represents hydrogen, $C_{1-3}$-alkyl optionally substituted by hydroxy, methoxy, mercapto, methylmercapto or by halogen, phenyl or phenylmethyl each optionally substituted by hydroxy, methoxy or halogen, or heterocyclyl or heterocyclylmethyl each containing one or two aza atoms and having 5 ring members, or $R^5$ and $R^6$ together represent trimethylene, one of the radicals $R^9$ and $R^{11}$ represents a radical of the formula Ie in which $X^5$ represents the group NH or oxygen, $Y^3$ represents $C_{2-3}$-alkylene or a radical of the formula

$$-U^2-COO-U^3- \quad \text{or} \quad -U^2-\overset{O}{\overset{\|}{C}}-NH-U^3- ,$$

in which each of $U^2$ and $U^3$, independently of the other, represents $C_{1-3}$-alkylene that is unsubstituted or substituted by $C_{1-3}$-alkyl optionally substituted by hydroxy, lower alkoxy, mercapto or by lower alkylmercapto, or by phenyl or phenyl-lower alkyl each of which is optionally substituted by hydroxy, methoxy or halogen, or by heterocyclyl or heterocyclyl-($C_{1-3}$-alkyl) each containing one or two aza atoms and having 5 or 6 ring members, $X^6$ represents oxygen, and $A^3$ represents a radical of the formula Ic in which $R^{16}$ represents hydrogen and $R^{17}$ represents a 1,2-dihydroxyethyl or 2-hydroxyethyl group in which at last one of the hydroxy groups is esterified by an aliphatic $C_{16-20}$-carboxylic acid that is optionally singly or doubly unsaturated, or is etherified by an aliphatic $C_{12-18}$-alkohol that is optionally singly or doubly unsaturated, and the other of the radicals $R^9$ and $R^{11}$ represents hydroxy, lower alkoxy, amino, lower alkylamino or aminocarbonyl-lower alkylamino, and/or a pharmaceutically acceptable salt thereof.

17. A composition according to claim 1 which contains a compound of the formula I that has at an asymmetrically substituted C(-$R^3$)-atom the (D)-configuration, at an asymmetrically substituted C(-$R^6$)-atom the (L)-configuration and at the C(-N-$R^8$)-atom the (D)-configuration, and in which the sugar moiety is derived from (D)-glucose, $X^1$ represents the group NH, $X^2$ represents oxygen, $R^1$, $R^4$, $R^7$, $R^8$, $R^{10}$, $R^{12}$ and $R^{13}$ each represents hydrogen, $R^2$ represents lower alkyl or phenyl, each of $R^3$ and $R^5$, independently of the other, represents hydrogen or methyl, $R^6$ represents $C_{1-4}$-alkyl, $R^9$ represents amino and $R^{11}$ represents a radical of the formula

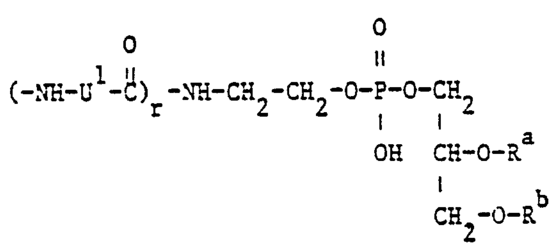

in which r represents 0 or 1, $U^1$ represents a radical of the formula

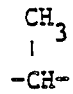 , 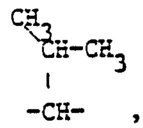 , 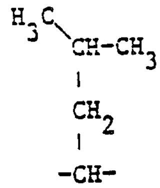 , 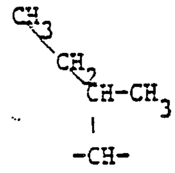

(L) (L) (L) (L)

and each of $R^a$ and $R^b$, independently of the other, represents the acyl radical of a saturated aliphatic carboxylic acid having from 12 to 22 carbon atoms or of an unsubstituted aliphatic carboxylic acid having from 12 to 22 carbon atoms and containing one or two double bonds, and/or a pharmaceutically acceptable salt thereof.

18. A pharmaceutical composition for topical application according to any one of claims 1 to 19 that contains from 0.001 % up to, but exclusive of, 1 % of active ingredient.

19. Eye drops according to claim 18.

20. Aqueous nose drops according to claim 18.

21. Anhydrous nose drops according to claim 18.

22. Gel according to claim 18.

23. Cream according to claim 18.

24. Nasal ointment according to claim 18.

25. Skin ointment according to claim 18.

26. Lipstick according to claim 18.

27. A pharmaceutical composition according to any one of claims 1 to 17 for oral administration in the form of tablets or capsules with an active ingredient content of 0.1 to 0.9 % by weight.

28. A pharmaceutical composition according to any one of claims 1 to 17 in dosage unit form having an active ingredient content of 0.1 to 1 mg per dosage unit.

29. A pharmaceutical composition according to any one of claims 1 to 28 for administration to warm-blooded animals for the prophylaxis or therapy of diseases caused by viruses.

30. Use of a compound of the formula I as defined in any one of claims 1 to 17, or of a pharmaceutically acceptable salt thereof, for the preparation of medicaments for administration to warm-blooded animals for the prophylaxis or therapy of diseases caused by viruses.

31. Use according to claim 30 for the prophylaxis or therapy of diseases caused by influenza or Herpes simplex viruses.

32. Use according to claim 30 for the prophylaxis or therapy of diseases caused by encephalomyocarditis, vaccinia or para-influenza viruses.

**Revendications**

1. Préparation pharmaceutique pour l'administration entérale ou parentérale, qui contient comme substance active unique une quantité efficace, mais inférieure à 1 % en poids, d'une substance active répondant à la formule I,

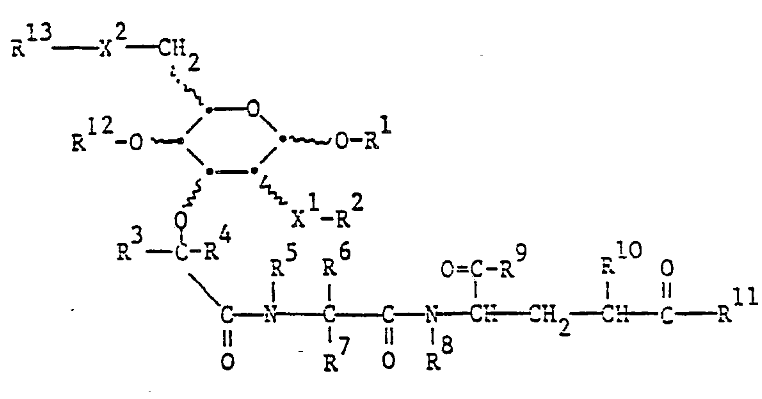

(I)

dans laquelle la partie sucre dérive du (D)-galactose, du (D)-mannose ou du (D)-glucose, qui présente sur un atome C(-$R^3$) substitué asymétriquement, la configuration (D), sur un atome C(-$R^6$) substitué

asymétriquement, la configuration (L) et sur l'atome C(-N-$R^8$), la configuration (D), et dans laquelle $X^1$ et $X^2$ désignent indépendamment l'un de l'autre un groupe répondant à la formule —O— ou —N($R^{14}$)—, dans laquelle $R^{14}$ désigne l'hydrogène ou un groupe alkyle inférieur, $R^1$, $R^2$, $R^{12}$ et $R^{13}$ désignent indépendamment l'un de l'autre un radical répondant à la formule Ia.

$$—(Z^1—Y^1—X^3)_n—A^1 \qquad \text{(Ia)}$$

dans laquelle n est égal à 0 ou 1, $Z^1$ désigne un groupe carbonyle ou thiocarbonyle, $Y^1$ un groupe alkylène non substitué ou substitué, qui peut être interrompu par un groupe iminocarbonyle ou oxycarbonyle, $X^3$ un groupe répondant à la formule —O— ou —N($R^{14}$)—, dans laquelle $R^{14}$ a la signification indiquée ci-dessus et $A^1$ désigne un radical répondant à la formule Ib,

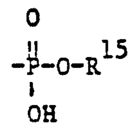

(Ib)

dans laquelle $R^{15}$ représente un radical aliphatique ou cycloaliphatique présentant au moins 7 atomes de carbone, ou désigne un groupe répondant à la formule Ic,

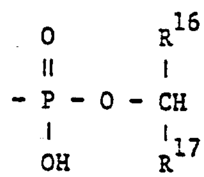

(Ic)

dans laquelle $R^{16}$ désigne l'hydrogène et $R^{17}$ un groupe 2-hydroxy ou 1,2-dihydroxyéthyle, au moins un groupe hydroxy étant estérifié ou éthérifié avec un radical présentant au moins 7 atomes de carbone, ou dans laquelle $R^{16}$ et $R^{17}$ désignent indépendamment l'un de l'autre un groupe hydroxyméthyle estérifié ou éthérifié, les radicaux estérifiants ou éthérifiants présentant au moins 7 atomes de carbone, ou bien $R^1$, $R^2$, $R^{12}$ et $R^{13}$ désignent indépendamment les uns des autres l'hydrogène, un groupe acyle différent du radical répondant à la formule Ia, dans laquelle n est égal à 1, ou un groupe silyle aliphatiquement substitué, $R^3$, $R^4$, $R^5$, $R^7$ et $R^8$ désignent indépendamment les uns des autres l'hydrogène ou un groupe alkyle inférieur, $R^6$ désigne l'hydrogène ou un groupe alkyle inférieur, qui peut être non substitué ou substitué par un groupe répondant à la formule Id,

$$—E—(Z^2—Y^2—X^4)_m—A^2 \qquad \text{(Id)}$$

dans laquelle m est égal à 0 ou 1, E désigne un groupe répondant à la formule —O—, —S— ou —N($R^{14}$)—, dans laquelle $R^{14}$ a la signification indiquée ci-dessus, $Z^2$ désigne un groupe carbonyle ou thiocarbonyle, $Y^2$ un groupe alkylène non substitué ou substitué, qui peut être interrompu par un groupe iminocarbonyle ou oxycarbonyle, $X^4$ un groupe répondant à la formule —O— ou —N($R^{14}$)—, $R^{14}$ ayant la signification indiquée ci-dessus, et $A^2$ un radical répondant à la formule Ib, par un groupe hydroxy ou mercapto libre ou éthérifié, par un groupe hydroxy ou mercapto estérifié différent d'un groupe répondant à la formule Id, par un groupe amino substitué libre ou différent d'un groupe répondant à la formule Id, par un groupe carboxy libre, estérifié ou amidé, ou par un groupe cycloalkyle, arylcarbocyclique ou éthéroaryle azoté ayant 5 à 6 maillons dans le cycle hétérocyclique, ou bien $R^5$ et $R^6$ représentent ensemble un groupe 1,3- ou 1,4-alkylène inférieur non substitué ou substitué, $R^9$ et $R^{11}$ représentent indépendamment l'un de l'autre un radical répondant à la formule Ie,

$$—X^5—Y^3—X^6—A^3 \qquad \text{(Ie)}$$

dans laquelle $X^5$ désigne un groupe répondant à la formule —O—, —S— ou —N($R^{14}$)— et $X^6$ désigne un groupe répondant à la formdule —O— ou —N($R^{14}$)—, $R^{14}$ ayant à chaque fois la signification indiquée ci-dessus, $Y^3$ désigne un groupe alkylène non substitué ou substitué, qui peut être interrompu par un groupe iminocarbonyle ou oxycarbonyle, et $A^3$ désigne un radical répondant à la formule Ib ou Ic, ou un groupe hydroxy ou mercapto libre, un groupe hydroxy ou mercapto éthérifié différent d'un radical répondant à la formule Ie, ou un groupe amino substitué libre ou différent d'un radical répondant à la formule Ie et $R^{10}$ désigne l'hydrogène ou un groupe carboxy libre, estérifié ou amidé, sous réserve que le qualificatif « inférieur » désigne des radicaux ayant jusqu'à 7 atomes de carbone compris et que les composés répondant à la formule I présentent au moins un radical $A^1$, $A^2$ ou $A^3$, et/ou un sel pharmaceutiquement utilisable de celle-ci, avec une matière de support pharmaceutique.

2. Préparation selon la revendication 1, qui contient un composé répondant à la formule I, dans lequel $R^1$, $R^2$, $R^{12}$ et $R^{13}$ désignent indépendamment les uns des autres l'hydrogène, le radical acyle d'un acide carboxylique aliphatique, cycloaliphatique, aromatique ou araliphatique ayant jusqu'à 90 atomes de carbone, un groupe tri(alkyle inférieur) silyle, ou un radical répondant à la formule Ia, dans laquelle n et $X^3$ ont les significations indiquées dans la revendication 1, $Z^1$ désigne un groupe carbonyle, $Y^1$ désigne un groupe alkylène inférieur, qui peut être interrompu par un groupe iminocarbonyle ou oxycarbonyle, et $A^1$ désigne le radical répondant à la formule Ib ou Ic, dans laquelle $R^{15}$ représente un radical aliphatique présentant au moins 7 atomes de carbone, $R^{16}$ désigne l'hydrogène et $R^{17}$ un groupe 2-hydroxy- ou 1,2-dihydroxyéthyle, dans lequel au moins un groupe hydroxy est éthérifié avec un radical aliphatique présentant au moins 7 et jusqu'à 90 atomes de carbone, ou estérifié avec un radical acyle aliphatique approprié, ou bien $R^{16}$ et $R^{17}$ représentent indépendamment l'un de l'autre un groupe hydroxyméthyle éthérifié par un radical aliphatique présentant au moins 7 et jusqu'à 90 atomes de carbone ou estérifié par un radical acyle aliphatique approprié, $R^6$ désigne l'hydrogène ou un groupe alkyle inférieur qui est non substitué ou substitué par un radical répondant à la formule Id, dans laquelle m, E et $X^4$ ont les significations indiquées dans la revendication 1, $Z^2$ désigne un groupe carbonyle, $Y^2$ désigne un groupe alkylène inférieur qui peut être interrompu par un groupe iminocarbonyle ou oxycarbonyle, et $A^2$ désigne un radical répondant aux formules Ib ou Ic, dans lesquelles $R^{15}$ désigne un radical aliphatique présentant au moins 7 atomes de carbone, $R^{16}$ l'hydrogène et $R^{17}$ un groupe 2-hydroxy- ou 1,2-dihydroxyéthyle, dans laquelle au moins un groupe hydroxy est estérifié par un radical aliphatique présentant au moins 7 et jusqu'à 90 atomes de carbone ou estérifié par un radical acyle aliphatique approprié, ou bien $R^{16}$ et $R^{17}$ désignent indépendamment l'un de l'autre un groupe hydroxyméthyle estérifié par un radical acyle aliphatique présentant au moins 7 et jusqu'à 90 atomes de carbone, ou bien $R^6$ désigne un groupe alkyle inférieur qui est substitué par un groupe hydroxy ou mercapto, par un groupe hydroxy ou mercapto éthérifié par un radical aliphatique contenant jusqu'à 90 atomes de carbone, par un groupe hydroxy ou mercapto estérifié par un radical acyle aliphatique présentant jusqu'à 90 atomes de carbone, qui est différent du groupe répondant à la formule Id, par un groupe amino, par un groupe amino substitué par un radical acyle contenant jusqu'à 90 atomes de carbone, qui est différent d'un radical répondant à la formule Id, par un groupe carboxy libre, par un groupe (alcoxy inférieur) carbonyle, carbamoyle, (alkyle inférieur) aminocarbonyle, carboxy(alkyle inférieur)-aminocarbonyle ou carboxylamidé répondant à la formule Ida

$$-\overset{O}{\overset{\|}{C}}-E-(Y^2-X^4)_m-A^2 \qquad \text{(Ida)}$$

dans laquelle m, E, $Y^2$, $X^4$ et $A^2$ ont les significations indiquées dans la revendication 1, par un groupe phényle non substitué ou substitué par un groupe hydroxy, alcoxy inférieur ou par un halogène, ou par un groupe imidazolyle ou indolyle, ou bien $R^5$ et $R^6$ désignent ensemble un groupe 1,3- ou 1,4-alkylène inférieur, $R^9$ et $R^{11}$ désignent indépendamment l'un de l'autre un groupe hydroxy, alcoxy inférieur, amino, alkylamino inférieur, carboxy(alkylamino inférieur) ou un radical répondant à la formule Ie, dans laquelle $X^5$ et $X^6$ ont les significations indiquées dans la revendication 1, $Y^3$ désigne un groupe alkylène inférieur qui peut être interrompu par un groupe iminocarbonyle ou oxycarbonyle, et $A^3$ désigne un radical répondant aux formules Ib ou Ic, dans lesquelles $R^{15}$ désigne un radical aliphatique présentant au moins 7 atomes de carbone, $R^{16}$ l'hydrogène et $R^{17}$ un groupe 2-hydroxy- ou 1,2-dihydroxyéthyle, dans laquelle au moins un groupe hydroxy est éthérifié par un radical aliphatique présentant au moins 7 et jusqu'à 90 atomes de carbone ou est estérifié par un radical acyle aliphatique approprié, ou bien $R^{16}$ et $R^{17}$ désignent indépendamment l'un de l'autre un groupe hydroxyméthyle éthérifié par un radical aliphatique présentant au moins 7 et jusqu'à 90 atomes de carbone ou estérifié par un radical acyle aliphatique approprié, et $R^{10}$ désigne l'hydrogène, un groupe carboxy, (alcoxy inférieur) carbonyle, carbamoyle, (alkyle inférieur) aminocarbonyle ou carboxy(alkyle inférieur) aminocarbonyle, sous réserve que les composés répondant à la formule I présentent au moins un radical $A^1$, $A^2$ ou $A^3$, et/ou un sel pharmaceutiquement utilisable d'un tel composé.

3. Préparation selon la revendication 1, qui contient un composé répondant à la formule I, dans lequel $X^1$ désigne le groupe NH et $X^2$ désigne l'oxygène, $R^1$ représente l'hydrogène, un groupe alcanoyle inférieur ou le groupe répondant à la formule Ia, dans laquelle n est égal à 1, $Z^1$ désigne un groupe carbonyle, $Y^1$ représente un groupe alkylène inférieur qui peut être interrompu par un groupe iminocarbonyle, $X^3$ représente un groupe répondant à la formule —O— ou —NH—, et $A^1$ le radical répondant aux formules Ib ou Ic, dans lesquelles $R^{15}$ représente un radical alkyle non substitué ou substitué par un groupe hydroxy, amino et/ou alcanoylamino ayant jusqu'à 30 atomes de carbone, $R^{16}$ représente l'hydrogène et $R^{17}$ 2-hydroxy- ou 1,2-dihydroxyéthyle, les groupes hydroxyle étant éthérifiés indépendamment l'un de l'autre par un radical alkyle ayant 7 à 3 atomes de carbone, qui peut être non substitué ou substitué par un groupe hydroxy, amino et/ou alcanoylamino ayant jusqu'à 30 atomes de carbone, ou estérifiés par un radical alcanoyle présentant 7 à 90 atomes de carbone, non substitué ou substitué par un groupe hydroxy, amino et/ou alcanoylamino ayant jusqu'à 30 atomes de carbone, ou bien $R^{16}$ et $R^{17}$ représentent indépendamment l'un de l'autre un groupe hydroxyle dans lequel le groupe

hydroxy est éthérifié par un radical alkyle présentant 7 à 30 atomes de carbone, non substitué ou substitué par un groupe hydroxy, amino et/ou alcanoylamino ayant jusqu'à 30 atomes de carbone, ou estérifié par un radical alcanoyle ayant de 7 à 90 atomes de carbone, non substitué ou substitué par un groupe hydroxy, amino et/ou alcanoylamino ayant jusqu'à 30 atomes de carbone, $R^2$ est un groupe alcanoyle inférieur, hydroxy(alcanoyle inférieur), benzoyle ou le groupe répondant à la formule Ia, dans laquelle n, $Z^1$, $Y^1$, $X^3$ et $A^1$ ont les significations indiquées dans cette revendication, $R^{12}$ représente l'hydrogène ou un groupe alcanoyle inférieur, $R^{13}$ représente l'hydrogène, un groupe alcanoyle ou hydroxyalcanoyle ayant jusqu'à 90 atomes de carbone, un groupe alcanoylaminoalcanoyle ayant jusqu'à 30 atomes de carbone ou le groupe répondant à la formule Ia, dans laquelle n, $Z^1$, $Y^1$, $X^3$ et $A^1$ ont les significations indiquées ci-dessus dans cette revendication, $R^3$ et $R^7$ représentent l'hydrogène ou un groupe méthyle, $R^4$, $R^5$, $R^8$ et $R^{10}$ représentent l'hydrogène, $R^6$ désigne l'hydrogène, un groupe alkyle inférieur non substitué ou substitué par un groupe hydroxy ou mercapto libre, un groupe alcoxy inférieur, alkylthio inférieur, alcanoyloxy ou hydroxyalcanoyloxy ayant jusqu'à 90 atomes de carbone, phényle, imidazolyle, indolyle ou par un groupe répondant à la formule Id, dans laquelle m est égal à 1, E représente un groupe répondant à la formule —O— ou —S—, $Z^2$ désigne un groupe carbonyle, $Y^2$ représente un groupe alkylène inférieur qui peut être interrompu par un groupe iminocarbonyle, $X^4$ représente un groupe répondant à la formule —O—, et $A^2$ désigne le radical répondant aux formules Ib ou Ic, dans lesquelles $R^{15}$ représente un radical alkyle présentant 7 à 30 atomes de carbone, non substitué ou substitué par un groupe hydroxy, amino et/ou alcanoylamino ayant jusqu'à 30 atomes de carbone, $R^{16}$ désigne l'hydrogène et $R^{17}$ un groupe 2-hydroxy- ou 1,2-dihydroxyéthyle, les groupes hydroxy étant éthérifiés indépendamment l'un de l'autre avec un radical alkyle présentant de 7 à 30 atomes de carbone, non substitué ou substitué par un groupe hydroxy, amino et/ou alcanoylamino ayant jusqu'à 30 atomes de carbone, ou estérifié par un radical alcanoyle non substitué ou substitué par un groupe hydroxy, amino et/ou alcanoylamino ayant jusqu'à 30 atomes de carbone, ou bien $R^{16}$ et $R^{17}$ représentent indépendamment l'un de l'autre un groupe hydroxyméthyle, dans lequel le groupe hydroxy est éthérifié par un radical alkyle présentant 7 à 30 atomes de carbone, non substitué ou substitué par un groupe hydroxy, amino et/ou alcanoylamino ayant jusqu'à 30 atomes de carbone, ou estérifié par un radical alcanoyle présentant 7 à 90 atomes de carbone, non substitué ou substitué par un groupe hydroxy, amino et/ou alcanoylamino ayant jusqu'à 30 atomes de carbone, et chacun des radicaux $R^9$ et $R^{11}$ représente indépendamment l'un de l'autre un groupe hydroxy, alcoxy inférieur, amino, alkylamino inférieur, carboxy(alkylamino inférieur) ou un radical répondant à la formule Ie, dans laquelle $X^5$ désigne un groupe répondant à la formule —O— ou —NH—, $Y^3$ désigne un groupe alkylène inférieur qui peut être interrompu par un groupe iminocarbonyle, $X^6$ désigne un groupe répondant à la formule —O— et $A^3$ représente le radical répondant aux formules Ib ou Ic, dans lesquelles $R^{15}$ représente un radical alkyle présentant 7 à 30 atomes de carbone, non substitué ou substitué par un groupe hydroxy, amino et/ou alcanoylamino ayant jusqu'à 30 atomes de carbone, $R^{16}$ désigne l'hydrogène et $R^{17}$ un groupe 2-hydroxy- ou 1,2-dihydroxyéthyle, les groupes hydroxy étant éthérifiés indépendamment l'un de l'autre dans un radical $R^{17}$ avec un radical alkyle présentant 7 à 30 atomes de carbone, non substitué ou substitué par un groupe hydroxy, amino et/ou alcanoylamino ayant jusqu'à 30 atomes de carbone, ou estérifiés par un radical alcanoyle présentant 7 à 90 atomes de carbone, non substitué ou substitué par un groupe hydroxy, amino et/ou alcanoylamino ayant jusqu'à 30 atomes de carbone, ou bien $R^{16}$ et $R^{17}$ représentent indépendamment l'un de l'autre un groupe hydroxyméthyle, dans lequel le groupe hydroxy est éthérifié par un radical alkyle ayant 7 à 30 atomes de carbone, non substitué ou substitué par un groupe hydroxy, amino et/ou alcanoylamino ayant jusqu'à 30 atomes de carbone, ou estérifié par un radical alcanoyle ayant 7 à 90 atomes de carbone, non substitué ou substitué par un groupe hydroxy, amino et/ou alcanoylamino ayant jusqu'à 30 atomes de carbone, sous réserve que les composés présentent au moins un radical du groupe $A^1$, $A^2$ et $A^3$, et/ou un sel pharmaceutiquement utilisable de ce composé.

4. Préparation selon la revendication 1, qui contient un composé répondant à la formule I, dans laquelle $X^1$ représente un groupe répondant à la formule —N($R^{14}$)—, dans laquelle $R^{14}$ désigne l'hydrogène ou un groupe alkyle en $C_1$ à $C_4$, $X^2$ désigne l'oxygène, $R^1$ désigne l'hydrogène, un groupe alcanoyle inférieur ou le groupe répondant à la formule Ia, dans laquelle n est égal à 0 ou 1, $Z^1$ représente un groupe carbonyle, $Y^1$ un groupe alkylène inférieur qui peut être interrompu par un groupe iminocarbonyle, $X^3$ un oxygène et $A^1$ un radical répondant aux formules Ib ou Ic, dans lesquelles $R^{15}$ désigne un radical alkyle ou alcényle présentant 7 à 30 atomes de carbone, non substitué ou substitué par un groupe hydroxy, amino et/ou alcanoylamino ayant jusqu'à 22 atomes de carbone, $R^{16}$ représente l'hydrogène et $R^{17}$ un groupe 2-hydroxy- ou 1,2-dihydroxyéthyle, au moins un groupe hydroxy iminocarbonyle, $X^6$ l'oxygène et $A^3$ indépendamment de $A^1$ et $A^2$, un radical répondant aux formules Ib ou Ic définies comme précédemment dans cette revendication, $R^{10}$ désigne l'hydrogène, $R^{12}$ l'hydrogène, un groupe alcanoyle inférieur ou le même radical que $R^3$, et $R^{13}$ désigne l'hydrogène, un groupe alcanoyle ou alcénoyle ayant chacun jusqu'à 30 atomes de carbone, ou indépendamment de $R^1$ et de $R^2$, un radical répondant à la formule Ia précédemment définie dans cette revendication, sous réserve que les composés présentent au moins un et au plus deux radicaux du groupe $A^1$, $A^2$ et $A^3$, et/ou un sel pharmaceutiquement acceptable de ce composé.

5. Préparation selon la revendication 4, qui contient un composé répondant à la formule I, dans

laquelle $X^1$ représente le groupe NH, $X^2$ représente l'oxygène, $R^1$ représente l'hydrogène ou un groupe alcanoyle inférieur, $R^2$ représente un groupe alcanoyle inférieur, benzyle, ou un groupe répondant à la formule Ia, dans laquelle n est égal à 0 ou 1, $Z^1$ désigne un groupe carbonyle, $Y^1$ désigne un groupe alkylène inférieur qui peut être interrompu par un ou deux groupes iminocarbonyle, $X^3$ désigne l'oxygène et $A^1$ désigne un radical répondant aux formules Ib ou Ic, dans lesquelles $R^{15}$ désigne un radical alkyle en $C_7$ à $C_{22}$, $R^{16}$ l'hydrogène et $R^{17}$ un groupe 1,2-dihydroxyéthyle, les deux groupes hydroxy étant estérifiés indépendamment l'un de l'autre par un groupe alcanoyle ou alcénoyle présentant à chaque fois 10 à 22 atomes de carbone, $R^3$ désigne l'hydrogène ou un groupe méthyle, $R^4$, $R^5$, $R^7$ et $R^8$ l'hydrogène, $R^6$ désigne l'hydrogène ou un groupe alkyle inférieur non substitué ou substitué par un groupe hydroxy libre, alcanoyloxy en $C_2$ à $C_{22}$, alcénoyloxy en $C_6$ à $C_{22}$, phényle ou par un groupe répondant à la formule Id, dans laquelle étant éthérifié dans le radical $R^{17}$ par un radical alkyle ou alcényle présentant 7 à 30 atomes de carbone ou estérifié avec un radical alcanoyle ou alcénoyle présentant 7 à 30 atomes de carbone, ou bien $R^{16}$ et $R^{17}$ représentent indépendamment l'un de l'autre un groupe hydroxyméthyle dans lequel le groupe hydroxy est éthérifié avec un radical alkyle ou alcényle présentant 7 à 30 atomes de carbone, ou estérifié avec un radical alcanoyle ou alcénoyle présentant 7 à 30 atomes de carbone, $R^2$ désigne un groupe alcanoyle inférieur, hydroxy(alcanoyle inférieur), benzoyle ou indépendamment de $R^1$, $R^2$ et $R^{13}$, un groupe répondant à la formule Ia définie comme précédemment dans cette revendication, $R^3$, $R^4$, $R^5$, $R^7$ et $R^8$ représentent indépendamment l'hydrogène, un groupe méthyle ou éthyle, $R^6$ désigne l'hydrogène ou un groupe alkyle inférieur non substitué ou substitué par un groupe hydroxy libre, un groupe mercapto libre, un groupe alcoxy inférieur, alkylthio inférieur, alcanoyloxy en $C_2$ à $C_{30}$, alcénoyloxy en $C_6$ à $C_{30}$, phényle, 4-hydroxyphényle ou par un groupe répondant à la formule Id, dans laquelle m est égal à 0 ou 1, R désigne l'oxygène ou le soufre, $Z^2$ un groupe carbonyle, $Y^2$ un groupe alkylène inférieur non substitué ou substitué par un groupe phényle qui peut être interrompu par un groupe iminocarbonyle, $X^4$ désigne l'oxygène et $A^2$ représente indépendamment de $A^1$ et de $A^3$ un radical répondant aux formules Ib ou Ic définies comme précédemment dans cette revendication, $R^9$ et $R^{11}$ représentent indépendamment l'un de l'autre un groupe hydroxy, alcoxy inférieur, amino, alkylamino inférieur, carboxy(alkylamino inférieur), (alcoxy inférieur) carbonyl(alkylamino inférieur), carbamoyl(alkylamino inférieur) ou un radical répondant à la formule Ie, dans laquelle $X^5$ désigne l'oxygène ou NH, $Y^3$ désigne un groupe alkylène inférieur, qui peut être interrompu par un groupe m est égal à 0 ou 1, E désigne l'oxygène, $Z^2$ un groupe carbonyle, $Y^2$ un groupe alkylène inférieur non substitué ou substitué par un groupe phényle, qui peut être interrompu par un groupe iminocarbonyle, $X^4$ désigne l'oxygène et $A^2$, indépendamment de $A^1$ et de $A^3$, un radical répondant aux formules Ib et Ic précédemment définies, $R^9$ et $R^{11}$ désignent indépendamment l'un de l'autre un groupe hydroxy, alcoxy inférieur, amino, alkylamino inférieur, -carboxy-(alkylamino inférieur), α-(alcoxy inférieur)carbonyl-alkylamino inférieur), α-carbamoyl-(alkylamino inférieur) ou un radical répondant à la formule Ie, dans laquelle $X^5$ désigne le groupe NH, $Y^3$ désigne un groupe alkylène inférieur qui peut être interrompu par un ou deux groupes imonocarbonyle, $X^6$ désigne l'oxygène et $A^3$ désigne indépendamment de $A^1$ et de $A^2$ un radical répondant aux formules Ib ou Ic précédemment définies, $R^{10}$ désigne l'hydrogène, $R^{12}$ désigne l'hydrogène, un groupe alcanoyle inférieur ou le même radical que $R^{13}$ et $R^{13}$ l'hydrogène, un groupe alcanoyle en $C_2$ à $C_{22}$, un groupe alcénoyle en $C_6$ à $C_{22}$ ou indépendamment de $R^2$, un radical répondant aux formules Ia telles que précédemment définies, et/ou un sel pharmaceutiquement utilisable de ces composés.

6. Préparation selon la revendication 5, qui contient un composé répondant à la formule I, dans lequel $X^1$ représente le groupe NH, $X^2$ l'oxygène, $R^1$ l'hydrogène ou un groupe alcanoyle en $C_2$ à $C_4$, $R^2$ un groupe alcanoyle en $C_2$ à $C_4$ ou un groupe répondant à la formule Ia, dans laquelle n est égal à 0 ou 1, $Z^1$ désigne un groupe carbonyle, $Y^1$ un groupe alkylène inférieur, qui peut être interrompu par un ou deux groupes iminocarbony, $X^3$ désigne l'oxygène et $A^1$ un radical répondant aux formules Ib ou Ic, dans lesquelles $R^{15}$ désigne un radical alkyle non ramifié en $C_{12}$ à $C_{18}$, $R^{16}$ l'hydrogène et $R^{17}$ un groupe 1,2-dipalmytoyloxy-éthyle ou 2-oléyloxy-1-palmytoyloxyéthyle, $R^3$ désigne l'hydrogène ou un groupe méthyle, $R^4$, $R^5$, $R^7$, $R^8$ et $R^{10}$ l'hydrogène, $R^6$ un groupe méthyle, éthyle ou isopropyle non substitué ou substitué par un radical répondant à la formule Id, dans laquelle m est égal à 0 ou 1, E désigne l'oxygène, $Z^2$ un groupe carbonyle, $Y^2$ un groupe alkylène inférieur non substitué ou substitué par un groupe phényle, qui peut être interrompu par un ou deux groupes iminocarbonyle, $X^4$ l'oxygène et $A^2$, indépendamment de $A^1$ et de $A^3$, un radical répondant aux formules Ib et Ic précédemment définies, et $R^9$ désigne un groupe amino, $R^{11}$ un groupe hydroxy ou un radical répondant à la formule Ie, dans laquelle $X^5$ désigne le groupe NH, $Y^3$ désigne un groupe alkylène inférieur qui peut être interrompu par un ou deux groupes iminocarbonyle, $X^6$ désigne l'oxygène et $A^3$ désigne, indépendamment de $A^1$ et de $A^2$, un radical répondant aux formules Ib ou Ic précédemment définies, $R^{12}$ représente l'hydrogène, un groupe acétyle ou le même radical que $R^{13}$ et $R^{13}$ représente l'hydrogène, un groupe acétyle ou indépendamment de $R^2$, un radical répondant à la formule Ia précédemment définie, et/ou un sel pharmaceutiquement utilisable de ce composé.

7. Préparation selon l'une des revendications 1 à 6, qui contient un composé répondant à la formule I, dans lequel les radicaux $A^1$, $A^2$ et $A^3$, s'ils sont présents, représentent un radical répondant à la formule Ic, et/ou un sel pharmaceutiquement utilisable de ce composé.

8. Préparation selon l'une des revendications 1 à 7, qui contient un composé répondant à la formule

I, dans lequel la partie sucre dérive du (D)-galactose ou du (D)-mannose, et/ou un sel pharmaceutiquement utilisable de ce composé.

9. Préparation selon l'une des revendications 1 à 7, qui contient un composé répondant à la formule I, dans laquelle la partie sucre dérive du (D)-glucose, et/ou un sel pharmaceutiquement utilisable de ce composé.

10. Préparation selon l'une des revendications 1 à 9, qui contient un composé répondant à la formule I, qui porte un seul substituant phosphoryle, et ceci dans le radical $R^{13}$, et/ou un sel pharmaceutiquement utilisable de ce composé.

11. Préparation selon l'une des revendications 1 à 9, qui contient un composé répondant à la formule I, qui porte un seul substituant phosphoryle, et ceci dans le radical $R^6$ et/ou un sel pharmaceutiquement utilisable de ce composé.

12. Préparation selon l'une des revendications 1 à 9, qui contient un composé répondant à la formule I qui porte un seul substituant phosphoryle, et ceci dans le radical $R^{11}$, et/ou un sel pharmaceutiquement utilisable de ce composé.

13. Préparation selon la revendication 1, qui contient du N-acétyl-muramyl-L-alanyl-D-isoglutaminyl-L-alanine-2-(1,2-dipalmitoyl-sn-glycéro-3-hydroxyphosphoryloxy)-éthylamide, et/ou un sel pharmaceutiquement utilisable de celui-ci.

14. Préparation selon l'une des revendications 1 à 13, qui contient un sel pharmaceutiquement utilisable d'un composé répondant à la formule I.

15. Préparation selon la revendication 14, qui contient un sel de sodium d'un composé répondant à la formule I.

16. Préparation selon la revendication 1, qui contient un composé répondant à la formule I, qui présente sur un atome C(-$R^3$) asymétriquement substitué la configuration (D), sur un atome C(-$R^6$) asymétriquement substitué la configuration (L), et sur l'atome C(-N-$R^8$) la configuration (D), et dans lequel la partie sucre dérive du (D)-glucose, $X^1$ désigne le groupe NH, $X^2$ l'oxygène, $R^1$, $R^4$, $R^5$, $R^7$, $R^8$, $R^{10}$, $R^{12}$ et $R^{13}$ l'hydrogène, $R^2$ un radical alkyle en $C_1$ à $C_3$ ou un radical phényle, $R^3$ l'hydrogène ou un radical alkyle en $C_1$ à $C_3$, $R^6$ l'hydrogène, un groupe alkyle en $C_1$ à $C_3$ substitué le cas échéant par un groupe hydroxy, méthoxy, mercapto, méthylmercapto ou par un halogène, un groupe phényle ou phénylméthyle substitué le cas échéant par un groupe hydroxy, méthoxy ou par un halogène, un groupe hétéroaryle ou hétéroarylméthyle contenant 1 ou 2 atomes Aza ayant 5 maillons dans le cycle, ou bien $R^5$ et $R^6$ forment ensemble un groupe triméthylène, un des radicaux $R^9$ et $R^{11}$ un radical répondant à la formule Ie, dans laquelle $X^5$ désigne le groupe NH ou l'oxygène, $Y^3$ désigne un groupe alkylène en $C_2$ à $C_3$ ou un radical répondant aux formules

$$-U^2-COO-U^3- \quad \text{ou} \quad -U^2-\overset{O}{\overset{\|}{C}}-NH-U^3- ,$$

dans lesquelles $U^2$ et $U^3$ désignent indépendamment l'un de l'autre chacun un groupe alkylène en $C_1$ à $C_3$, qui est non substitué ou substitué par un groupe alkyle en $C_1$ à $C_3$ éventuellement substitué par un groupe hydroxy, alcoxy inférieur, mercapto ou (alkyle inférieur) mercapto ou par un groupe phényle ou phényl(alkyle inférieur) substitué le cas échéant par un groupe hydroxy, méthoxy ou par un halogène, ou par un groupe hétérocyclyle ou hétérocyclyl-(alkyle en $C_1$ à $C_3$) contenant 1 ou 2 atomes Aza, ayant chacun 5 ou 6 maillons dans le cycle, $X^6$ désigne l'oxygène et $A^3$ un radical répondant à la formule Ic, dans laquelle $R^{16}$ désigne l'hydrogène et $R^{17}$ un groupe 1,2-dihydroxyéthyle ou 2-hydroxyéthyle dans lequel au moins un des groupes hydroxy est estérifié avec un acide carboxylique aliphatique en $C_{16}$ à $C_{20}$, le cas échéant une ou deux fois insaturé, ou éthérifié avec un alcool aliphatique en $C_{12}$ à $C_{18}$, insaturé le cas échéant une ou deux fois, et l'autre des radicaux $R^9$ et $R^{11}$ désigne un groupe hydroxy, alcoxy inférieur, amino, alkylamino inférieur et aminocarbonyle (alkylamino inférieur) et/ou un sel pharmaceutiquement utilisable d'un tel composé.

17. Préparation selon la revendication 1, qui contient un composé répondant à la formule I, qui présente sur un atome C(-$R^3$) asymétriquement substitué, la configuration (D), sur un atome C(-$R^6$) asymétriquement substitué, la configuration (L) et sur l'atome C(-N-$R^8$), la configuration (D), et dans lequel la partie sucre dérive du (D)-glucose, $X^1$ désigne le groupe NH, $X^2$ l'oxygène, $R^1$, $R^4$, $R^7$, $R^8$, $R^{10}$, $R^{12}$ et $R^{13}$ l'hydrogène, $R^2$ un groupe alkyle inférieur ou un groupe phényle, $R^3$ et $R^5$ désignent indépendamment l'un de l'autre l'hydrogène ou un groupe méthyle, $R^6$ un groupe alkyle en $C_1$ à $C_4$, $R^9$ un groupe amino et $R^{11}$ un radical répondant à la formule

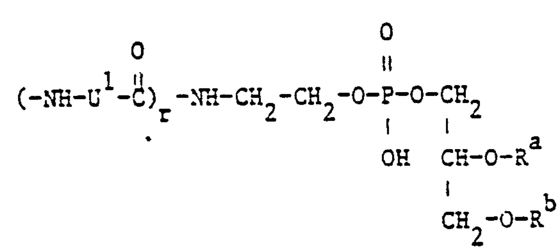

dans laquelle r est égal à 0 ou 1, $U^1$ désigne un radical répondant à l'une des formules

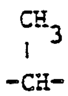 , 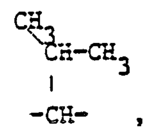 , 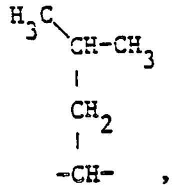 , 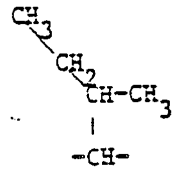

(L) (L) (L) (L)

et $R^a$ et $R^b$ désignent indépendamment l'un de l'autre le radical acyle d'un acide carboxylique aliphatique en $C_{12}$ à $C_{22}$, saturé ou présentant une ou deux double liaisons, et/ou un sel pharmaceutiquement utilisable d'un tel composé.

18. Préparation pharmaceutique applicable localement selon l'une des revendications 1 à 19, qui contient 0,001 % à 1 % non compris de substance active.

19. Collyre selon la revendication 18.

20. Gouttes nasales aqueuses selon la revendication 18.

21. Gouttes nasales aqueuses selon la revendication 18.

22. Gel selon la revendication 18.

23. Crème selon la revendication 18.

24. Pommade nasale selon la revendication 18.

25. Pommade nasale selon la revendication 18.

26. Baton de rouge à lèvres selon la revendication 18.

27. Préparation pharmaceutique selon l'une des revendications 1 à 17, pour l'administration orale sous la forme de comprimés ou de capsules ayant une teneur en substances actives de 0,1 à 0,9 % en poids.

28. Préparation pharmaceutique selon l'une des revendications 1 à 19, sous la forme d'unités de doses ayant une teneur en substance active de 0,1 à 1 mg par unité de doses.

29. Préparation pharmaceutique selon l'une des revendications 1 à 28, qui est destinée à l'utilisation chez des animaux à sang chaud pour la prophylaxie ou le traitement de maladies provoquées par des virus.

30. Utilisation d'un composé répondant à la formule I définie dans l'une des revendications 1 à 17 ou d'un sel pharmaceutiquement utilisable d'un tel composé pour la préparation de médicaments qui sont destinés à l'utilisation chez des animaux à sang chaud pour la prophylaxie ou le traitement de maladies provoquées par des virus.

31. Utilisation selon la revendication 30 pour la prophylaxie ou le traitement de maladies qui sont provoquées par les virus de l'influenza ou de l'herpès.

32. Utilisation selon la revendication 30 pour la prophylaxie ou le traitement de maladies qui sont provoquées par les virus de l'encéphalomyocardite, de la vaccine ou du parainfluenza.

Fig. 1

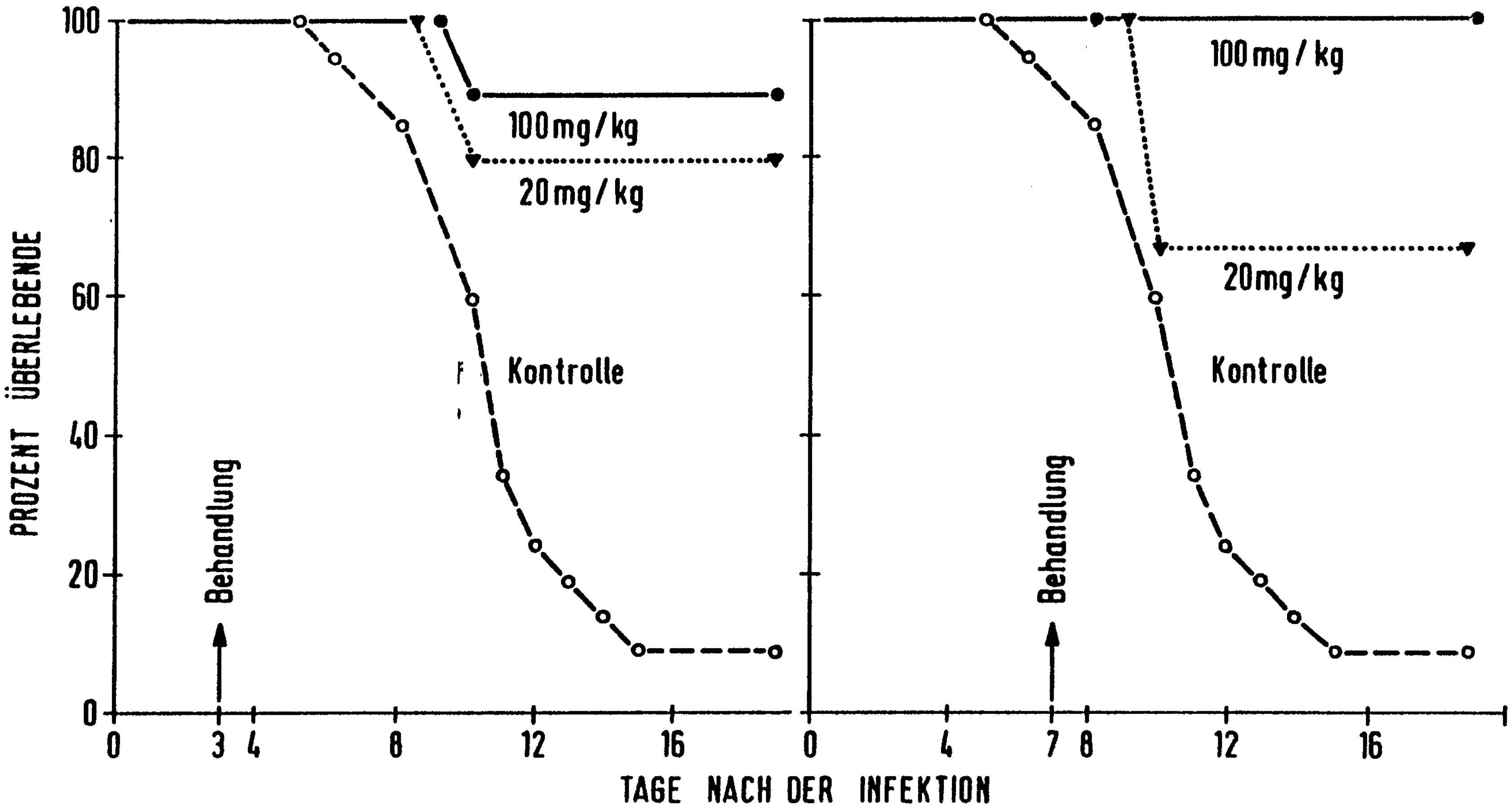


Therapeutischer Effekt von oral applizierter Verbindung I (Einzeldosis am Tag 3) auf eine Gruppe von 10 mit dem Virusstamm Influenza A/Victoria/3/75 intranasal infizierten Mäusen. (20 Mäuse in der Kontrollgruppe).

Fig. 2

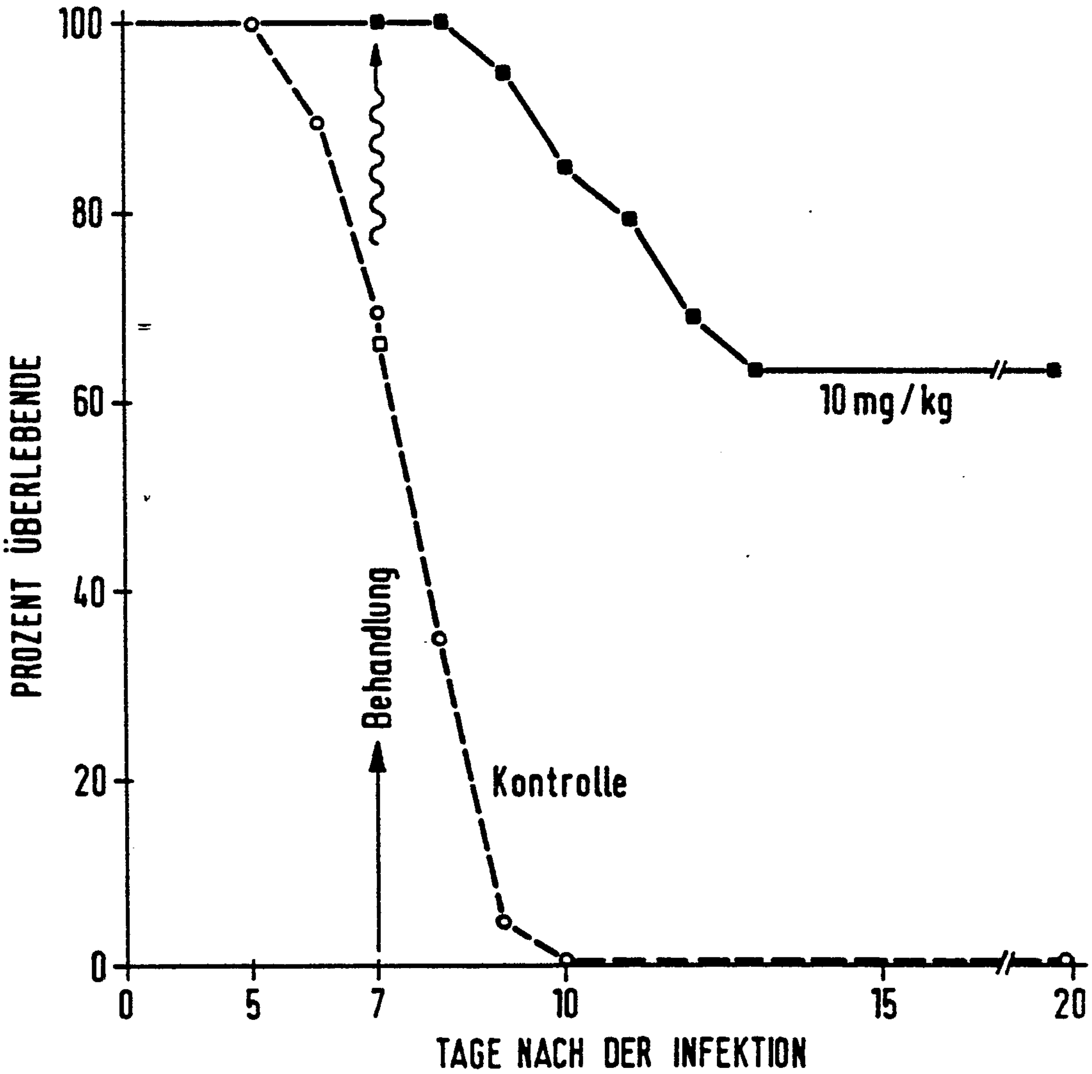


Therapeutischer Effekt von oral applizierter Verbindung I (Einzeldosis am Tag 7) auf eine Gruppe von 30 mit dem Virusstamm Influenza A/Texas/1/77 intranasal infizierten Mäusen (20 Mäuse in der Kontrollgruppe).

Fig. 3

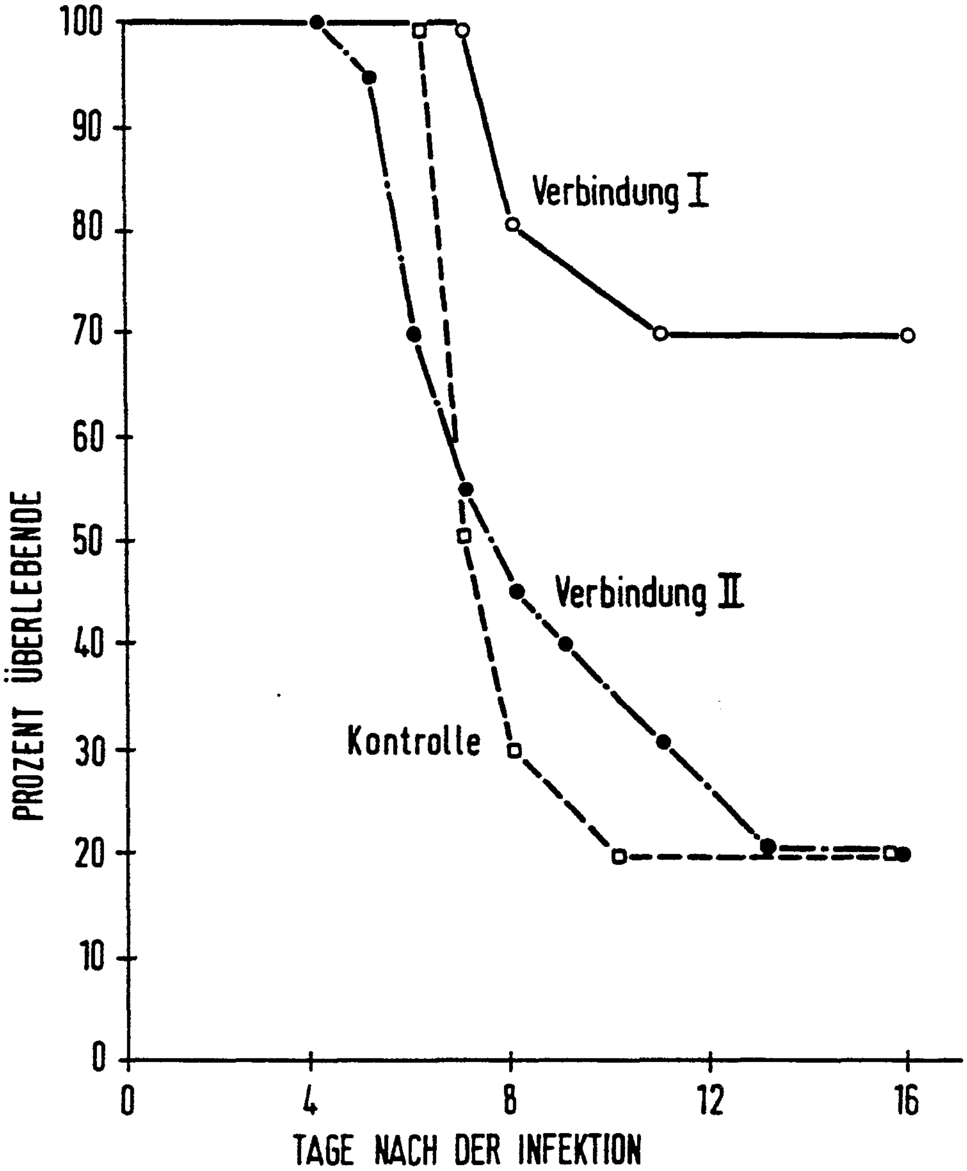


Prophylaktische Wirkung von je 10 mg/kg (Einzeldosis am 7. Tag vor der Infektion) intranasal applizierter Verbindung I bzw. Verbindung II auf eine Gruppe von 10 mit Herpesvirus hominis 1/TUP intranasal infizierten Mäusen (20 Mäuse in der Kontrollgruppe).

Fig. 4

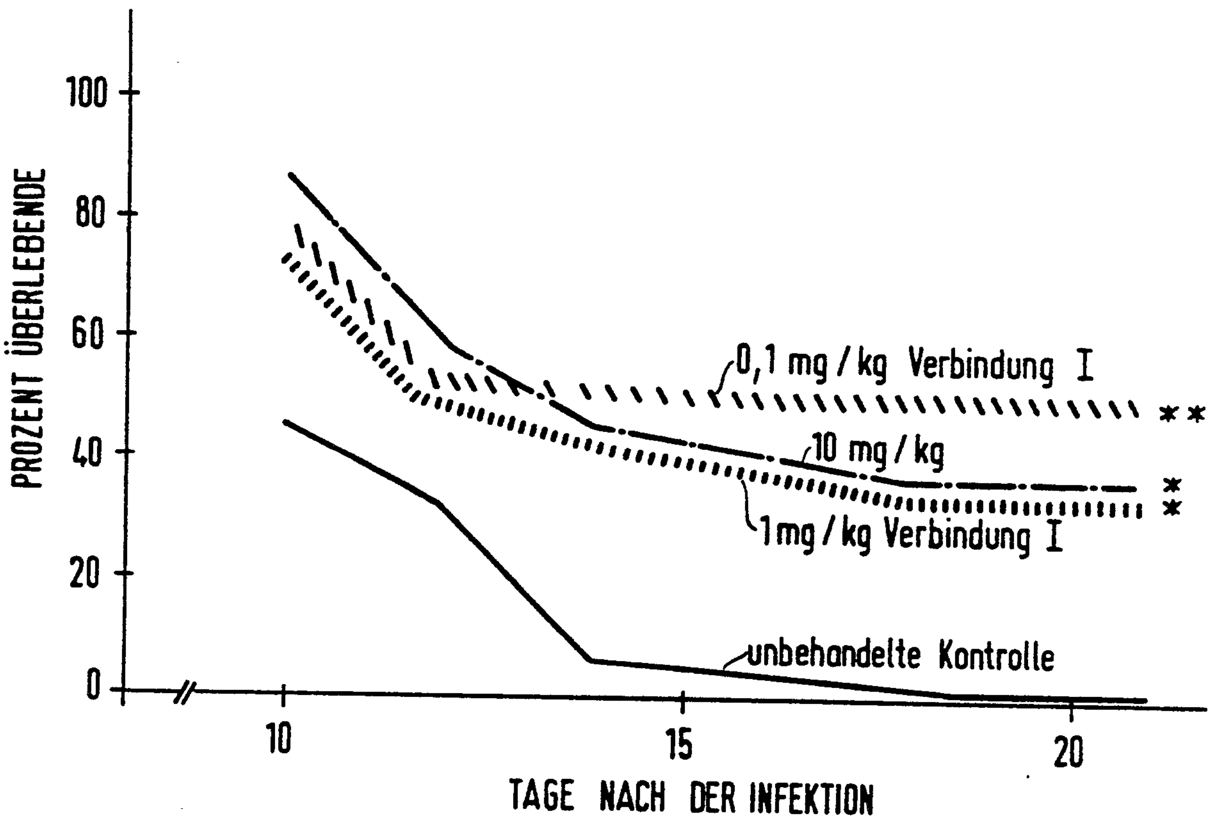


Therapeutische Wirkung von oral applizierter Verbindung I auf mit Herpes Simplex Virus 2 / Alabama infizierte Meerschweinchen.

statistische Signifikanz $^{*}P \leq 0{,}05$, $^{**}P \leq 0{,}01$ (Vierfelder Test)

Fig. 5

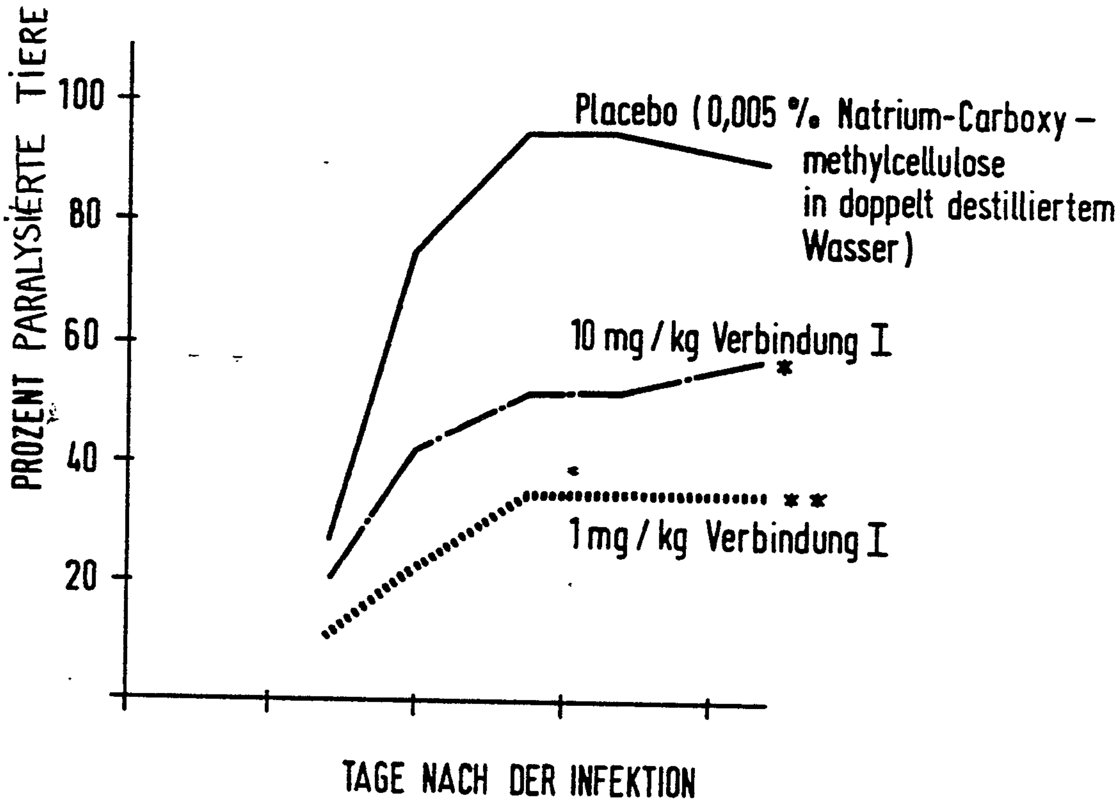


Prophylaktische Wirkung von intranasal applizierter Verbindung I auf mit Herpes Simplex Virus 2/MS infizierte Meerschweinchen.

statistische Signifikanz *P≤0,05, **P≤0,01 (Vierfelder Test)

**Fig. 6a**

Wirkstoffgabe am 3. Tag nach der Infektion

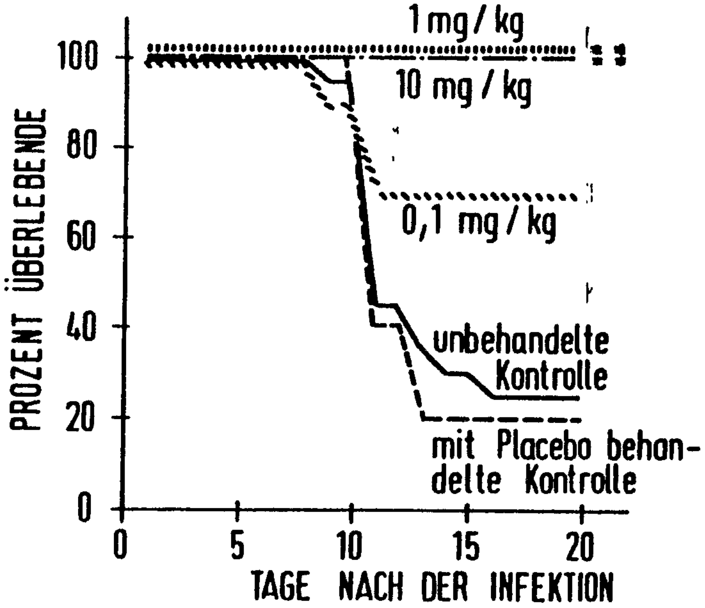


**Fig. 6b**

Wirkstoffgabe am 7. Tag nach der Infektion

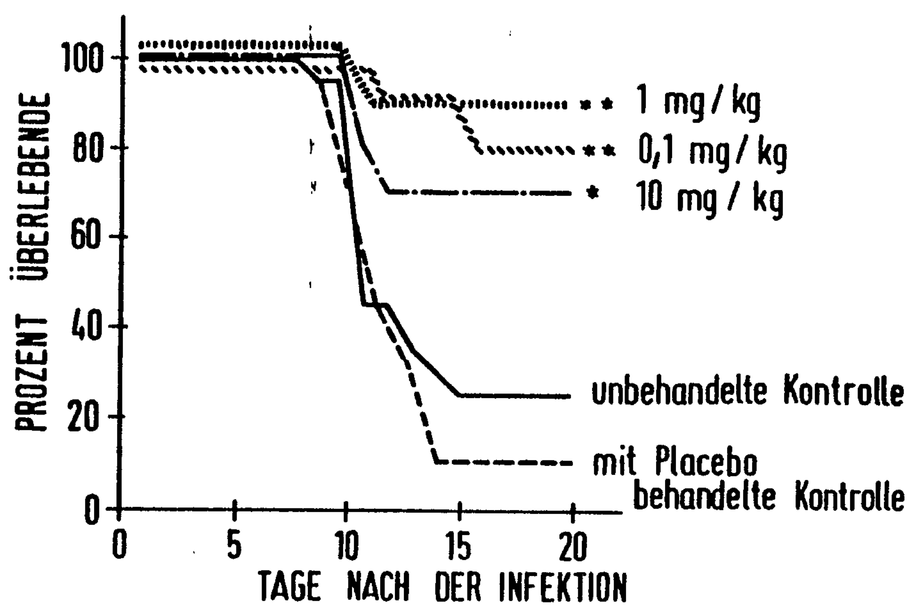


**Fig. 6** Therapeutische Wirkung oral applizierter Verbindung I auf mit Parainfluenza Virus I (Sendai) / 52 infizierte Mäuse.

statistische Signifikanz $^{*}P \leq 0{,}05$, $^{**}P \leq 0{,}01$ (Vierfelder Test)

## Fig. 7a

Wirkstoffgabe am 21. Tag vor der Infektion

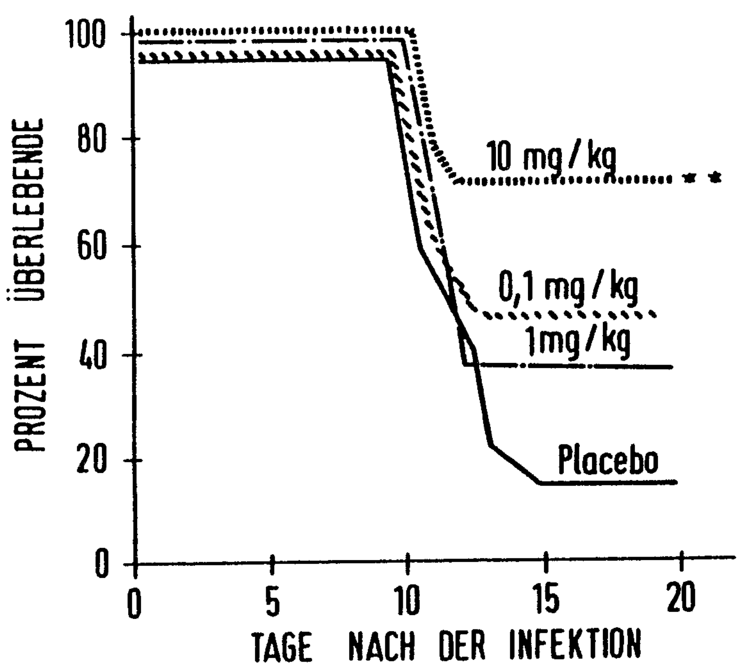


## Fig. 7b

Wirkstoffgabe am 14. Tag vor der Infektion

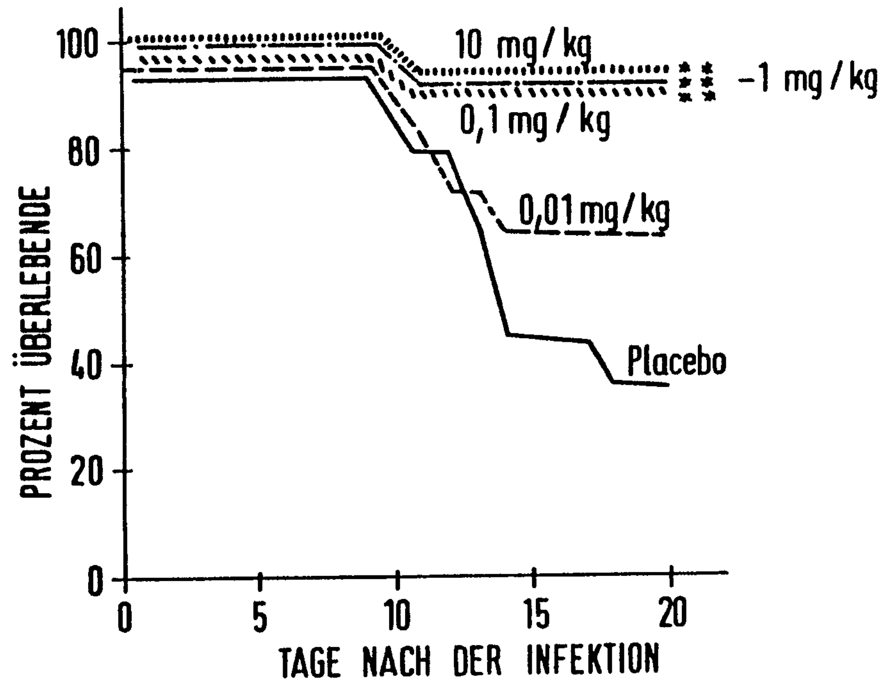


**Fig. 7** TEIL I: Wirkung intranasal applizierter Verbindung I auf mit Parainfluenza Virus I (Sendai)/52 infizierte Mäuse.

statistische Signifikanz ** $P \leq 0{,}01$ (Vierfelder Test)

**Fig. 7c**

Wirkstoffgabe am 7. Tag vor der Infektion

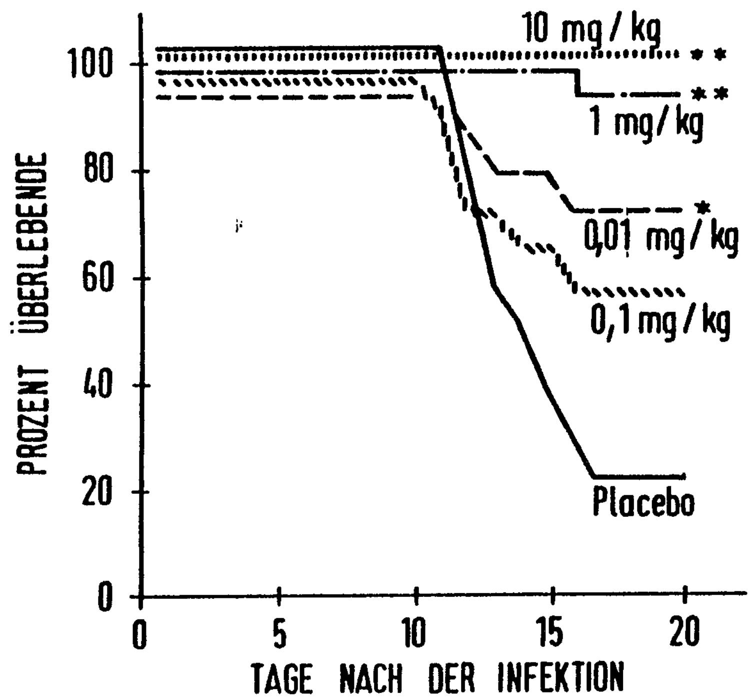


**Fig. 7d**

Wirkstofgabe am 7. Tag nach der Infektion

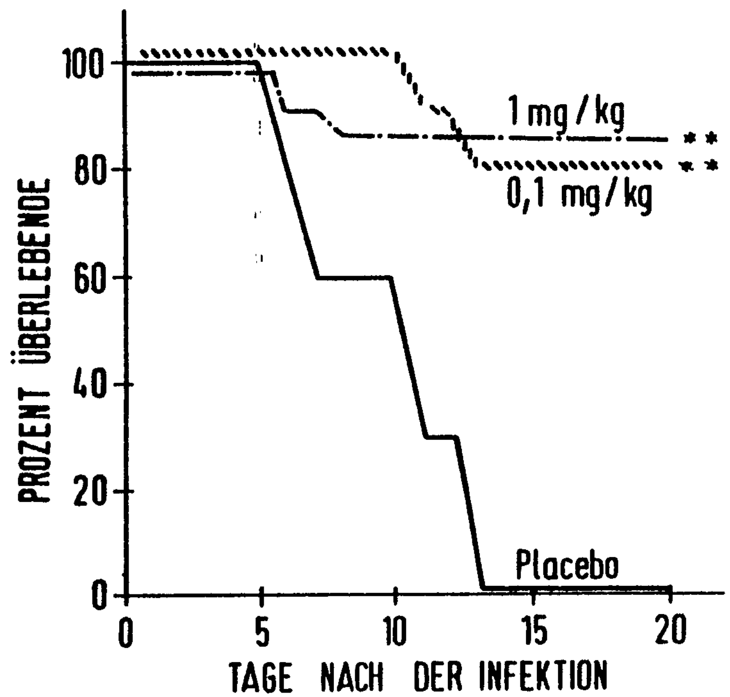


**Fig. 7** TEIL II : Wirkung intranasal applizierter Verbindung I auf mit Parainfluenza Virus I (Sendai)/52 infizierte Mäuse.

statistische Signifikanz $^{*}P \leq 0{,}05$, $^{**}P \leq 0{,}01$ (Vierfelder Test)